# EUROPÄISCHE PATENTSCHRIFT

(11) **EP 2 379 526 B1**
(45) Veröffentlichungstag und Bekanntmachung des Hinweises auf die Patenterteilung: **27.05.2015**
(21) Anmeldenummer: 09764749.9
(22) Anmeldetag: 09.12.2009
(51) Int. Cl.: C07D 401/14, C07D 405/14, A01N 43/713

(54) **Tetrazolsubstituierte Anthranilsäureamide als Pestizide**
Tetrazol-substituted anthranilic acid amides as pesticides
Amides d'acide anthranilique substitués au tétrazole en tant que pesticides

(30) Priorität: 18.12.2008 EP 08172205
(43) Veröffentlichungstag der Anmeldung: 26.10.2011
(60) Teilanmeldung: 12159076.4
(73) Patentinhaber: Bayer Intellectual Property GmbH, 40789 Monheim am Rhein (DE)
(72) Erfinder: FISCHER, Rüdiger, 50259 Pulheim (DE); FUNKE, Christian, 42799 Leichlingen (DE); GESING, Ernst, Rudolf, 40699 Erkrath (DE); GRONDAL, Christoph, 50937 Köln (DE); HENSE, Achim, 51379 Leverkusen (DE); BECKER, Angela, 40235 Düsseldorf (DE); FRANKEN, Eva-Maria, 51381 Leverkusen (DE); MALSAM, Olga, Rösrath 51503 (DE); VOERSTE, Arnd, 50674 Köln (DE); GÖRGENS, Ulrich, 40882 Ratingen (DE); WROBLOWSKY, Heinz-Juergen, 40764 Langenfeld (DE)
(74) Vertreter: BIP Patents
(86) Internationale Anmeldenummer: PCT/EP2009/008775
(87) Internationale Veröffentlichungsnummer: WO 2010/069502

(56) Entgegenhaltungen:
- WO-A-2007/144100
- WERMUTH C G: "MOLECULAR VARIATIONS BASED ON ISOSTERIC REPLACEMENTS" PRACTICE OF MEDICINAL CHEMISTRY,, 1. Januar 1996 (1996-01-01), Seiten 203-237, XP002190259

## Beschreibung

Die vorliegende Erfindung betrifft tetrazolsubstituierte Anthranilsäureamide, mehrere Verfahren zu ihrer Herstellung und ihre Verwendung als Wirkstoffe auch in Kombination mit weiteren Mitteln zur Wirksamkeitssteigerung, insbesondere ihre Verwendung als Schädlingsbekämpfungsmittel.

In der Literatur ist bereits beschrieben, daß bestimmte Anthranilsäureamide (z.B. WO 01/70671, WO 03/015519, WO 03/016284, WO 03/015518, WO 03/024222, WO 03/016282, WO 03/016283, WO 03/062226, WO 03/027099, WO 04/027042, WO 04/033468, WO 2004/046129, WO 2004/067528, WO 2005/118552, WO 2005/077934, WO 2005/085234, WO 2006/023783, WO 2006/000336, WO 2006/040113, WO 2006/111341, WO 2007/006670, WO 2007/024833, WO2007/020877 und WO 07/144100) insektizide Eigenschaften besitzen.

In der Literatur wurde bereits ebenfalls beschrieben, dass sich die Wirkung verschiedener Wirkstoffe durch Zugabe weiterer Mittel, u.a. von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosal und Phosphinothricin beschrieben (US 6 645 914, EP-A2 0 036 106). Der Einsatz von Ammoniumsulfat als Formulierhilfsmittel ist für bestimmte Wirkstoffe und Anwendungen ebenfalls beschrieben (WO 92/16108), es dient dort aber zur Stabilisierung der Formulierung, nicht zur Wirkungssteigerung. Desweiteren werden Kombinationen von Ammoniumsalzen mit insektiziden Wirkstoffen beschrieben in WO 07/068356, WO 07/068428, WO 07/068355, WO 07/068357, und WO 07/068350. Auf diese Veröffentlichungen wird hiermit ausdrücklich Bezug genommen.

Es wurde nun gefunden, dass die neuen tetrazolhaltigen Anthranilsäureamide Vorteile gegenüber dem Stand der Technik darstellen, z.B. durch bessere biologische oder ökologische Eigenschaften. Als weitere Vorteile seien breitere Anwendungsmethoden, eine bessere insektizide, akarizide Wirkung, sowie eine gute Verträglichkeit gegenüber Nutzpflanzen beispielhaft genannt. Die tetrazolhaltigen Anthranilsäureamide können in Kombination mit weiteren Mitteln zur Verbesserung der Wirksamkeit insbesondere gegen schwierig zu bekämpfende Insekten eingesetzt werden.

Gegenstand der vorliegenden Erfindung sind tetrazolsubstituierte Anthranilsäureamide der allgemeinen Formel (I) 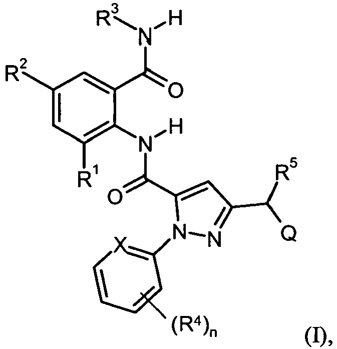 in welcher
R¹ für Methyl oder Chlor steht
R² für Halogen, Cyano, Methyl oder C₁-C₄-Alkylsulfonyl steht,
R³ für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl, C₃-C₁₂-Cycloalkyl, C₃-C₁₂-Cycloalkyl-C₁-C₆-Alkyl steht, wobei die Substituenten unabhängig voneinander ausgewählt sein können aus Halogen, Amino, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₂-C₆-Alkoxycarbonyl, C₂-C₆-Alkylcarbonyl C₃-C₆-Cycloalkylamino oder einem 5- oder 6-gliedrigen heteroaromatischen Ring,
n für 1, 2, 3 oder 4 steht,
X für N, CH, CF, CCl, CBr steht,
R⁴ unabhängig voneinander für Wasserstoff, Cyano, Halogen(C₁-C₆)alkyl, Halogen oder Halogen(C₁-C₄)alkoxy steht,
R⁵ für H oder C₁-C₆-Alkyl steht,
Q für den einfach substituiertenTetrazol-Rest Q-2 steht, 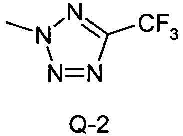 sowie Salze von Verbindungen der Formel (I).

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen tetrazolsubstituierten Anthranilsäureamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.

Erfindungsgemäß bevorzugt sind Verbindungen der Formel (I-1) 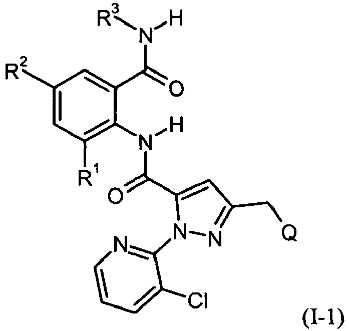 in welcher
R¹ für Methyl oder Chlor steht,
R² für Halogen, Cyano oder Methyl steht,
R³ für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl, C₃-C₁₂-Cycloalkyl, C₃-C₁₂-Cycloalkyl-C₁-C₆-Alkyl steht, wobei die Substituenten unabhängig voneinander ausgewählt sein können aus Halogen, Amino, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₂-C₆-Alkoxycarbonyl, C₂-C₆-Alkylcarbonyl C₃-C₆-Cycloalkylamino oder einem 5- oder 6-gliedrigen heteroaromatischen Ring,
Q für den einfach substituierten Tetrazol-Rest Q-2 steht, 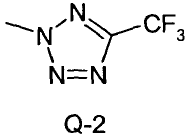 sowie Salze von Verbindungen der Formel (I-1).

Bevorzugt, besonders bevorzugt und ganz besonders bevorzugt sind Verbindungen der allgemeinen Formel (I-1)
wobei
R¹ bevorzugt und besonders bevorzugt für Methyl steht,
R² bevorzugt für Halogen, Cyano oder Methyl steht,
R² besonders bevorzugt für Chlor und Cyano steht,
R² ebenfalls besonders bevorzugt für Brom, Fluor, Jod oder Methyl steht,
R³ bevorzugt für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl, C₃-C₆-Cycloalkyl, C₃-C₆-Cycloalkyl-C₁-C₆-Alkyl steht, wobei die Substituenten unabhängig voneinander ausgewählt sein können aus Halogen, Cyano, Amino, Hydroxy, C₁-C₆-Alkyl, C₁-C₄-Alkoxy, C₁-C₄-Haloalkoxy, C₁-C₄-Alkylthio, C₃-C₆-Cycloalkyl, einem 5 oder 6-gliedrigen heteroaromatischen Ring enthaltend 1-2 Heteroatome aus der Reihe N,O, S, wobei nicht zwei Sauerstoffatome im Ring benachbart sind,
R³ besonders bevorzugt für einen der folgenden Reste steht,

| | | | | |
|---|---|---|---|---|
| H | 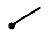 | 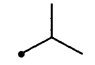 | 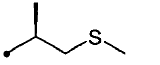 | 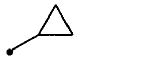 |
| 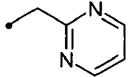 | 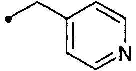 | 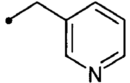 | 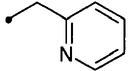 | 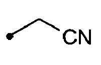 |
| 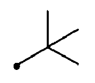 | 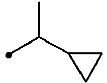 | 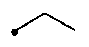 | 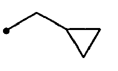 | 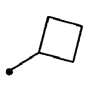 |
| 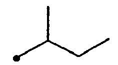 | 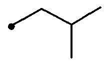 | 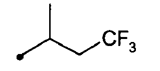 | 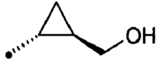 | 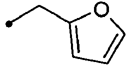 |
| 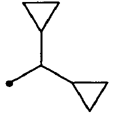 | 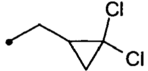 | 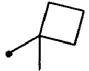 | 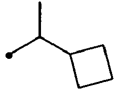 | 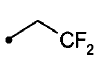 |
| 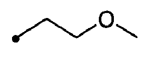 | 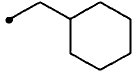 | 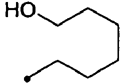 | 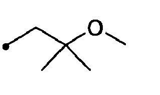 | 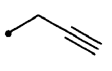 |
| 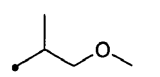 | 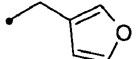 | 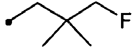 | 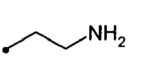 | 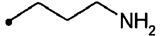 |
| 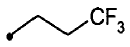 | 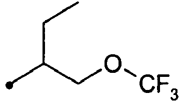 | | | |

R³ ganz besonders bevorzugt für einen der folgenden Reste steht

| | | | | |
|---|---|---|---|---|
| H | 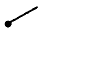 | 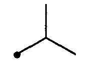 | 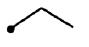 | 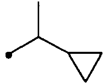 |
| 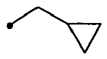 | 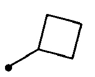 | 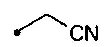 | 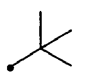 | 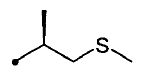 |

Ebenfalls erfindungsgemäß bevorzugt sind Verbindnungen der Formel (I), wobei die Reste R¹, R² und R³ die oben als bevorzugt, besonders bevorzugt und ganz besonders bevorzugt angegebenen Bedeutungen aufweisen und wobei
R⁴ bevorzugt für Halogen(C₁-C₆)alkyl oder Halogen, besonders bevorzugt für Chlor oder Brom steht, ganz besonders bevorzugt für Chlor steht.
R⁵ bevorzugt für Wasserstoff, Methyl, Ethly, Propyl oder iso-Propyl, besonders bevorzugt für Wasserstoff oder Methyl steht,
X bevorzugt für N, CCl oder CH steht, besonders bevorzugt für N oder CH steht,
n bevorzugt für 1,2 oder 3 steht, besonders bevorzugt für 1 oder 2 steht, ganz besonders bevorzugt für 1 steht.

Die Verbindungen der Formeln (I) und (I-1) können in Form verschiedener Isomere vorliegen. Gegenstand der vorliegenden Erfindung sind daher auch die Isomere von Verbindungen der Formeln (I) und (I-1), sowie Mischungen verschiedener isomerer Formen.

Insbesondere können die Verbindungen der Formeln (I) und (I-1) in Form verschiedener Regioisomere vorliegen. Beispielsweise in Form von Mischungen aus Verbindungen mit der Definition Q2 bzw. Q6.

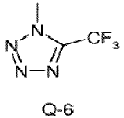

Daher können Mischungen aus Verbindungen der Formeln (I) und (I-1), wobei Q die Bedeutungen Q2 und Q6 hat in verschiedenen Mischungsverhältnissen vorliegen Bevorzugt sind dabei Mischungsverhältnisse aus Verbindungen der Formel (I) oder (I-1), worin der Rest Q für Q2 steht zu Verbindungen der Formel (I) oder (I-1), worin der Rest Q für Q6 steht, von 60:40 bis 99:1, besonders bevorzugt von 70:30 bis 97:3. ganz besonders bevorzugt von 80:20 bis 95:5. Insbesonders bevorzugt sind die folgenden Mischungsverhältnisse einer Verbindung der Formel (I) oder (I-1), wobei Q die Bedeutung Q2 hat zur Verbindung der Formeln (I) oder (I-1), wobei Q die Bedeutung Q6 hat: 80:20; 81:19; 82:18; 83:17; 84:16; 85:15, 86:14; 87:13; 88:12; 89:11; 90:10, 91:9; 92:8; 93:7; 96:6; 95;5.

### Herstellung der erfindungsgemäßen Verbindungen der allgemeinen Formel (I)

Anthranilamide der Formel (I) werden nach nach einem der folgenden Verfahren erhalten. Anthranilamide der Formel (I) 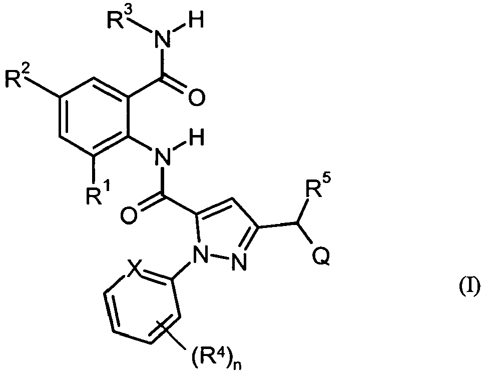 in welcher R¹, R², R³, R⁴, R⁵, n, X und Q die oben angegebenen Bedeutungen haben, werden erhalten, indem man

### (A) Aniline der Formel (II)

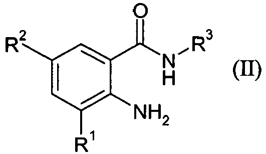

in welcher R¹, R² und R³ die oben angegebenen Bedeutungen haben,
mit Carbonsäurechloriden der Formel (III) 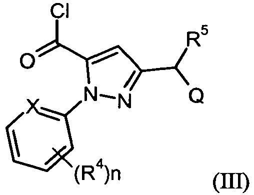 in welcher X, Q, R⁴, R⁵ und n die oben angegebene Bedeutung hat, in Gegenwart eines Säurebindemittels umsetzt,

### (B) Aniline der Formel (II)

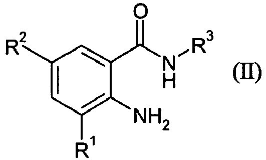

in welcher R¹, R² und R³ die oben angegebenen Bedeutungen haben,
mit einer Carbonsäure der Formel (IV) 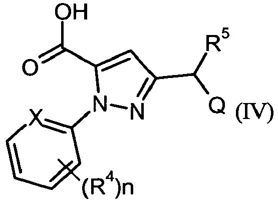 in welcher Q, R⁴, R⁵, n, X die oben angegebene Bedeutung haben,
in Gegenwart eines Kondensationsmittels umsetzt oder indem man

### (C) Benzoxazinone der Formel (V)

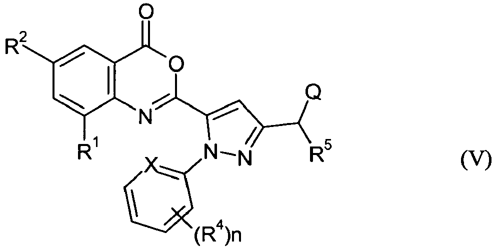

in welcher R¹, R², R⁴, R⁵, n, X und Q die oben angegebenen Bedeutungen haben,
mit einem Amin der Formel (X) 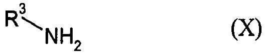 in welcher R³ die oben angegebene Bedeutung hat,
in Gegenwart eines Verdünnungsmittels umsetzt.

Weiterhin wurde gefunden, dass man Anthranilamide der Formel (I-1) nach einem der folgenden Verfahren erhält.

### Anthranilamide der Formel (I-1)

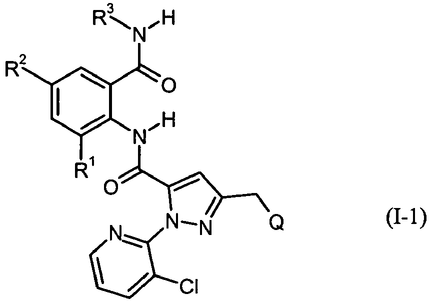

in welcher R¹, R², R³ und Q die oben angegebenen Bedeutungen haben, werden erhalten, indem man

### (A) Aniline der Formel (II)

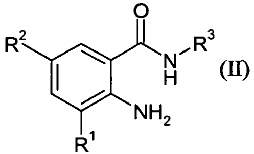

in welcher R¹, R² und R³ die oben angegebenen Bedeutungen haben,
mit Carbonsäurechloriden der Formel (III-1) 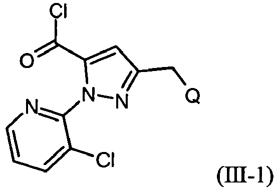 in welcher Q die oben angegebene Bedeutung hat, in Gegenwart eines Säurebindemittels umsetzt,

### (B) Aniline der Formel (II)

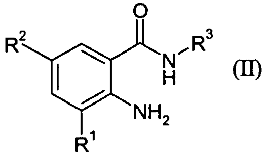

in welcher R¹, R² und R³ die oben angegebenen Bedeutungen haben,
mit einer Carbonsäure der Formel (IV-1) 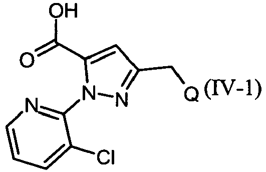 in welcher Q die oben angegebene Bedeutung hat,
in Gegenwart eines Kondensationsmittels umsetzt oder indem man

### (C) Benzoxazinone der Formel (V-1)

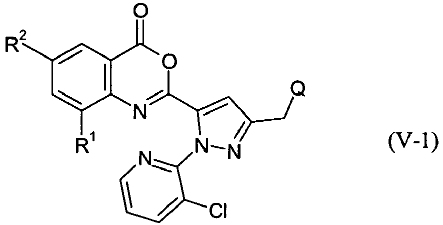

in welcher R¹, R² und Q die oben angegebenen Bedeutungen haben,
mit einem Amin der Formel (X) 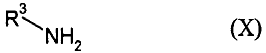 in welcher R³ die oben angegebene Bedeutung hat,
in Gegenwart eines Verdünnungsmittels umsetzt.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura furniferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die Wirksamkeit der Verbindungen der Formel (I) lässt sich durch die Zugabe von Ammonium- und Phosphoniumsalzen steigern. Die Ammonium- und Phosphoniumsalze werden durch Formel (XI) definiert 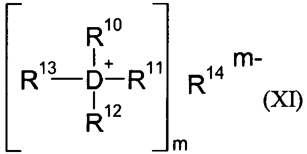 in welcher
D für Stickstoff oder Phosphor steht,
D bevorzugt für Stickstoff steht,
R¹⁰, R¹¹, R¹², and R¹³ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
R¹⁰, R¹¹, R¹², and R¹³ bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
R¹⁰, R¹¹, R¹², and R¹³ besonders bevorzugt unabhängig voneinander für Wasserstoff, Methyl, Ethyl, n-Propyl, i-Propyl, n-Butyl, i-Butyl, s-Butyl oder t-Butyl stehen,
R¹⁰, R¹¹, R¹², and R¹³ ganz besonders bevorzugt für Wasserstoff stehen,
m für 1, 2, 3 oder 4 steht,
m bevorzugt für 1 oder 2 steht,
R¹⁴ für ein anorganisches oder organisches Anion steht,
R¹⁴ bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat, Citrat oder Oxalat steht,
R¹⁴ besonders bevorzugt für Laktat, Sulfat, Monohydrogenphosphat, Dihydrogenphosphat, Nitrat, Thiosulfat, Thiocyanat, Citrat, Oxalat oder Formiat steht.
R¹⁴ ganz besonders bevorzugt für Sulfat steht.

Die Ammonium- und Phosphoniumsalze der Formel (XI) können in einem breiten Konzentrationsbereich zur Steigerung der Wirkung von Pflanzenschutzmitteln enthaltend Verbindungen der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern ein Penetrationsförderer zugegeben. Selbst in diesen Fällen ist eine Wirkungssteigerung zu beobachten. Gegenstand der vorliegenden Erfindung ist also ebenfalls die Verwendung eines Penetrationsförders, sowie die Verwendung einer Kombination von Penetrationsförderer und Ammonium- und/oder Phosphoniumsalzen zur Wirkungssteigerung von Pflanzenschutzmitteln, die akarizid/insektizid wirksame Verbindungen der Formel (I) als Wirkstoff enthalten. Gegenstand der Erfindung ist schließlich weiterhin die Verwendung dieser Mittel zur Bekämpfung von Schadinsekten.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfmdungsgemäße Penetrationsförderer sind Alkanol-alkoxylate der Formel

R-O-(-AO)ᵥ₋R' (XII)

in welcher
R für geradkettiges oder verzweigtes Alkyl mit 4 bis 20 Kohlenstoffatomen steht,
R' für Wasserstoff, Methyl, Ethyl, n-Propyl, i-Propyl, n-Butyl, i-Butyl, t-Butyl, n-Pentyl oder n-Hexyl steht,
AO für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
v für Zahlen von 2 bis 30 steht.

Eine bevorzugte Gruppe von Penetrationsförderern sind Alkanolalkoxylate der Formel

R-O-(-EO-)ₙ-R' (XII-a)

in welcher
R die oben angegebene Bedeutung hat,
R' die oben angegebene Bedeutung hat,
EO für -CH₂-CH₂-O- steht und
n für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (XII-b)

in welcher
R die oben angegebene Bedeutung hat,
R' die oben angegebene Bedeutung hat,
EO für -CH₂-CH₂-O- steht,
PO für 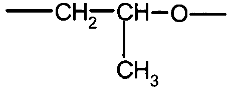 steht,
p für Zahlen von 1 bis 10 steht und
q für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (XII-c)

in welcher
R die oben angegebene Bedeutung hat,
R' die oben angegebene Bedeutung hat,
EO für -CH₂-CH₂-O- steht,
PO für 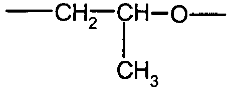 steht,
r für Zahlen von 1 bis 10 steht und
s für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (XII-d)

in welcher
R und R' die oben angegebenen Bedeutungen haben,
EO für CH₂-CH₂-O- steht,
BO für 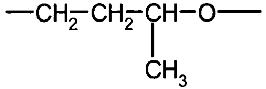 steht,
p für Zahlen von 1 bis 10 steht und
q für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (XII-e)

in welcher
R und R' die oben angegebenen Bedeutungen haben,
BO für 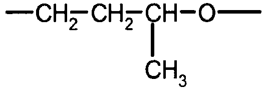 steht,
EO für CH₂-CH₂-O- steht,
r für Zahlen von 1 bis 10 steht und
s für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (XII-f)

in welcher
R' die oben angegebene Bedeutung hat, t für Zahlen von 8 bis 13 steht
u für Zahlen von 6 bis 17 steht.
In den zuvor angegebenen Formeln steht
R vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (XII-c) sei 2-Ethyl-hexyl-alkoxylat der Formel 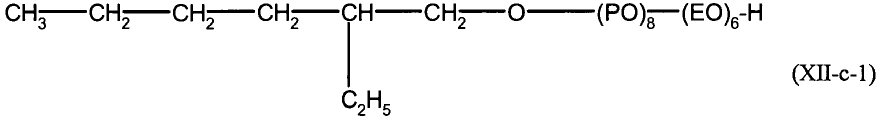 in welcher
EO für -CH₂-CH₂-O- steht,
PO für 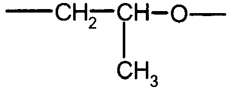 steht und
die Zahlen 8 und 6 Durchschnittswerte darstellen, genannt.

Als Beispiel für ein Alkanol-Alkoxylat der Formel (XII-d) sei die Formel

CH₃-(CH₂)₁₀-O-(-EO-)₆-(-BO-)₂-CH₃ (XII-d-1)

in welcher
EO für CH₂-CH₂-O- steht,
BO für 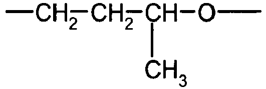 steht und
die Zahlen 10, 6 und 2 Durchschnittswerte darstellen, genannt.

Besonders bevorzugte Alkanol-Alkoxylate der Formel (XII-f) sind Verbindungen dieser Formel, in denen
t für Zahlen von 9 bis 12 und
u für Zahlen von 7 bis 9
steht.

Ganz besonders bevorzugt genannt sei Alkanol-Alkoxylat der Formel (XII-f-1)

CH₃-(CH₂)ₜ-CH₂-O-(-CH₂-CH₂-O-)ᵤ-R' (XII-f-1)

in welcher
t für den Durchschnittswert 10,5 steht und
u für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Löslichkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäß hervorgehobene Kombinationen von Wirkstoff, Salz und Penetrationsförderer, sind in folgender Tabelle aufgeführt. "Gemäß Test" bedeutet dabei, dass jede Verbindung geeignet ist, die in dem Test für die Kutikelpenetration (Baur et al., 1997, Pesticide Science 51, 131-152) als Penetrationsförderer wirkt:

| # | Wirkstoff | Salz | Penetrationsförderer |
|---|---|---|---|
| 1 | I | Ammoniumsulfat | Gemäß Test |
| 2 | I | Ammoniumlaktat | Gemäß Test |
| 3 | I | Ammoniumnitrat | Gemäß Test |
| 4 | I | Ammoniumthiosulfat | Gemäß Test |
| 5 | I | Ammoniumthiocyanat | Gemäß Test |
| 6 | I | Ammoniumcitrat | Gemäß Test |
| 7 | I | Ammoniumoxalat | Gemäß Test |
| 8 | I | Ammoniumformiat | Gemäß Test |
| 9 | I | Ammoniumhydrogenphosphat | Gemäß Test |
| 10 | I | Ammoniumdihydrogenphosphat | Gemäß Test |
| 11 | I | Ammoniumcarbonat | Gemäß Test |
| 12 | I | Ammoniumbenzoat | Gemäß Test |
| 13 | I | Ammoniumsulfit | Gemäß Test |
| 14 | I | Ammoniumbenzoat | Gemäß Test |
| 15 | I | Ammoniumhydrogenoxalat | Gemäß Test |
| 16 | I | Ammoniumhydrogencitrat | Gemäß Test |
| 17 | I | Ammoniumacetat | Gemäß Test |
| 18 | I | Tetramethylammoniumsulfat | Gemäß Test |
| 19 | I | Tetramethylammoniumlaktat | Gemäß Test |
| 20 | I | Tetramethylammoniumnitrat | Gemäß Test |
| 21 | I | Tetramethylammoniumthiosulfat | Gemäß Test |
| 22 | I | Tetramethylammoniumthiocyanat | Gemäß Test |
| 23 | I | Tetramethylammoniumcitrat | Gemäß Test |
| 24 | I | Tetramethylammoniumoxalat | Gemäß Test |
| 25 | I | Tetramethylammoniumformiat | Gemäß Test |
| 26 | I | Tetramethylammoniumhydrogenphosphat | Gemäß Test |
| 27 | I | Tetramethylammoniumdihydrogenphosphat | Gemäß Test |
| 28 | I | Tetraethylammoniumsulfat | Gemäß Test |
| 29 | I | Tetraethylammoniumlaktat | Gemäß Test |
| 30 | I | Tetraethylammoniumnitrat | Gemäß Test |
| 31 | I | Tetraethylammoniumthiosulfat | Gemäß Test |
| 32 | I | Tetraethylammoniumthiocyanat | Gemäß Test |
| 33 | I | Tetraethylammoniumcitrat | Gemäß Test |
| 34 | I | Tetraethylammoniumoxalat | Gemäß Test |
| 35 | I | Tetraethylammoniumformiat | Gemäß Test |
| 36 | I | Tetraethylammoniumhydrogenphosphat | Gemäß Test |
| 37 | I | Tetraethylammoniumdihydrogenphosphat | Gemäß Test |

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoffimprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenem, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.
Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus

Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

### Verfahren (A)

Verwendet man beispielsweise 2-Amino-5-chloro-*N*-isopropyl-3-methyl-benzamid und 2-(3-Chloro-pyridin-2-yl)-5-(5-heptafluoropropyl-tetrazol-2-ylmethyl)-2*H*-pyrazole-3-carbonsäurechlorid als Ausgangsstoffe, so kann der Verlauf des Verfahrens (A) durch das folgende Formelschema veranschaulicht werden.

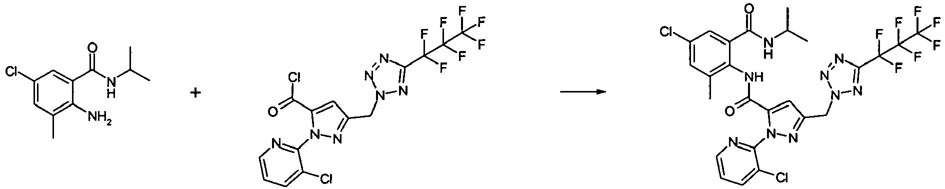

Die bei der Durchführung des Verfahrens (A) als Ausgangsstoffe benötigten Aminobenzamide sind durch die Formel (II) allgemein definiert.

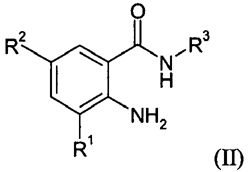

In dieser Formel (II) haben R¹, R² und R³ die oben angegebene Bedeutung.

Das Verfahren (A) wird in Gegenwart eines Säurebindemittels durchgeführt. Hierzu eignen sich alle für solche Kupplungsreaktionen üblichen anorganischen oder organischen Basen. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natrium-methylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, Diisopropylethylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU). Ebenso lassen sich gegebenenfalls polymer-gestützte Säurebindemittel, wie z.B. polymer-gebundenes Diisopropylamin und polymer-gebundenes Dimethylaminopyridin einsetzen.

Das Verfahren (A) kann gegebenenfalls in Gegenwart eines für solche Reaktionen üblichen inerten organischen Verdünnungsmittel durchgeführt werden. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Aminobenzamide der Formel (II) sind bekannt oder können nach bekannten Methoden hergestellt werden (vgl. z.B. M. J. Kornet, J. Heterocyl. Chem. 1992, 29, 103-105; G. P. Lahm et al., Bioorg. Med. Chem. Letters 2005, 15, 4898-4906; WO 2003/016284, WO 2006/055922, WO 2006/062978, WO 2008/010897, WO 2008/070158).

Die bei der Durchführung des Verfahrens (A) als Ausgangsstoffe benötigten Pyrazolcarbonsäurechloride sind durch die Formel (III-1) allgemein definiert.

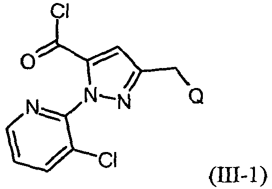

In dieser Formel (III-1) hat Q die oben angegebene Bedeutung.

Pyrazolcarbonsäurechloride der Formel (III-1) sind neu. Sie lassen sich beispielsweise herstellen, indem man

Pyrazolcarbonsäure-Derivate der Formel (IV-1) 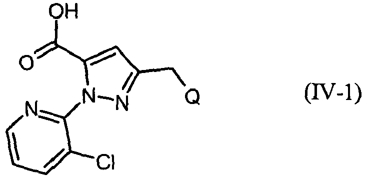 in welcher Q die oben angegebene Bedeutung hat,
mit einem Chlorierungsmittel (z.B. Thionylchlorid und Oxalylchlorid) in Gegenwart eines inerten Verdünnungsmittels (z.B. Toluol und Dichlormethan) in Gegenwart von einer katalytischen Menge von N,N-Dimethylformamid umsetzt.

Pyrazolcarbonsäure-Derivate der Formel (IV-1) sind neu. Sie lassen sich beispielsweise herstellen, indem man

Pyrazolcarbonsäureester der Formel (VI-1) 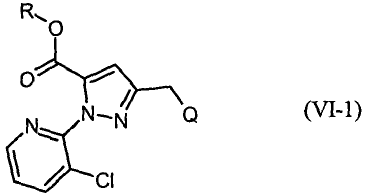 in welcher Q die oben angegebene Bedeutungen hat und R für C₁-C₆-Alkyl steht,
mit einem Alkalimetallhydroxid (z.B. Natriumhydroxid oder Kaliumhydroxid) in Gegenwart eines inerten Verdünnungsmittels (z.B. Dioxan/Wasser oder Ethanol/Wasser) umsetzt.

Pyrazolcarbonsäureester der Formel (VI) sind neu. Sie lassen sich beispielsweise herstellen, indem man

Pyrazolcarbonsäureester-Derivate der Formel (VII-1) 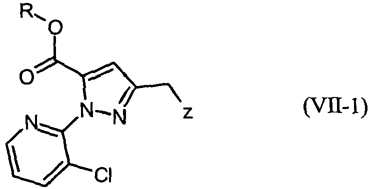 in welcher R die oben angegebene Bedeutung hat und Z für Chlor, Brom, Iod, Methylsulfonyl oder Toluolsulfonyl steht, mit einem Tetrazol der Formel (VIII), in welcher Q die oben angegebene Bedeutung hat, in Gegenwart einer Base (z. B. Natriumhydroxid, Kaliumcarbonat, Natriumcarbonat, Cäsiumcarbonat, Natriummethylat, Triethylamin oder Natriumhydrid) in Gegenwart eines Lösungsmittels (z.B. Tetrahydrofuran, Toluol, Aceton, Acetonitril, Methanol, Dimethylformamid oder Dioxan) umsetzt.

Q-H (VIII)

Tetrazole der Formel (VIII) sind bekannt, teilweise sogar kommerziell erhältlich oder können nach bekannten Verfahren erhalten werden (vgl. z.B. WO2004/020445; William P. Norris, J. Org. Chem., 1962, 27 (9), 3248-3251; Henry C. Brown, Robert J. Kassal, J. Org. Chem., 1967, 32 (6), 1871-1873; Dennis P. Curran, Sabine Hadida, Sun-Young Kim, Tetrahedron, 1999, 55 (29), 8997-9006; L.D. Hansen, E.J. Baca, P. Scheiner, Journal of Heterocyclic Chemistry, 1970, 7, 991-996).

Pyrazolcarbonsäureester-Derivate der Formel (VII) sind bekannt oder können nach bekannten Verfahren erhalten werden (vgl. z.B. WO2007/144100)

### Verfahren (B)

Verwendet man beispielsweise 2-Amino-5-chloro-*N*-isopropyl-3-methyl-benzamid und 2-(3-Chloro-pyridin-2-yl)-5-(5-heptafluoropropyl-tetrazol-2-ylmethyl)-2*H*-pyrazole-3-carbonsäure als Ausgangsstoffe, so kann der Verlauf des Verfahrens (B) durch das folgende Formelschema veranschaulicht werden.

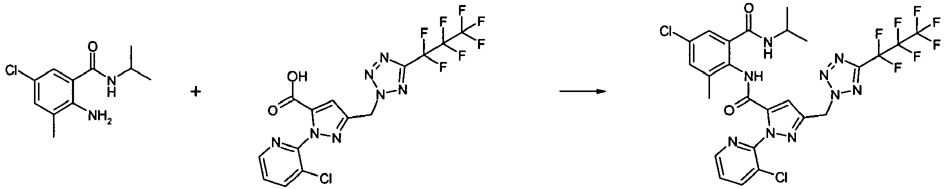

Die bei der Durchführung des Verfahrens (B) als Ausgangsstoffe benötigten Anthranilamide der Formel (II) sind bereits in Zusammenhang mit dem Verfahren (A) beschrieben worden.

Die bei der Durchführung des Verfahrens (B) weiterhin als Ausgangsstoffe benötigten Pyrazolcarbonsäuren sind durch die Formel (IV-1) allgemein definiert.

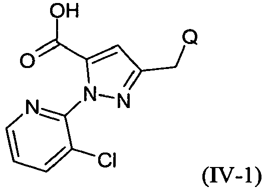

In dieser Formel (IV-1) hat Q die oben angegebene Bedeutung.

Das Verfahren (B) wird in Gegenwart eines Kondensationsmittels durchgeführt. Hierzu eignen sich alle für solche Kupplungsreaktionen üblichen Mittel. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, 1,1'-Carbonyldiinüdazol, 2-Ethoxy-N-ethoxycarbonyl-1,2-dihydrochinolin (EEDQ), Triphenylphosphin/Tetrachlorkohlenstoff, Brom-tripyrrolidinophosphonium-hexafluorophosphat, Bis(2-oxo-3-oxazolidinyl)-phosphinchlorid oder Benzotriazol-1-yloxytris(dimethylamino)-phosphonium-hexafluorphosphat. Auf Polymer gestützte Reagenzien, wie z.B. polymer-gebundenes Cyclohexylcarbodiimid, können ebenfalls verwendet werden.

Das Verfahren (B) wird gegebenenfalls in Gegenwart eines Katalysators durchgeführt. Beispielsweise genannt seien 4-Dimethylaminopyridin, 1-Hydroxy-benzotriazol oder Dimethylformamid.

Das Verfahren (B) kann gegebenenfalls in Gegenwart eines für solche Reaktionen üblichen inerten organischen Verdünnungsmittel durchgeführt werden. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

### Verfahren (C)

Verwendet man 6-Chloro-2-[2-(3-chloro-pyridin-2-yl)-5-(5-heptafluoropropyl-tetrazol-2-ylmethyl)-2*H*-pyrazol-3-yl]-8-methyl-benzo[d][1,3]oxazin-4-on und Isopropylamin, so kann der Verlauf des Verfahrens (C) durch das folgende Formelschema veranschaulicht werden.

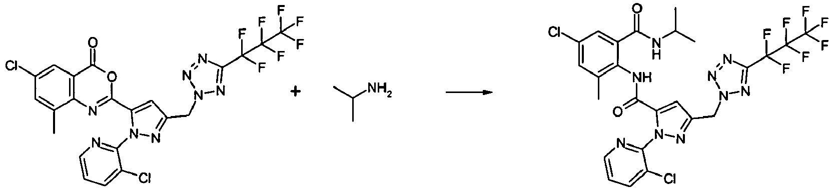

Die bei der Durchführung des Verfahrens (C) als Ausgangsstoffe benötigten Benzoxazinone sind durch die Formel (V-1) allgemein definiert.

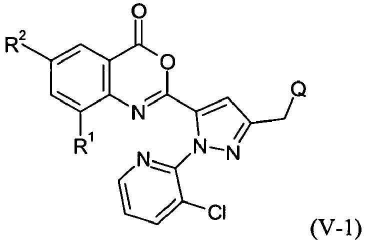

In dieser Formel (V-1) haben R¹, R² und Q die oben angegebene Bedeutung.

Benzoxazinone der Formel (V-1) sind neu. Sie werden beispielsweise erhalten, indem man Pyrazolcarbonsäure-Derivate der Formel (IV-1) 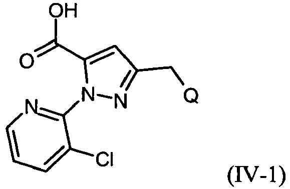 in welcher Q die oben angegebene Bedeutung hat,
mit Anthranilsäure der Formel (IX) 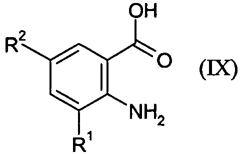 in welcher R¹ und R² die oben angegebenen Bedeutungen haben, in Anwesenheit einer Base (z.B. Triethylamin oder Pyridin) und in Gegenwart eines Sulfonsäurechlorids (z.B. Methansulfonsäurechlorid) sowie gegebenenfalls in Gegenwart eines Verdünnungsmittels (z.B. Acetonitril) umsetzt.

Die bei der Durchführung des Verfahrens als Ausgangsstoffe benötigten Pyrazolcarbonsäure-Derivate der Formel (IV-1) sind bereits oben in Zusammenhang mit dem Verfahren (A) beschrieben worden.

Anthranilsäuren der Formel (IX) sind bekannt oder können nach allgemeinen Synthesemethoden hergestellt werden (vgl. z. B. Baker et al. J. Org. Chem. 1952, 149-153; G. Reissenweber et al., Angew. Chem 1981, 93, 914-915, P.J. Montoya-Pelaez, J. Org. Chem. 2006, 71, 5921-5929; F. E. Sheibley, J. Org. Chem. 1938, 3, 414-423, WO 2006023783).

### Verfahren (D)

Verwendet man beispielsweise 2-Amino-N-tert-butyl-5-chlor-3-methylbenzamid und 1-(3-Chlorpyridin-2-yl)-3-{1-[5-(trifluormethyl)-2H-tetrazol-2-yl]ethyl}-1H-pyrazole-5-carbonsäure als Ausgangsstoffe, so kann der Verlauf des Verfahrens (D) durch das folgende Formelschema veranschaulicht werden.

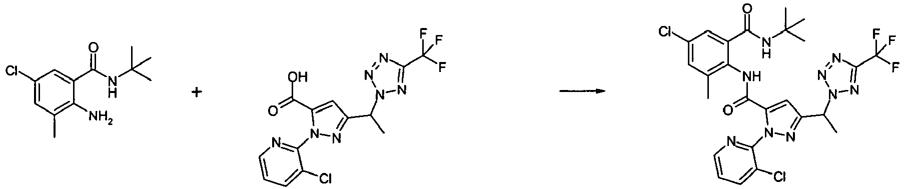

Die bei der Durchführung des Verfahrens (D) als Ausgangsstoffe benötigten Anthranilamide der Formel (II) sind bereits in Zusammenhang mit dem Verfahren (A) beschrieben worden.

Die bei der Durchführung des Verfahrens (D) weiterhin als Ausgangsstoffe benötigten Pyrazolcarbonsäuren sind durch die Formel (IV) allgemein definiert.

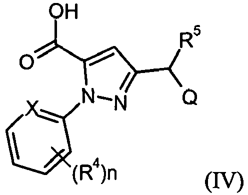

In dieser Formel (IV) haben X, Q, R⁴, R⁵ und n die oben angegebene Bedeutung.

Das Verfahren (D) wird in Gegenwart eines Kondensationsmittels durchgeführt. Hierzu eignen sich alle für solche Kupplungsreaktionen üblichen Mittel. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodümid (DCC) oder andere übliche Kondensationsmittel, wie Phosphorpentoxid, Polyphosphorsäure, 1,1'-Carbonyldiimidazol, 2-Ethoxy-N-ethoxycarbonyl-1,2-dihydrochinolin (EEDQ), Triphenylphosphin/Tetrachlorkohlenstoff, Brom-tripyrrolidinophosphonium-hexafluorophosphat, Bis(2-oxo-3-oxazolidinyl)-phosphinchlorid oder Benzotriazol-1-yloxytris(dimethylamino)-phosphonium-hexafluorphosphat. Auf Polymer gestützte Reagenzien, wie z.B. polymer-gebundenes Cyclohexylcarbodiimid, können ebenfalls verwendet werden.

Das Verfahren (D) wird gegebenenfalls in Gegenwart eines Katalysators durchgeführt. Beispielsweise genannt seien 4-Dimethylaminopyridin, 1-Hydroxy-benzotriazol oder Dimethylformamid.

Das Verfahren (D) kann gegebenenfalls in Gegenwart eines für solche Reaktionen üblichen inerten organischen Verdünnungsmittel durchgeführt werden. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Pyrazolcarbonsäuren der Formel (IV) sind neu. Sie lassen sich beispielsweise herstellen, indem man

### Pyrazolcarbonsäureester der Formel (XIII)

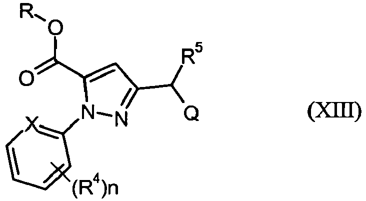

in welcher X, Q, R⁴, R⁵ und n die die oben angegebene Bedeutungen haben und R für C₁-C₆-Alkyl steht,
mit einem Alkalimetallhydroxid (z.B. Natriumhydroxid oder Kaliumhydroxid) in Gegenwart eines inerten Verdünnungsmittels (z.B. Dioxan/Wasser oder Ethanol/Wasser) umsetzt.

Pyrazolcarbonsäureester der Formel (XIII) sind neu. Sie lassen sich beispielsweise herstellen, indem man

Pyrazolcarbonsäureester-Derivate der Formel (XIV) 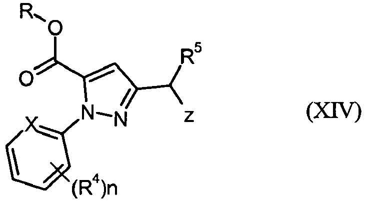 in welcher X, Q, R, R⁴, R⁵ und n die oben angegebene Bedeutung haben und Z für Chlor, Brom, Iod, Methylsulfonyl oder Toluolsulfonyl steht, mit einem Tetrazol der Formel (VIII), in welcher Q die oben angegebene Bedeutung hat, in Gegenwart einer Base (z. B. Natriumhydroxid, Kaliumcarbonat, Natriumcarbonat, Cäsiumcarbonat, Natriummethylat, Triethylamin oder Natriumhydrid) in Gegenwart eines Lösungsmittels (z.B. Tetrahydrofuran, Toluol, Aceton, Acetonitril, Methanol, Dimethylformamid oder Dioxan) umsetzt.

Q-H (VIII)

Tetrazole der Formel (VIII) sind bekannt, teilweise sogar kommerziell erhältlich oder können nach bekannten Verfahren erhalten werden (vgl. z.B. WO2004/020445; William P. Norris, J. Org. Chem., 1962, 27 (9), 3248-3251; Henry C. Brown, Robert J. Kassal, J. Org. Chem., 1967, 32 (6), 1871-1873; Dennis P. Curran, Sabine Hadida, Sun-Young Kim, Tetrahedron, 1999, 55 (29), 8997-9006; L.D. Hansen, E.J. Baca, P. Scheiner, Journal of Heterocyclic Chemistry, 1970, 7, 991-996).

Pyrazolcarbonsäureester der Formel (XIV) sind neu. Sie lassen sich beispielsweise herstellen, indem man

### Alkohol-Derivate der Formel (XV)

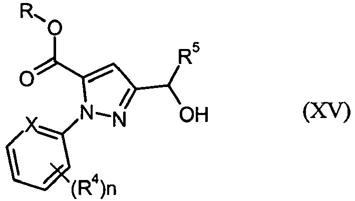

in welcher X, R, R⁴, R⁵ und n die oben angegebene Bedeutung haben mit einem Sulfonylchlorid (z.B. Methylsulfonylchlorid oder Toluolsulfonylchlorid) oder einem Halogenierungsmittel (z.B. Thionylchlorid) gegebenenfalls unter Anwesenheit eines Lösungsmittels (z.B. Dichlormethan) und gegebenenfalls unter Anwesenheit einer Base (z.B. Triethylamin oder Pyridin) umsetzt.

### Alkohol-Derivate der Formel (XV) sind neu. Sie lassen sich beispielsweise herstellen, indem man Keton-Derivate der Formel (XVI)

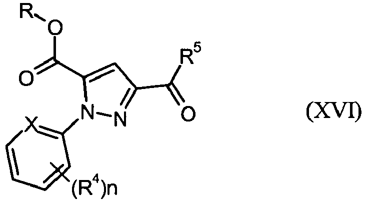

in welcher X, R, R⁴, R⁵ und n die oben angegebene Bedeutung haben mit einem geeigneten Reduktionsmittel (z.B. Natriumborhydrid) in Gegenwart eines Lösungsmittels (z.B. Ethanol) umsetzt.

### Keton-Derivate der Formel (XVI) sind neu. Sie lassen sich beispielsweise herstellen, indem man Pyrazol-Derivate der Formel (XVII)

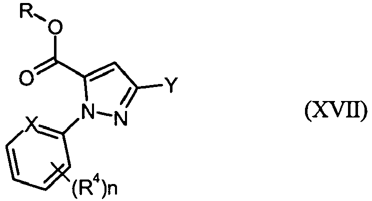

in welcher X, R, R⁴ und n die oben angegebene Bedeutung haben und Y für Chlor oder Brom steht mit einem Zinnderivat der Formel (XVIII), in welchem R⁷ für H bzw. C₁-C₃-Alkyl steht in Gegenwart eines Übergangmetalls (z.B. Tetrakis(triphenylphosphin)palladium(0)) und eines Salzes (z.B Lithiumchlorid) in Gegenwart eines Lösungsmittels (z.B. Tetrahydrofuran) umsetzt.

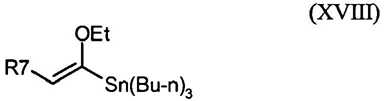

Zinnderivate der Formel (XVIII) sind bekannt bzw. kommerziell erhältlich.

Pyrazol-Derivate der Formel (XVII) sind bekannt oder können nach bekannten Verfahren erhalten werden (vgl. z.B. WO2004/033468, WO2003/015518, WO2003/016283).

### Herstellungsbeispiele

(erfindungsgemäße Verbindungen und Referenzverbindungen) Verbindungen: Synthese von 2-(3-Chloro-pyridin-2-yl)-5-(5-heptafluoropropyl-tetrazol-2-ylmethyl)-2*H*-pyrazol-3-carbonsäure (4-chloro-2-isopropylcarbamoyl-6-methyl-phenyl)-amid (Beispiel 1)

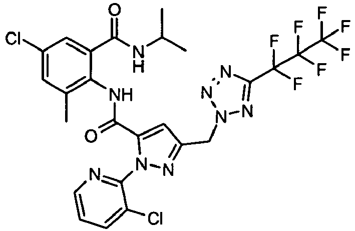

200 mg (0.3 mmol) 6-Chloro-2-[2-(3-chloro-pyridin-2-yl)-5-(5-heptafluoropropyl-tetrazol-2-ylmethyl)-2*H*-pyrazol-3-yl]-8-methyl-benzo[*d*][1,3]oxazin-4-on wurden in 2 ml Tetrahydrofuran vorgelegt und mit 0.08 ml (1 mmol) Isopropylamin versetzt. Die Mischung wurde 1 h bei 50 °C gerührt und nach dem Abkühlen eingeengt. Nach dem Reinigen des Rückstands durch Kristallisation oder chromatographische Trennung konnte das gewünschte Produkt erhalten werden (logP: 4.23, MH⁺: 682, ¹H-NMR (400 MHz, DMSO, δ, ppm): 1.02 (d, 6H), 2.14 (s, 3H), 3.91 (m, 1H), 6.30 (s, 2H), 7.30 (m, 2H), 7.40 (d, 1H), 7.55 (dd, 1H), 7.77 (d, 1H), 8.08 (dd, 1H), 8.44 (dd, 1H), 10.07 (s, 1H).

Die folgenden Beispiele können auf analoge Art und Weise erhalten werden:

Angegeben sind dabei für das Beispiel Nummer 1 die vollständigen NMR-Signale, für die weiteren Beispiele eine Kombination aus logP-Wert, Masse (MH⁺) und diejenigen NMR-Signale, die sich auf den im Verfahren zuletzt eingeführten Molekülteil beziehen.

| **Beispielnr.** | **Struktur** | **logP** | **MH⁺** | **NMR** |
|---|---|---|---|---|
| 1 | 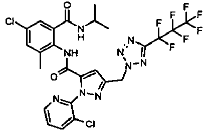 | 4.23 | 682 | DMSO: 1.02 (d, 6H, NHCH(CH₃)₂), 3.91 (m, 1H, NHCH(CH₃)₂) |
| 2 | 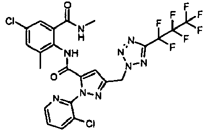 | 3.75 | 654 | DMSO: 2.67 (d, 3H, NHCH₃) |
| 3 | 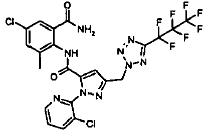 | 3.49 | 640 | DMSO: 7.40 (bs, 2H, N*H*₂) |
| 4 | 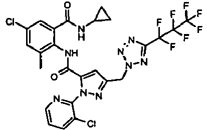 | 3.96 | 680 | DMSO: 0.43 (m, 2H, NHCH(C*H*₂)₂), 0.60 (m, 2H, NHCH(C*H*₂)₂), 2.69 (m, 1H, NHC*H*(CH₂)₂) |
| 5 | 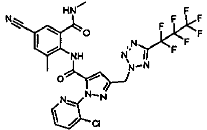 | 3.43 | 645 | DMSO: 2.68 (d, 3H, NHC*H*₃) |
| 6 | 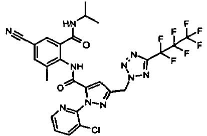 | 3.82 | 673 | DMSO: 1.04 (d, 6H, NHCH(C*H*₃)₂), 3.92 (m, 1H, NHC*H*(CH₃)₂) |
| 7 | 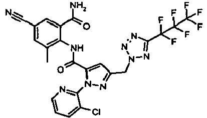 | 3.17 | 631 | DMSO: 7.43 (bs, 1H, N*H*₂), 7.67 (bs, 1 H, N*H*₂) |
| 8 | 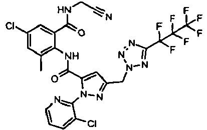 | 3.65 | 679 | DMSO: 4.15 (d, 2H, NHC*H*₂CN) |
| 9 | 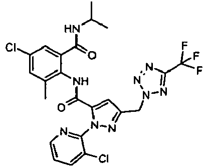 | 3.50 | 580 (M-H⁺) | DMSO: 1.02 (d, 6H, NHCH(C*H*₃)₂), 3.91 (m, 1H, NHC*H*(CH₃)₂) |
| 10 | 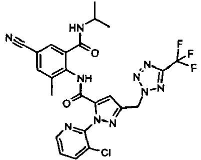 | 3.07 | 571 (M-H⁺) | DMSO: 1.04 (d, 6H, NHCH(C*H*₃)₂), 3.92 (m, 1H, NHC*H*(CH₃)₂) |
| 11 | 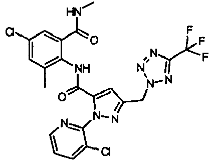 | 3.05 | 552 (M-H⁺) | DMSO: 2.67 (d, 3H, NHC*H*₃) |
| 12 | 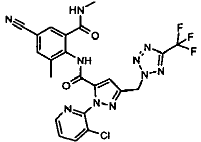 | 2.66 | 545 | DMSO: 2.68 (d, 3H, NHC*H*3) |
| 13 | 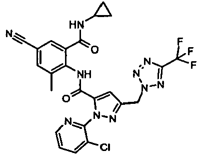 | 2.84 | 569 (M-H⁺) | CD₃CN: 0.53 (m, 2H, NHCH(C*H*₂)₂), 0.72 (m, 2H, NHCH(C*H*₂)₂), 2.77 (m, 1H, NHC*H*(CH₂)₂) |
| 14 | 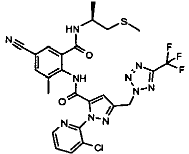 | 3.28 | 617 (M-H⁺) | CD₃CN: 1.22 (d, 3H, NHCH(C*H*₃)CH₂SCH₃), 2.20 (s, 3H, NHCH(CH₃)CH₂SC*H*₃), 2.61 (d, 2H, NHCH(CH₃)C*H*₂SCH₃), 4.16 (m, 1H, NHC*H*(CH₃)CH₂SCH₃) |
| 15 | 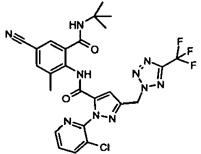 | 3.40 | 585 (M-H⁺) | DMSO: 1.22 (s, 9H, NHC(C*H*₃)₃) |
| 16 | 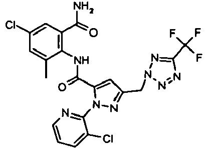 | 2.77 | 540 | CD₃CN: 6.30 (bs, 2H, N*H*₂) |
| 17 | 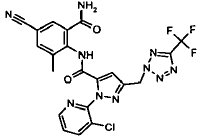 | 2.45 | 531 | CD₃CN: 6.33 (bs, 1H, N*H*₂), 6.85 (bs, 1H, N*H*₂) |
| 18 | 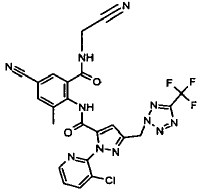 | 2.61 | 570 | CD₃CN: 4.16 (d, 2H, NHC*H*₂CN) |
| 19 | 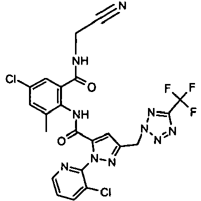 | 2.93 | 579 | CD₃CN: 4.14 (d, 2H, NHC*H*₂CN) |
| 20 | 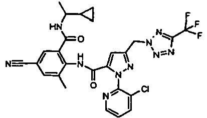 | 3.33 | 599 | DMSO: 0.11-0.38 (m, 4H, NHCH(CH₃)CH(C*H*₂)₂), 0.84 (m, 1H, NHCH(CH₃)C*H*(CH₂)₂), 1.06 (dd, 3H, NHCH(C*H*₃)CH(CH₂)₂), 3.34 (m, 1H, NHC*H*(CH₃)CH(CH₂)₂) |
| 21 | 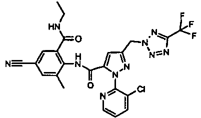 | 2.85 | 559 | DMSO: 1.01 (t, 3H, NHCH₂C*H*₃), 3.17 (m, 2H, NHC*H*₂CH₃) |
| 22 | 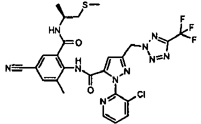 | 3.27 | 619 | DMSO: 1.10 (d, 3H, NHCH(C*H*₃)CH₂SCH₃), 2.20 (s, 3H, NHCH(CH₃)CH₂SC*H*₃), 2.49 (m, 1H, NHCH(CH₃)C*H*₂SCH₃), 2.57 (m, 1H, NECH(CH₃)C*H*₂SCH₃), 4.00 (m, 1H, NHC*H*(CH₃)CH₂SCH₃) |
| 23 | 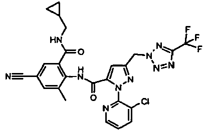 | 3.13 | 585 | DMSO: 0.12 (m, 2H, NHCH₂C*H*(CH₂)₂), 0.33 (m, 2H, NHCH₂CH(C*H*₂)₂), 0.88 (m, 1H, NHCH₂C*H*(CH₂)₂), 3.03 (t, 2H, NHC*H*₂CH(CH₂)₂) |
| 24 | 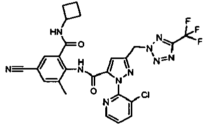 | 3.18 | 585 | DMSO: 1.61 (m, 2H, NHCH(C*H*₂)₃), 1.95 (m, 2H, NHCH(C*H*₂)₃), 2.15 (m, 2H, NHCH(C*H*₂)₃), 4.22 (m, 1H, NHC*H*(CH₂)₃) |
| 25 | 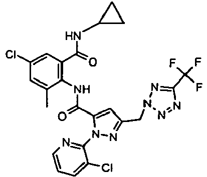 | 3.23 | 580 | DMSO: 0.44 (m, 2H, NHCH(C*H*₂)₂), 0.59 (m, 2H, NHCH(C*H*₂)₂), 2.70 (m, 1H, NHC*H*(CH₂)₂) |
| 26 | 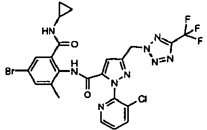 | 3.30 | 624 | DMSO: 0.44 (m, 2H, NHCH(C*H*₂)₂), 0.59 (m, 2H, NHCH(C*H*₂)₂), 2.70 (m, 1H, NHC*H*(CH₂)₂) |
| 27 | 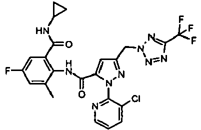 | 2.87 | 564 | DMSO: 0.43 (m, 2H, NHCH(C*H*₂)₂), 0.60 (m, 2H, NHCH(C*H*₂)₂), 2.69 (m, 1H, NHC*H*(CH₂)₂) |
| 28 | 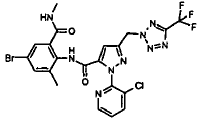 | 3.05 | 598 | DMSO: 2.63 (d, 3H, NHC*H*₃) |
| 29 | 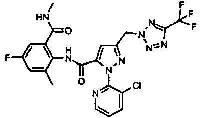 | 2.66 | 538 | DMSO: 2.67 (d, 3H, NHC*H*₃) |
| 30 | 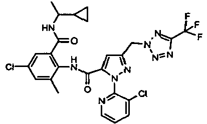 | 3.79 | 608 | DMSO: 0.09-0.37 (m, 4H, NHCH(CH₃)CH(C*H*₂)₂), 0.82 (m, 1H, NHCH(CH₃)C*H*(CH₂)₂), 1.05 (d, 3H, NHCH(C*H*₃)CH(CH₂)₂), 3.33 (m, 1H, NHC*H*(CH₃)CH(CH₂)₂) |
| 31 | 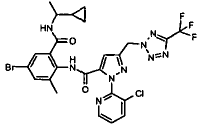 | 3.89 | 652 | DMSO: 0.09-0.37 (m, 4H, NHCH(CH₃)CH(C*H*₂)₂), 0.81 (m, 1H, NHCH(CH₃)C*H*(CH₂)₂), 1.05 (d, 3H, NHCH(C*H*₃)CH(CH₂)₂), 3.32 (m, 1H, NHC*H*(CH₃)CH(CH₂)₂) |
| 32 | 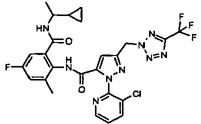 | 3.41 | 592 | DMSO: 0.08-0.36 (m, 4H, NHCH(CH₃)CH(C*H*₂)₂), 0.83 (m, 1H, NHCH(CH₃)C*H*(CH₂)₂), 1.05 (d, 3H, NHCH(C*H*₃)CH(CH₂)₂), 3.33 (m, 1H, NHC*H*(CH₃)CH(CH₂)₂) |
| 33 | 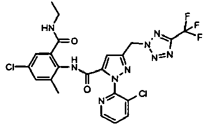 | 3.23 | 568 | DMSO: 1.00 (t, 3H, NHCH₂C*H*₃), 3.15 (m, 2H, NHC*H*₂CH₃) |
| 34 | 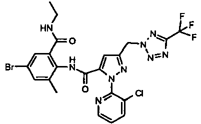 | 3.32 | 612 | DMSO: 1.00 (t, 3H, NHCH₂C*H*₃), 3.14 (m, 2H, NHC*H*₂CH₃) |
| 35 | 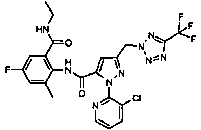 | 2.88 | 552 | DMSO: 1.00 (t, 3H, NHCH₂C*H*₃), 3.15 (m, 2H, NHC*H*₂CH₃) |
| 36 | 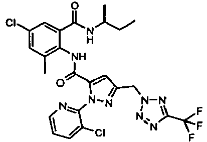 | 3.74 | 596 | DMSO: 0.75 (t, 3H, NHCH(CH₃)CH₂C*H*₃), 0.96 (d, 3H, NHCH(C*H*₃)CH₂CH₃), 1.30-1.39 (m, 2H, NHCH(CH₃)C*H*₂CH₃), 3.69-3.74 (m, 1H, NHC*H*(CH₃)CH₂CH₃) |
| 37 | 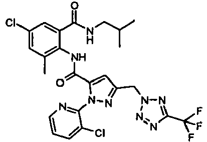 | 3.78 | 596 | DMSO: 0.79 (d, 6H, NHCH₂CH(C*H*₃)₂), 1.67-1.76 (m, 1H, NHCH₂C*H*(CH₃)₂), 2.94-2.97 (dd, 2H, NHC*H*₂CH(CH₃)₂) |
| 38 | 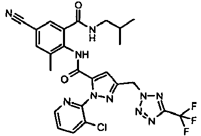 | 3.37 | 587 | DMSO: 0.81 (d, 6H, NHCH₂CH(C*H*₃)₂), 1.69-1.75 (m, 1H, NHCH₂C*H*(CH₃)₂), 2.95-2.97 (dd, 2H, NHC*H*₂CH(CH₃)₂) |
| 39 | 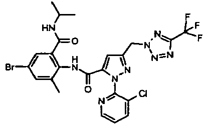 | 3.57 | 626 | DMSO: 1.02 (d, 6H, NHCH(C*H*₃)₂), 3.90 (m, 1H, NHC*H*(CH₃)₂) |
| 40 | 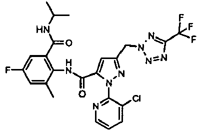 | 3.11 | 566 | DMSO: 1.01 (d, 6H, NHCH(C*H*₃)₂), 3.90 (m, 1H, NHC*H*(CH₃)₂) |
| 41 | 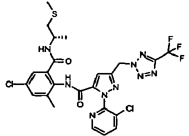 | 3.68 | 628 | DMSO: 1.10 (d, 3H, NHCH(C*H*₃)CH₂SCH₃), 2.15 (s, 3H, NHCH(CH₃)CH₂SC*H*₃), 2.47 (m, 1H, NHCH(CH₃)C*H*₂SCH₃), 2.54 (m, 1H, NHCH(CH₃)C*H*₂SCH₃), 3.98 (m, 1H, NHC*H*(CH₃)CH₂SCH₃) |
| 42 | 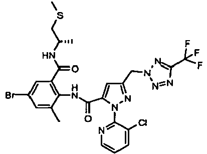 | 3.77 | 672 | DMSO: 1.10 (d, 3H, NHCH(C*H*₃)CH₂SCH₃), 2.15 (s, 3H, NHCH(CH₃)CH₂SC*H*₃), 2.47 (m, 1H, NHCH(CH₃)C*H*₂SCH₃), 2.54 (m, 1H, NHCH(CH₃)C*H*₂SCH₃), 3.97 (m, 1H, NHC*H*(CH₃)CH₂SCH₃) |
| 43 | 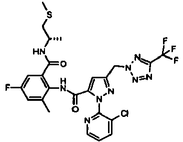 | 3.31 | 612 | DMSO: 1.09 (d, 3H, NHCH(C*H*₃)CH₂SCH₃), 2.16 (s, 3H, NHCH(CH₃)CH₂SC*H*₃), 2.44 (m, 1H, NHCH(CH₃)C*H*₂SCH₃), 2.54 (m, 1H, NHCH(CH₃)C*H*₂SCH₃), 3.98 (m, 1H, NHC*H*(CH₃)CH₂SCH₃) |
| 44 | 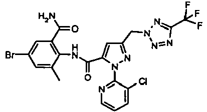 | 2.81 | 584 | DMSO: 7.32 (bs, 1H, N*H*₂), 7.53 (bs, 1H, N*H*₂) |
| 45 | 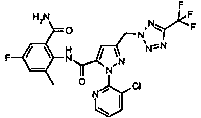 | 2.46 | 524 | DMSO: 7.37 (bs, 2H, N*H*₂) |
| 46 | 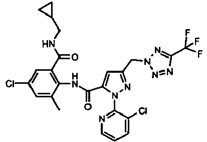 | 3.53 | 594 | DMSO: 0.11 (m, 2H, NHCH₂CH(C*H*₂)₂), 0.31 (m, 2H, NHCH₂CH(C*H*₂)₂), 0.87 (m, 1H, NHCH₂C*H*(CH₂)₂), 3.01 (t, 2H, NHC*H*₂CH(CH₂)₂) |
| 47 | 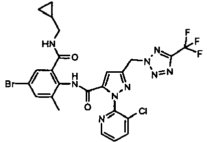 | 3.88 | 638 | DMSO: 0.10 (m, 2H, NHCH₂CH(C*H*₂)₂), 0.31 (m, 2H, NHCH₂CH(C*H*₂)₂), 0.88 (m, 1H, NHCH₂C*H*(CH₂)₂), 3.01 (t, 2H, NHC*H*₂CH(CH₂)₂) |
| 48 | 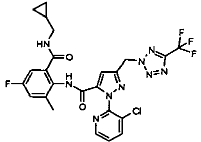 | 3.18 | 578 | DMSO: 0.11 (m, 2H, NHCH₂CH(C*H*₂)₂), 0.30 (m, 2H, NHCH₂CH(C*H*₂)₂), 0.88 (m, 1H, NHCH₂C*H*(CH₂)₂), 3.02 (t, 2H, NHC*H*₂CH(CH₂)₂) |
| 49 | 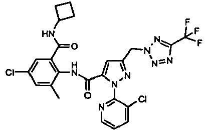 | 3.60 | 594 | DMSO: 1.62 (m, 2H, NHCH(C*H*₂)₃), 1.98 (m, 2H, NHCH(C*H*₂)₃), 2.10 (m, 2H, NHCH(C*H*₂)₃), 4.22 (m, 1H, NHC*H*(CH₂)₃) |
| 50 | 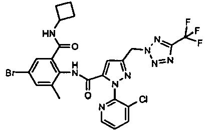 | 3.71 | 638 | DMSO: 1.62 (m, 2H, NHCH(C*H*₂)₃), 1.90 (m, 2H, NHCH(C*H*₂)₃), 2.10 (m, 2H, NHCH(C*H*₂)₃), 4.21 (m, 1H, NHC*H*(CH₂)₃) |
| 51 | 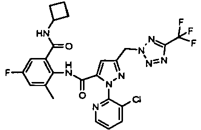 | 3.23 | 578 | DMSO: 1.61 (m, 2H, NHCH(C*H*₂)₃), 1.88 (m, 2H, NHCH(C*H*₂)₃), 2.10 (m, 2H, NHCH(C*H*₂)₃), 4.22 (m, 1H, NHC*H*(CH₂)₃) |
| 52 | 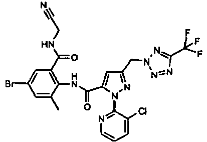 | 3.02 | 623 | DMSO: 4.14 (d, 2H, NHC*H*₂CN) |
| 53 | 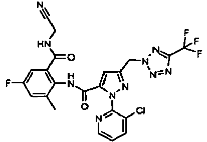 | 2.67 | 563 | DMSO: 4.15 (d, 2H, NHC*H*₂CN) |
| 54 | 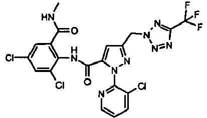 | 2.93 | 575 | DMSO: 2.68 (d, 3H, NHC*H*₃) |
| 55 | 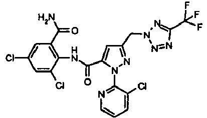 | 2.72 | 560 | DMSO: 7.42 (bs, 1H, N*H*₂), 7.53 (bs, 1 H, N*H*₂) |
| 56 | 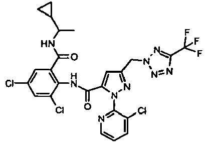 | 3.71 | 628 | DMSO: 0.09-0.37 (m, 4H, NHCH(CH₃)CH(C*H*₂)₂), 0.82 (m, 1H, NHCH(CH₃)C*H*(CH₂)₂), 1.04 (d, 3H, NHCH(C*H*₃)CH(CH₂)₂), 3.30 (m, 1H, NHC*H*(CH₃)CH(CH₂)₂) |
| 57 | 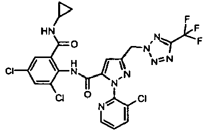 | 3.18 | 600 | DMSO: 0.42 (m, 2H, NHCH(C*H*₂)₂), 0.60 (m, 2H, NHCH(C*H*₂)₂), 2.66 (m, 1H, NHC*H*(CH₂)₂) |
| 58 | 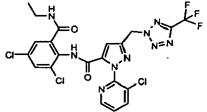 | 3.17 | 588 | DMSO: 0.98 (t, 3H, NHCH₂C*H*₃), 3.14 (m, 2H, NHC*H*₂CH₃) |
| 59 | 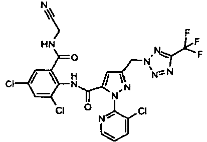 | 2.92 | 599 | DMSO: 4.15 (d, 2H, NHC*H*₂CN) |
| 60 | 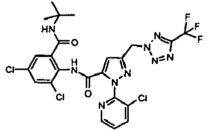 | 3.78 | 616 | DMSO: 1.26 (s, 9H, NHC(C*H*₃)₃) |
| 61 | 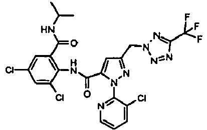 | 3.41 | 602 | DMSO: 1.01 (d, 6H, NHCH(C*H*₃)₂), 3.88 (m, 1H, NHC*H*(CH₃)₂) |
| 62 | 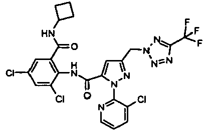 | 3.51 | 614 | DMSO: 1.62 (m, 2H, NHCH(C*H*₂)₃), 1.87 (m, 2H, NHCH(C*H*₂)₃), 2.11 (m, 2H, NHCH(C*H*₂)₃), 4.18 (m, 1H, NHC*H*(CH₂)₃) |
| 63 | 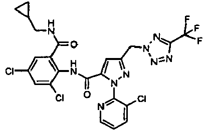 | 3.47 | 614 | DMSO: 0.11 (m, 2H, NHCH₂CH(C*H*₂)₂), 0.30 (m, 2H, NHCH₂CH(C*H*₂)₂), 0.85 (m, 1H, NHCH₂CH(C*H*₂)₂), 2.99 (t, 2H, NHC*H*₂CH(CH₂)₂) |
| 64 | 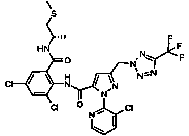 | 3.62 | 648 | DMSO: 1.09 (d, 3H, NHCH(C*H*₃)CH₂SCH₃), 2.00 (s, 3H, NHCH(CH₃)CH₂SC*H*₃), 2.44 (m, 1H, NHCH(CH₃)C*H*₂SCH₃), 2.53 (m, 1H, NHCH(CH₃)C*H*₂SCH₃), 3.94 (m, 1H, NHC*H*(CH₃)CH₂SCH₃) |
| 65 | 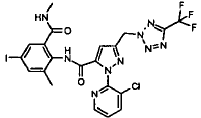 | 3.21 | 646 | DMSO: 2.65 (d, 3H, NHC*H*₃) |
| 66 | 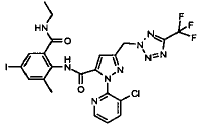 | 3.47 | 660 | DMSO: 1.00 (t, 3H, NHCH₂C*H*₃), 3.14 (m, 2H, NHC*H*₂CH₃) |
| 67 | 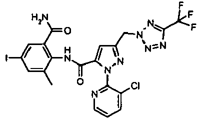 | 2.95 | 632 | DMSO: 7.31 (bs, 1H, N*H*₂), 7.51 (bs, 1H, N*H*₂) |
| 68 | 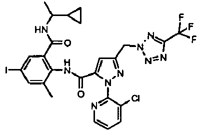 | 4.01 | 700 | DMSO: 0.10-0.37 (m, 4H, NHCH(CH₃)CH(C*H*₂)₂), 0.81 (m, 1H, NHCH(CH₃)C*H*(CH₂)₂), 1.05 (d, 3H, NHCH(C*H*₃)CH(CH₂)₂), 3.32 (m, 1H, NHC*H*(CH₃)CH(CH₂)₂) |
| 69 | 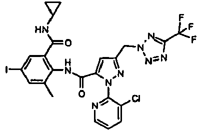 | 3.46 | 672 | DMSO: 0.43 (m, 2H, NHCH(C*H*₂)₂), 0.59 (m, 2H, NHCH(C*H*₂)₂), 2.68 (m, 1H, NHC*H*(CH₂)₂) |
| 70 | 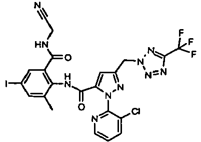 | 3.14 | 671 | DMSO: 4.14 (d, 2H, NHC*H*₂CN) |
| 71 | 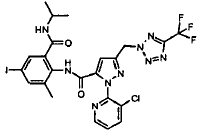 | 3.73 | 674 | DMSO: 1.02 (d, 6H, NHCH(C*H*₃)₂), 3.90 (m, 1H, NHC*H*(CH₃)₂) |
| 72 | 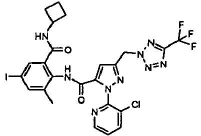 | 3.85 | 686 | DMSO: 1.63 (m, 2H, NHCH(C*H*₂)₃), 1.91 (m, 2H, NHCH(C*H*₂)₃), 2.12 (m, 2H, NHCH(C*H*₂)₃), 4.21 (m, 1H, NHC*H*(CH₂)₃) |
| 73 | 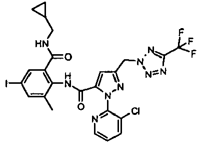 | 3.78 | 686 | DMSO: 0.12 (m, 2H, NHCH₂CH(C*H*₂)₂), 0.31 (m, 2H, NHCH₂CH(C*H*₂)₂), 0.88 (m, 1H, NHCH₂C*H*(CH₂)₂), 3.01 (t, 2H, NHC*H*₂CH(CH₂)₂) |
| 74 | 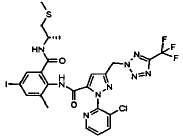 | 3.90 | 720 | DMSO: 1.10 (d, 3H, NHCH(C*H*₃)CH₂SCH₃), 2.10 (s, 3H, NHCH(CH₃)CH₂SC*H*₃), 2.49 (m, 1H, NHCH(CH₃)C*H*₂SCH₃), 2.55 (m, 1H, NHCH(CH₃)C*H*₂SCH₃), 3.97 (m, 1H, NHC*H*(CH₃)CH₂SCH₃) |
| 75 | 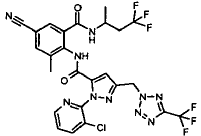 | 3.38 | 641 | DMSO: 1.11 (d, 3H, NHCH(C*H*₃)CH₂CF₃), 2.24-2.37 (m, 2H, NHCH(CH₃)C*H*₂CF₃), 4.12-4.17 (m, 1H, NHC*H*(CH₃)CH₂CF₃) |
| 76 | 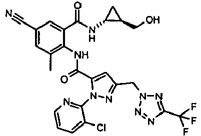 | 2.39 | 601 | DMSO: 0.58-0.65 (m, 2H, NHCH(C*H*₂)CHCH₂OH), 1.11-1.20 (m, 1H, NHCH(C*H*₂)CHCH₂OH), 2.60-2.64 (m, 1H, NHC*H*(CH₂)CHCH₂OH), 3.28-3.42 (m, 2H, NHCH(CH₂)CHC*H*₂OH), 4.22 (t, 1H, NHCH(CH₂)CHCH₂O*H*) |
| 77 | 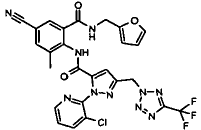 | 3.14 | 611 | DMSO: 4.34 (d, 2H, NHCH₂C₄H₃O), 6.22 (1H, NHCH₂C₄*H*₃O), 6.31 (1H, NHCH₂C₄*H*₃O), 7.46 (1H, NHCH₂C₄*H*₃O) |
| 78 | 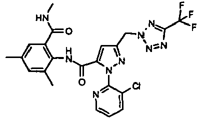 | 2.81 | 534 | DMSO: 2.67 (d, 3H, NHC*H*₃) |
| 79 | 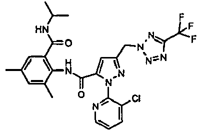 | 3.29 | 562 | DMSO: 1.02 (d, 6H, NHCH(C*H*₃)₂), 3.92 (m, 1H, NHC*H*(CH₃)₂) |
| 80 | 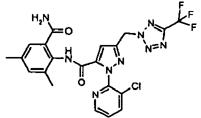 | 2.62 | 520 | DMSO: 7.24 (bs, 2H, N*H*₂) |
| 81 | 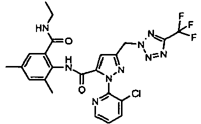 | 3.06 | 548 | DMSO: 1.10 (t, 3H, NHCH₂C*H*₃), 3.15 (m, 2H, NHC*H*₂CH₃) |
| 82 | 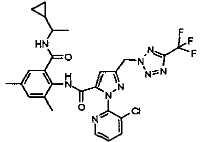 | 3.59 | 588 | DMSO: 0.08-0.36 (m, 4H, NHCH(CH₃)CH(C*H*₂)₂), 0.81 (m, 1H, NHCH(CH₃)C*H*(CH₂)₂), 1.05 (d, 3H, NHCH(C*H*₃)CH(CH₂)₂), 3.34 (m, 1H, NHC*H*(CH₃)CH(CH₂)₂) |
| 83 | 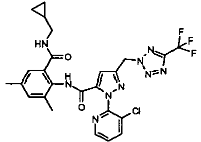 | 3.35 | 574 | DMSO: 0.11 (m, 2H, NHCH₂CH(C*H*₂)₂), 0.30 (m, 2H, NHCH₂CH(C*H*₂)₂), 0.87 (m, 1H, NHCH₂C*H*(CH₂)₂), 3.02 (t, 2H, NHC*H*₂CH(CH₂)₂) |
| 84 | 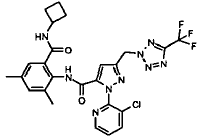 | 3.42 | 574 | DMSO: 1.62 (m, 2H, NHCH(C*H*₂)₃), 1.89 (m, 2H, NHCH(C*H*₂)₃), 2.14 (m, 2H, NHCH(C*H*₂)₃), 4.24 (m, 1H, NHC*H*(CH₂)₃) |
| 85 | 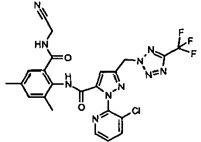 | 2.83 | 559 | DMSO: 4.14 (d, 2H, NHC*H*₂CN) |
| 86 | 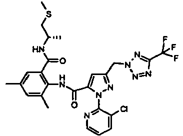 | 3.50 | 608 | DMSO: 1.10 (d, 3H, NHCH(C*H*₃)CH₂SCH₃), 2.36 (s, 3H, NHCH(CH₃)CH₂SC*H*₃), 2.46 (m, 1H, NHCH(CH₃)C*H*₂SCH₃), 2.54 (m, 1H, NHCH(CH₃)C*H*₂SCH₃), 3.99 (m, 1H, NHC*H*(CH₃)CH₂SCH₃) |
| 87 | 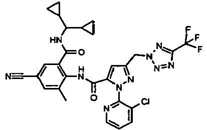 | 3.65 | 625 | DMSO: 0.21 (m, 6H, NHCH(CH(C*H*₂)₂)₂), 0.37, (m, 2H, NHCH(CH(C*H*₂)₂)₂), 0.91 (m, 2H, NHCH(C*H*(CH₂)₂)₂), 2.96 (q, 1H, NHC*H*(CH(CH₂)₂)₂) |
| 88 | 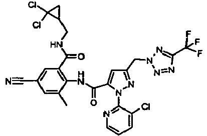 | 3.50 | 653 | |
| 89 | 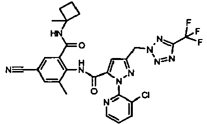 | 3.46 | 599 | |
| 90 | 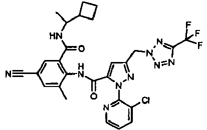 | 3.63 | 613 | DMSO: 1.10 (d, 3H, NHCH(C*H*₃)CH(CH₂)₃), 1.72-2.01 (m, 6H, NHCH(CH₃)CH(C*H*₂)₃), 2.39 (m, 1H, NHCH(CH₃)C*H*(CH₂)₃), 3.06 (m, 1H, NHC*H*(CH₃)CH(CH₂)₃) |
| 91 | 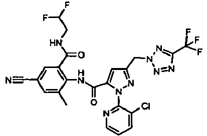 | 2.91 | 595 | DMSO: 3.52 (m, 2H, NHC*H*₂CHF₂), 5.88 (tt, 1H, NHCH₂C*H*F₂) |
| 92 | 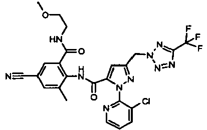 | 2.73 | 589 | DMSO: 3.18 (s, 3H, NHCH₂CH₂OC*H*₃), 3.27-3.68 (m, 4H, NHC*H*₂CH₂OCH₃) |
| 93 | 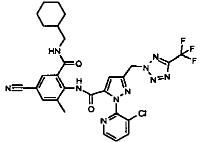 | 3.96 | 627 | DMSO: 0.82-1.63 (m, 11H, NHCH₂C*H*(C*H*₂)₅), 3.00 (t, 2H, NHC*H*₂CH(CH₂)₅) |
| 94 | 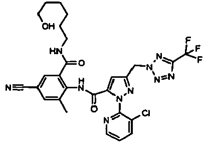 | 2.71 | 631 | DMSO: 121-1.49 (m, 8H, NHCH₂(C*H*₂)₄CH₂OH), 3.19 (t, 1H, NHCH₂(CH₂)₄CH₂O*H*), 3.37 (m, 4H, NHC*H*₂(CH₂)₄C*H*₂OH) |
| 95 | 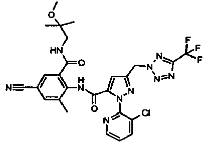 | 3.12 | 617 | DMSO: 1.02 (s, 6H, NHCH₂C(C*H*₃)₂OCH₃), 3.04 (s, 3H, NHCH₂C(CH₃)₂OC*H*₃), 3.20 (d, 2H, NHC*H*₂C(CH₃)₂OCH₃) |
| 96 | 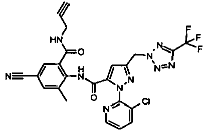 | 2.90 | 569 | DMSO: 2.94 (t, 1H, NHCH₂C≡C*H*), 3.94 (m, 2H, NHC*H*₂C≡CH) |
| 97 | 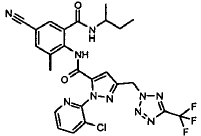 | 3.32 | 587 | DMSO: 0.78 (t, 3H, NHCH(CH₃)CH₂C*H*₃), 1.00 (d, 3H, NHCH(C*H*₃)CH₂CH₃), 1.33-1.42 (m, 2H, NHCH(CH₃)C*H*₂CH₃), 3.71-3.78 (m, 1H, NHC*H*(CH₃)CH₂CH₃) |
| 98 | 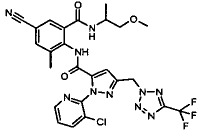 | 2.95 | 603 | DMSO: 1.01 (d, 3H, NHCH(C*H*₃)CH₂OCH₃), 3.18 (s, 3H, NHCH(CH₃)CH₂OC*H*₃), 3.27-3.30 (m 2H, NHCH(CH₃)C*H*₂OCH₃), 3.97-4.03 (m, 1H, NHC*H*(CH₃)CH₂OCH₃) |
| 99 | 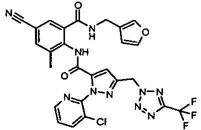 | 3.09 | 611 | DMSO: 4.19 (d, 2H, NHCH₂C₄H₃O), 6.27 (1H, NHCH₂C₄*H*₃O), 6.37 (1H, NHCH₂C₄*H*₃O), 7.46 (1H, NHCH₂C₄*H*₃O) |
| 100 | 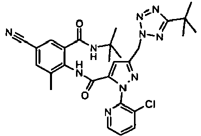 | 3.44 | 575 | DMSO: 1.21 (s, 9H, NHC(C*H*₃)₃) |
| 101 | 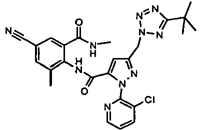 | 2.62 | 533 | DMSO: 2.63 (d, 3H, NHC*H*₃) |
| 102 | 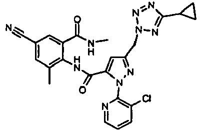 | 2.15 | 517 | DMSO: 2.66 (d, 3H, NHC*H*₃) |
| 103 | 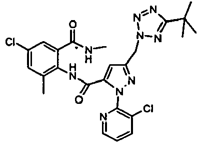 | 3.02 | 542 | DMSO: 2.66 (d, 3H, NHC*H*₃) |
| 104 | 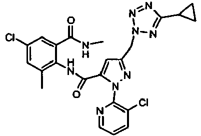 | 2.52 | 526 | DMSO: 2.68 (d, 3H, NHC*H*₃) |
| 105 | 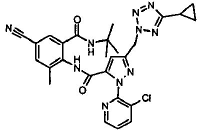 | 2.93 | 559 | DMSO: 1.24 (s, 9H, NHC(C*H*₃)₃) |
| 106 | 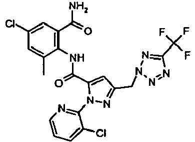 | 2.76 | 540 | CD₃CN: 6.30 (bs, 2H, N*H*₂) |
| 107 | 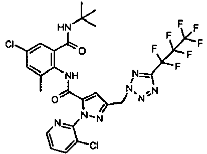 | 4.60 | 696 | CD₃CN: 1.29 (s, 9H, NHC(C*H*₃)₃) |
| 108 | 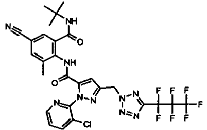 | 4.16 | 687 | CD₃CN: 1.33 (s, 9H, NHC(C*H*₃)₃) |
| 109 | 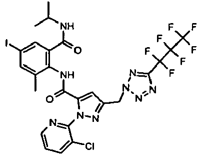 | 4.48 | 774 | CD₃CN: 1.12 (d, 6H, NHCH(C*H*₃)₂), 4.02 (m, 1H, NHC*H*(CH₃)₂) |
| 110 | 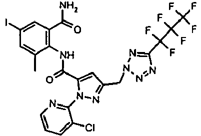 | 3.70 | 732 | CD₃CN: 6.19 (bs, 1H, N*H*₂), 6.72 (bs, 1H, N*H*₂) |
| 111 | 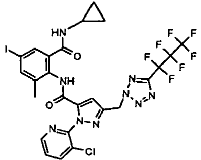 | 4.23 | 772 | CD₃CN: 0.50 (m, 2H, NHCH(C*H*₂)₂), 0.69 (m, 2H, NHCH(C*H*₂)₂), 2.73 (m, 1H, NHC*H*(CH₂)₂) |
| 112 | 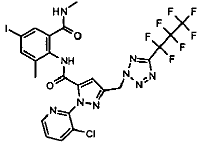 | 3.99 | 746 | CD₃CN: 2.78 (d, 3H, NHC*H*₃) |
| 113 | 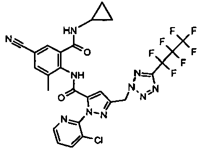 | 3.58 | 671 | CD₃CN: 0.53 (m, 2H, NHCH(C*H*₂)₂), 0.72 (m, 2H, NHCH(C*H*₂)₂), 2.76 (m, 1H, NHC*H*(CH₂)₂) |
| 114 | 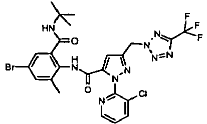 | 4.00 | 640 | DMSO: 1.21 (s, 9H, NHC(C*H*₃)₃) |
| 115 | 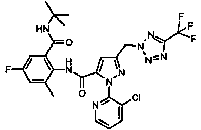 | 3.51 | 580 | DMSO: 1.21 (s, 9H, NHC(C*H*₃)₃) |
| 116 | 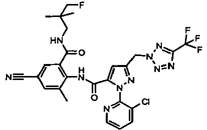 | 3.35 | 619 | DMSO: 0.83 8s, 6H, NHCH₂C(C*H*₃)₂CH₂F), 2.19 (s, 2H, NHCH₂C(CH₃)₂C*H*₂F), 4.02 (s, 1H, NHC*H*₂C(CH₃)₂CH₂F), 4.14 (s, 1H, NHC*H*₂C(CH₃)₂CH₂F) |
| 117 | 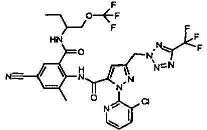 | 3.74 | 671 | DMSO: 0.81 (t, 3H, NHCH(CH₂C*H*₃)CH₂OCF₃), 1.39-1.58 (m, 2H, NHCH(C*H*₂CH₃)CH₂OCF₃), 3.92 (d, 2H, NHCH(CH₂CH₃)C*H*₂OCF₃), 4.03 (m, 1H, NHC*H*(CH₂CH₃)CH₂OCF₃) |
| 118 | 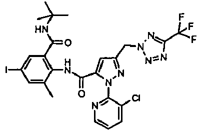 | 4.12 | 688 | DMSO: 1.21 (s, 9H, NHC(C*H*₃)₃) |
| 119 | 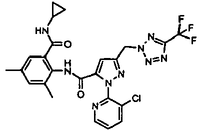 | 3.04 | 560 | DMSO: 0.42 (m, 2H, NHCH(C*H*₂)₂), 0.59 (m, 2H, NHCH(C*H*₂)₂), 2.69 (m, 1H, NHC*H*(CH₂)₂) |
| 120 | 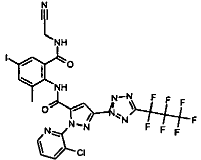 | 3.86 | 771 | CD₃CN: 4.13 (d, 2H, NHC*H*₂CN) |
| 121 | 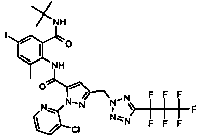 | 4.86 | 788 | CD₃CN: 1.29 (s, 9H, NHC(C*H*₃)₃) |
| 122 | 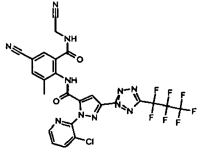 | 3.33 | 670 | CD₃CN: 4.16 (d, 2H, NHC*H*₂CN) |
| 123 | 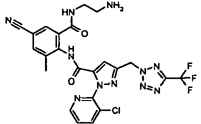 | 1.63 | 574 | |
| 124 | 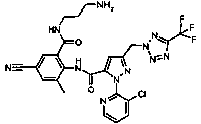 | 1.63 | 588 | DMSO: 1.62 (m, 2H, NHCH₂C*H*₂CH₂NH₂), 2.69 (t, 2H, NHCH₂CH₂C*H*₂NH₂), 3.23 (t, 2H, NHC*H*₂CH₂CH₂NH₂) |
| 125 | 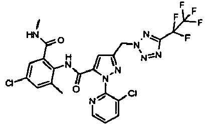 | 3.39 | 604 | DMSO: 2.66 (d, 3H, NHC*H*₃) |
| 126 | 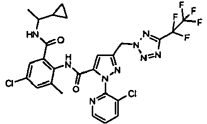 | 4.25 | 658 | DMSO: 0.09-0.36 (m, 4H, NHCH(CH₃)CH(C*H*₂)₂), 0.83 (m, 1H, NHCH(CH₃)C*H*(CH₂)₂), 1.04 (d, 3H, NHCH(C*H*₃)CH(CH₂)₂), 3.30 (m, 1H, NHC*H*(CH₃)CH(CH₂)₂) |
| 127 | 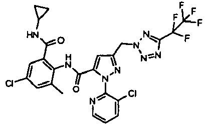 | 3.70 | 630 | DMSO: 0.43 (m, 2H, NHCH(C*H*₂)₂), 0.59 (m, 2H, NHCH(C*H*₂)₂), 2.66 (m, 1H, NHC*H*(CH₂)₂) |
| 128 | 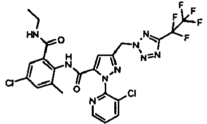 | 3.65 | 618 | DMSO: 0.99 (t, 3H, NHCH₂C*H*₃), 3.15 (m, 2H, NHC*H*₂CH₃) |
| 129 | 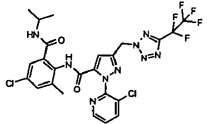 | 3.94 | 632 | DMSO: 1.02 (d, 6H, NHCH(C*H*₃)₂), 3.91 (m, 1H, NHC*H*(CH₃)₂) |
| 130 | 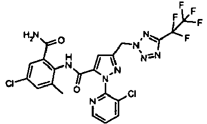 | 3.15 | 590 | DMSO: 7.29-7.58 (bs, 2H, N*H*₂) |
| 131 | 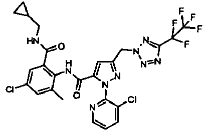 | 3.93 | 644 | DMSO: 0.11 (m, 2H, NHCH₂C*H*(CH₂)₂), 0.29 (m, 2H, NHCH₂CH(C*H*₂)₂), 0.88 (m, 1H, NHCH₂C*H*(CH₂)₂), 3.00 (t, 2H, NHC*H*₂CH(CH₂)₂) |
| 132 | 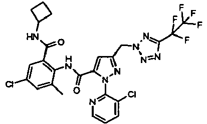 | 4.03 | 644 | DMSO: 1.63 (m, 2H, NHCH(C*H*₂)₃), 1.89 (m, 2H, NHCH(C*H*₂)₃), 2.08 (m, 2H, NHCH(C*H*₂)₃), 4.22 (m, 1H, NHC*H*(CH₂)₃) |
| 133 | 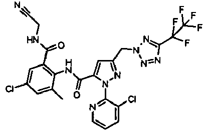 | 3.32 | 629 | DMSO: 4.15 (d, 2H, NHC*H*₂CN) |
| 134 | 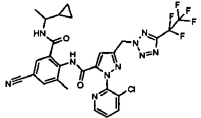 | 3.84 | 649 | DMSO: 0.09-0.38 (m, 4H, NHCH(CH₃)CH(C*H*₂)₂), 0.84 (m, 1H, NHCH(CH₃)C*H*(CH₂)₂), 1.07 (d, 3H, NHCH(C*H*₃)CH(CH₂)₂), 3.34 (m, 1H, NHC*H*(CH₃)CH(CH₂)₂) |
| 135 | 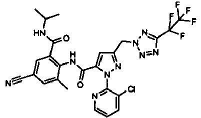 | 3.55 | 623 | DMSO: 1.03 (d, 6H, NHCH(C*H*₃)₂), 3.91 (m, 1H, NHC*H*(CH₃)₂) |
| 136 | 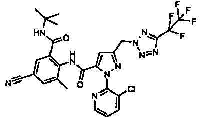 | 3.86 | 637 | DMSO: 1.22 (s, 9H, NHC(C*H*₃)₃) |
| 137 | 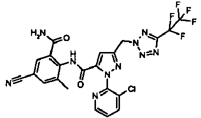 | 2.83 | 581 | DMSO: 7.44 (bs, 1H, N*H*₂), 7.70 (bs, 1H, N*H*₂) |
| 138 | 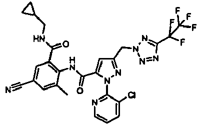 | 3.56 | 635 | DMSO: 0.12 (m, 2H, NHCH₂CH(C*H*₂)₂), 0.31 (m, 2H, NHCH₂CH(C*H*₂)₂), 0.78 (m, 1H, NHCH₂C*H*(CH₂)₂), 2.90 (m, 2H, NHC*H*₂CH(CH₂)₂) |
| 139 | 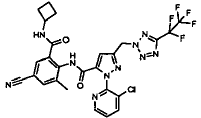 | 3.61 | 635 | DMSO: 1.64 (m, 2H, NHCH(C*H*₂)₃), 1.93 (m, 2H, NHCH(C*H*₂)₃), 2.13 (m, 2H, NHCH(C*H*₂)₃), 4.22 (m, 1H, NHC*H*(CH₂)₃) |
| 140 | 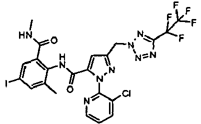 | 3.64 | 695 | DMSO: 2.65 (d, 3H, NHC*H*₃) |
| 141 | 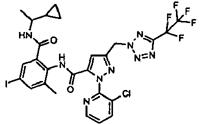 | 4.42 | 750 | DMSO: 0.10-0.48 (m, 4H, NHCH(CH₃)CH(C*H*₂)₂), 0.82 (m, 1H, NHCH(CH₃)C*H*(CH₂)₂), 1.05 (d, 3H, NHCH(C*H*₃)CH(CH_{Z})₂), 3.29 (m, 1H, NHC*H*(CH₃)CH(CH₂)₂) |
| 142 | 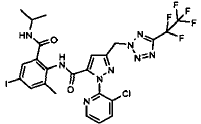 | 4.19 | 723 | DMSO: 1.01 (d, 6H, NHCH(C*H*₃)₂), 3.90 (m, 1H, NHC*H*(CH₃)₂) |
| 143 | 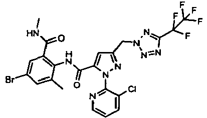 | 3.50 | 647 | DMSO: 2.66 (d, 3H, NHC*H*₃) |
| 144 | 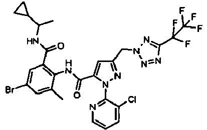 | 4.29 | 702 | DMSO: 0.10-0.48 (m, 4H, NHCH(CH₃)CH(C*H*₂)₂), 0.81 (m, 1H, NHCH(CH₃)C*H*(CH₂)₂), 1.05 (d, 3H, NHCH(C*H*₃)CH(CH₂)₂), 3.33 (m, 1H, NHC*H*(CH₃)CH(CH₂)₂) |
| 145 | 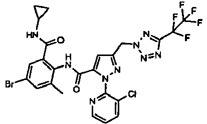 | 3.74 | 674 | DMSO: 0.42 (m, 2H, NHCH(C*H*₂)₂), 0.59 (m, 2H, NHCH(C*H*₂)₂), 2.68 (m, 1H, NHC*H*(CH₂)₂) |
| 146 | 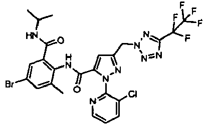 | 3.95 | 676 | DMSO: 1.01 (d, 6H, NHCH(C*H*₃)₂), 3.90 (m, 1H, NHC*H*(CH₃)₂) |
| 147 | 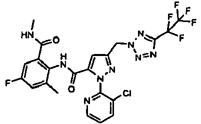 | 3.09 | 588 | DMSO: 2.66 (d, 3H, NHC*H*₃) |
| 148 | 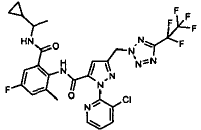 | 3.80 | 642 | DMSO: 0.10-0.35 (m, 4H, NHCH(CH₃)CH(C*H*₂)₂), 0.80 (m, 1H, NHCH(CH₃)CH(C*H*₂)₂), 1.03 (d, 3H, NHCH(C*H*₃)CH(CH₂)₂), 3.31 (m, 1H, NHC*H*(CH₃)CH(CH₂)₂) |
| 149 | 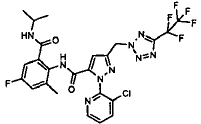 | 3.59 | 616 | DMSO: 1.01 (d, 6H, NHCH(C*H*₃)₂), 3.90 (m, 1H, NHC*H*(CH₃)₂) |
| 150 | 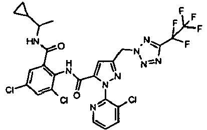 | 4.12 | 678 | DMSO: 0.10-0.36 (m, 4H, NHCH(CH₃)CH(C*H*₂)₂), 0.80 (m, 1H, NHCH(CH₃)C*H*(CH₂)₂), 1.05 (d, 3H, NHCH(C*H*₃)CH(CH₂)₂), 3.29 (m, 1H, NHC*H*(CH₃)CH(CH₂)₂) |
| 151 | 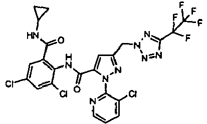 | 3.57 | 650 | DMSO: 0.41 (m, 2H, NHCH(C*H*₂)₂), 0.61 (m, 2H, NHCH(C*H*₂)₂), 2.66 (m, 1H, NHC*H*(CH₂)₂) |
| 152 | 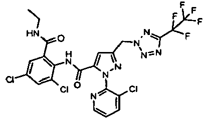 | 357 | 638 | DMSO: 0.97 (t, 3H, NHCH₂C*H*₃), 3.18 (m, 2H, NHC*H*₂CH₃) |
| 153 | 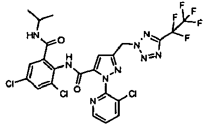 | 3.81 | 652 | DMSO: 1.01 (d, 6H, NHCH(C*H*₃)₂), 3.88 (m, 1H, NHC*H*(CH₃)₂) |
| 154 | 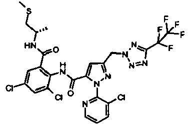 | 4.02 | 698 | DMSO: 1.07 (dd, 3H, NHCH(C*H*₃)CH₂SCH₃), 2.01 (s, 3H, NHCH(CH₃)CH₂SC*H*₃), 2.46 (m, 1H, NHCH(CH₃)C*H*₂SCH₃), 2.54 (m, 1H, NHCH(CH₃)C*H*₂SCH₃), 3.94 (m, 1H, NHC*H*(CH₃)CH₂SCH₃) |
| 155 | 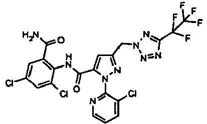 | 3.13 | 610 | DMSO: 7.41 (bs, 1H, N*H*₂), 7.51 (bs, 1H, N*H*₂) |
| 156 | 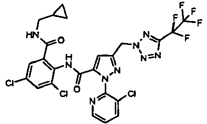 | 3.88 | 664 | DMSO: 0.11 (m, 2H, NHCH₂CH(C*H*₂)₂), 0.31 (m, 2H, NHCH₂CH(C*H*₂)₂), 0.85 (m, 1H, NHCH₂C*H*(CH₂)₂), 3.01 (m, 2H, NHC*H*₂CH(CH₂)₂) |
| 157 | 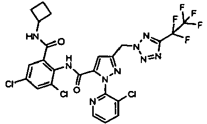 | 3.92 | 664 | DMSO: 1.62 (m, 2H, NHCH(C*H*₂)₃), 1.90 (m, 2H, NHCH(C*H*₂)₃), 2.12 (m, 2H, NHCH(C*H*₂)₃), 4.18 (m, 1H, NHC*H*(CH₂)₃) |
| 158 | 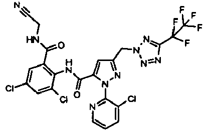 | 3.32 | 649 | DMSO: 4.15 (d, 2H, NHC*H*₂CN) |
| 159 | 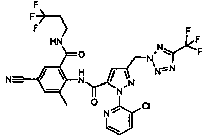 | 3.22 | 627 | DMSO: 2.42 (m, 2H, NHCH₂C*H*₂CF₃), 3.33 (m, 2H, NHC*H*₂CH₂CF₃) |
| 160 | 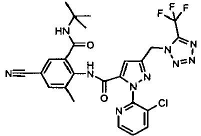 | 3.05 | 587 | DMSO: 1.22 (s, 9H, NHC(C*H*₃)₃) |
| 161 | 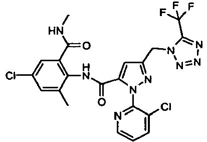 | 2.63 | 554 | DMSO: 2.66 (d, 3H, NHC*H*₃) |
| 162 | 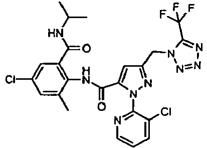 | 3.12 | 582 | DMSO: 1.01 (d, 6H, NHCH(C*H*₃)₂), 3.88 (m, 1H, NHC*H*(CH₃)₂) |
| 163 | 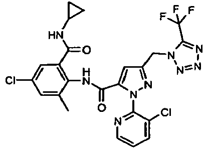 | 2.89 | 580 | DMSO: 0.43 (m, 2H, NHCH(C*H*₂)₂), 0.60 (m, 2H, NHCH(C*H*₂)₂), 2.68 (m, 1H, NHC*H*(CH₂)₂) |
| 164 | 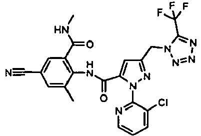 | 2.29 | 645 | DMSO: 2.67 (d, 3H, NHC*H*₃) |
| 165 | 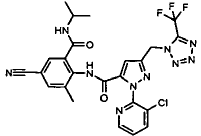 | 2.72 | 573 | DMSO: 1.04 (d, 6H, NHCH(C*H*₃)₂), 3.92 (m, 1H, NHC*H*(CH₃)₂) |
| 166 | 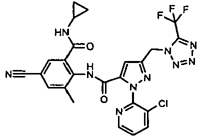 | 2.53 | 571 | DMSO: 0.45 (m, 2H, NHCH(C*H*₂)₂), 0.62 (m, 2H, NHCH(C*H*₂)₂), 2.70 (m, 1H, NHC*H*(CH₂)₂) |
| 167 | 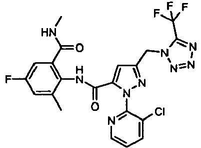 | 2.32 | 538 | DMSO: 2.66 (d, 3H, NHCH₃) |
| 168 | 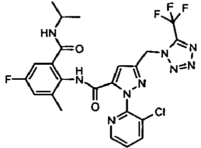 | 2.76 | 566 | DMSO: 1.02 (d, 6H, NHCH(CH₃)₂), 3.91 (m, 1H, NHCH(CH₃)₂) |
| 169 | 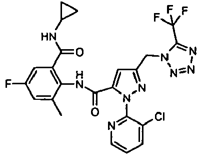 | 2.57 | 564 | DMSO: 0.42 (m, 2H, NHCH(CH₂)₂), 0.60 (m, 2H, NHCH(CH₂)₂), 2.68 (m, 1H, NHCH(CH₂)₂) |
| 170 | 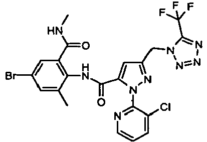 | 2.72 | 598 | DMSO: 2.66 (d, 3H, NHC*H*₃) |
| 171 | 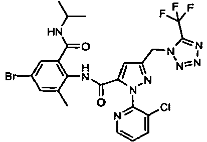 | 3.22 | 626 | DMSO: 1.03 (d, 6H, NHCH(C*H*₃)₂), 3.90 (m, 1H, NHC*H*(CH₃)₂) |
| 172 | 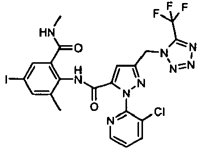 | 2.86 | 646 | DMSO: 2.66 (d, 3H, NHC*H*₃) |
| 173 | 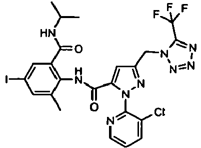 | 3.36 | 674 | DMSO: 1.02 (d, 6H, NHCH(C*H*₃)₂), 3.91 (m, 1H, NHC*H*(CH₃)₂) |
| 174 | 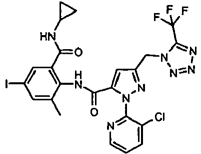 | 3.12 | 672 | DMSO: 0.43 (m, 2H, NHCH(C*H*₂)₂), 0.60 (m, 2H, NHCH(C*H*₂)₂), 2.67 (m, 1 H, NHC*H*(CH₂)₂) |
| 175 | 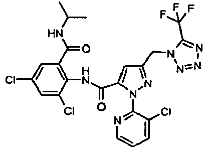 | 3.08 | 602 | DMSO: 1.01 (d, 6H, NHCH(C*H*₃)₂), 3.88 (m, 1H, NHC*H*(CH₃)₂) |
| 176 | 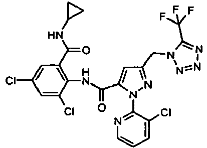 | 2.85 | 600 | DMSO: 0.41 (m, 2H, NHCH(C*H*₂)₂), 0.60 (m, 2H, NHCH(C*H*₂)₂), 2.66 (m, 1H, NHC*H*(CH₂)₂) |
| 177 | 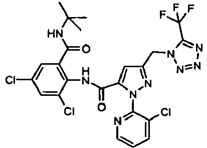 | 3.43 | 616 | DMSO: 1.20 (s, 9H, NHC(C*H*₃)₃) |
| 178 | 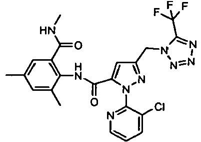 | 2.48 | 534 | DMSO: 2.69 (d, 3H, NHC*H*₃) |
| 179 | 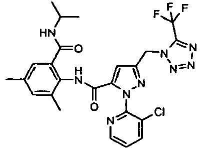 | 2.94 | 562 | DMSO: 1.02 (d, 6H, NHCH(C*H*₃)₂), 3.92 (m, 1H, NHC*H*(CH₃)₂) |
| 180 | 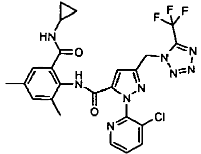 | 2.71 | 560 | DMSO: 0.42 (m, 2H, NHCH(C*H*₂)₂), 0.60 (m, 2H, NHCH(C*H*₂)₂), 2.69 (m, 1H, NHC*H*(CH₂)₂) |
| 181 | 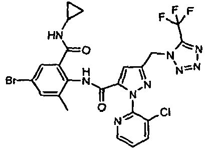 | 2.99 | 624 | DMSO: 0.43 (m, 2H, NHCH(C*H*₂)₂), 0.60 (m, 2H, NHCH(C*H*₂)₂), 2.68 (m, 1H, NHC*H*(CH₂)₂) |
| 182 | 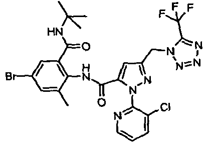 | 3.62 | 640 | DMSO: 1.22 (s, 9H, NHC(C*H*₃)₃) |
| 183 | 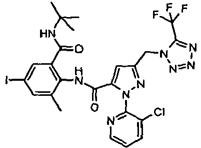 | 3.74 | 688 | DMSO: 1.29 (s, 9H, NHC(C*H*₃)₃) |
| 184 | 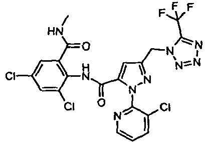 | 2.63 | 574 | DMSO: 2.64 (d, 3H, NHC*H*₃) |
| 185 | 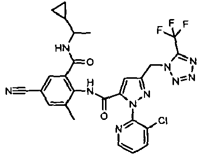 | 3.00 | 599 | DMSO: 0.14-0.37 (m, 4H, NHCH(CH₃)CH(C*H*₂)₂), 0.83 (m, 1H, NHCH(CH₃)C*H*(CH₂)₂), 1.13 (d, 3H, NHCH(C*H*₃)CH(CH₂)₂), 3.35 (m, 1H, NHC*H*(CH₃)CH(CH₂)₂) |
| 186 | 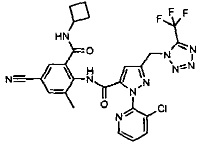 | 2.83 | 585 | DMSO: 1.65 (m, 2H, NHCH(C*H*₂)₃), 1.91 (m, 2H, NHCH(C*H*₂)₃), 2.15 (m, 2H, NHCH(C*H*₂)₃), 4.23 (m, 1H, NHC*H*(CH₂)₃) |
| 187 | 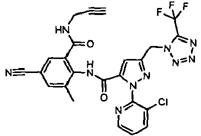 | 2.48 | 569 | DMSO: 2.94 (t, 1 H, NHCH₂C≡C*H*), 3.94 (m, 2H, NHC*H*₂C≡CH) |
| 188 | 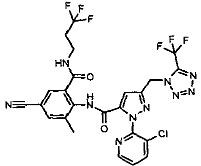 | 2.88 | 627 | DMSO: 2.40 (m, 2H, NHCH₂C*H*₂CF₃), 3.35 (m, 2H, NHC*H*₂CH₂CF₃) |
| 189 | 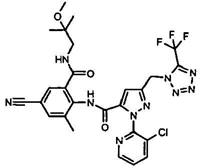 | 2.74 | 617 | DMSO: 1.02 (s, 6H, NHCH₂C(C*H*₃)₂OCH₃), 3.04 (s, 3H, NHCH₂C(CH₃)₂OC*H*₃), 3.22 (d, 2H, NHC*H*₂C(CH₃)₂OCH₃) |
| 190 | 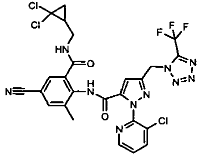 | 3.16 | 654 | |
| 191 | 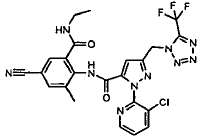 | 2.50 | 559 | DMSO: 1.02 (t, 3H, NHCH₂C*H*₃), 3.16 (m, 2H, NHC*H*₂CH₃) |
| 192 | 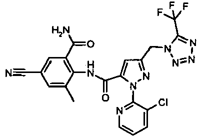 | 2.12 | 531 | DMSO: 7.45 (bs, 1H, N*H*₂), 7.70 (bs, 1H, N*H*₂) |
| 193 | 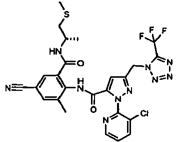 | 2.92 | 619 | DMSO: 1.13 (d, 3H, NHCH(C*H*₃)CH₂SCH₃), 2.18 (s, 3H, NHCH(CH₃)CH₂SC*H*₃), 2.47 (m, 1H, NHCH(CH₃)C*H*₂SCH₃), 2.56 (m, 1H, NHCH(CH₃)C*H*₂SCH₃), 3.98 (m, 1H, NHC*H*(CH₃)CH₂SCH₃) |
| 194 | 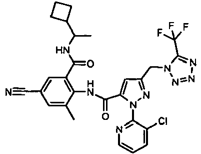 | 3.25 | 613 | DMSO: 1.11 (d, 3H, NHCH(C*H*₃)CH(CH₂)₃), 1.72-2.01 (m, 6H, NHCH(CH₃)CH(C*H*₂)₃), 2.48 (m, 1H, NHCH(CH₃)C*H*(CH₂)₃), 3.10 (m, 1H, NHC*H*(CH₃)CH(CH₂)₃) |
| 195 | 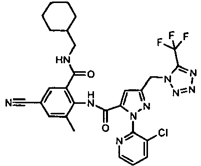 | 3.26 | 628 | DMSO: 1.05-1.81 (m, 11H, NHCH₂C*H*(C*H*₂)₅), 3.62 (m, 2H, NHC*H*₂CH(CH₂)₅) |
| 196 | 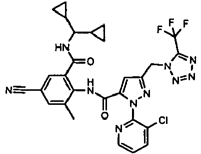 | 3.28 | 625 | DMSO: 0.18-0.41 (m, 8H, NHCH(CH(C*H*₂)₂)₂), 0.92 (m, 2H, NHCH(C*H*(CH₂)₂)₂), 3.02 (m, 1H, NHC*H*(CH(CH₂)₂)₂) |
| 197 | 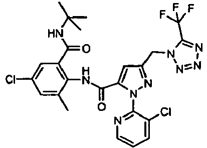 | 3.47 | 596 | CD₃CN: 1.30 (s, 9H, NHC(C*H*₃)₃) |
| 198 | 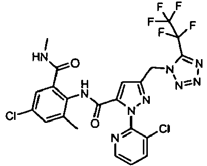 | 3.10 | 604 | DMSO: 2.66 (d, 3H, NHC*H*₃) |
| 199 | 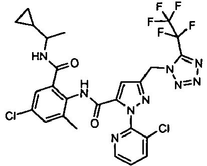 | 3.88 | 658 | DMSO: 0.11-0.37 (m, 4H, NHCH(CH₃)CH(C*H*₂)₂), 0.82 (m, 1H, NHCH(CH₃)C*H*(CH₂)₂), 1.06 (d, 3H, NHCH(C*H*₃)CH(CH₂)₂), 3.33 (m, 1H, NHC*H*(CH₃)CH(CH₂)₂) |
| 200 | 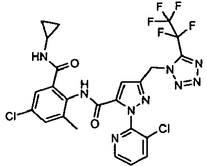 | 3.32 | 630 | DMSO: 0.42 (m, 2H, NHCH(C*H*₂)₂), 0.60 (m, 2H, NHCH(C*H*₂)₂), 2.68 (m, 1H, NHC*H*(CH₂)₂) |
| 201 | 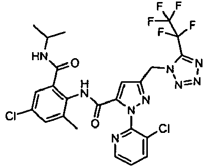 | 3.59 | 632 | DMSO: 1.02 (d, 6H, NHCH(C*H*₃)₂), 3.91 (m, 1H, NHC*H*(CH₃)₂) |
| 202 | 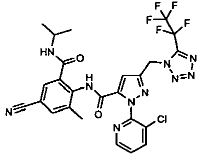 | 3.14 | 623 | DMSO: 1.04 (d, 6H, NHCH(C*H*₃)₂), 3.91 (m, 1H, NHC*H*(CH₃)₂) |
| 203 | 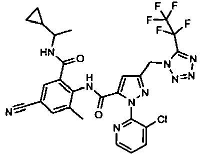 | 3.48 | 649 | DMSO: 0.11-0.38 (m, 4H, NHCH(CH₃)CH(C*H*₂)₂), 0.84 (m, 1H, NHCH(CH₃)C*H*(CH₂)₂), 1.07 (d, 3H, NHCH(C*H*₃)CH(CH₂)₂), 3.91 (m, 1H, NHC*H*(CH₃)CH(CH₂)₂) |
| 204 | 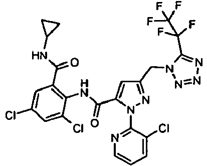 | 3.24 | 650 | DMSO: 0.41 (m, 2H, NHCH(C*H*₂)₂), 0.61 (m, 2H, NHCH(C*H*₂)₂), 2.66 (m, 1H, NHC*H*(CH₂)₂) |
| 205 | 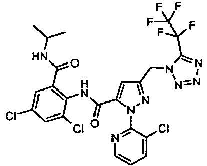 | 3.52 | 652 | DMSO: 1.06 (d, 6H, NHCH(C*H*₃)₂), 3.88 (m, 1H, NHC*H*(CH₃)₂) |
| 206 | 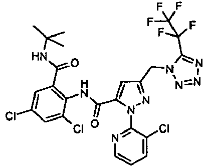 | 3.88 | 666 | DMSO: 1.20 (s, 9H, NHC(C*H*₃)₃) |
| 207 | 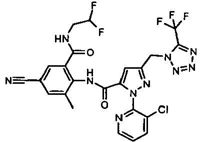 | 2.57 | 595 | DMSO: 3.52 (m, 2H, NHC*H*₂CHF₂), 5.88 (tt, 1H, NHCH₂C*H*F₂) |
| 208 | 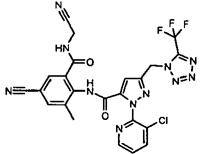 | 2.31 | 570 | DMSO: 4.16 (d, 2H, NHC*H*₂CN) |
| 209 | 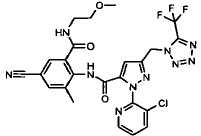 | 2.40 | 589 | DMSO: 3.18 (s, 3H, NHCH₂CH₂OC*H*₃), 3.27-3.37 (m, 4H, NHC*H*₂C*H*₂OCH₃) |
| 210 | 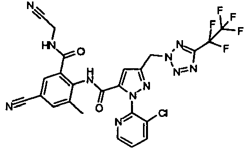 | 3.01 | 620 | DMSO: 4.16 (d, 2H, NHC*H*₂CN) |
| 211 | 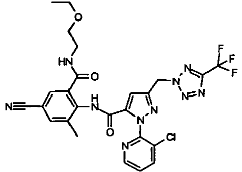 | 2.99 | 603 | DMSO: 1.05 (t, 3H, NHCH₂CH₂OCH₂C*H*₃), 3.27-3.40 (m, 6H, NHC*H*₂C*H*₂OC*H*₂CH₃) |
| 212 | 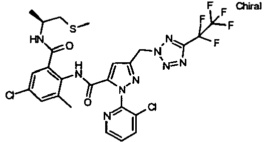 | 4.09 | 678 | DMSO: 1.08 (d, 3H, NHCH(C*H*₃)CH₂SCH₃), 2.15 (s, 3H, NHCH(CH₃)CH₂SC*H*₃), 2.47 (m, 1H, NHCH(CH₃)C*H*₂SCH₃), 2.51 (m, 1H, NHCH(CH₃)C*H*₂SCH₃), 3.98 (m, 1H, NHC*H*(CH₃)CH₂SCH₃) |
| 213 | 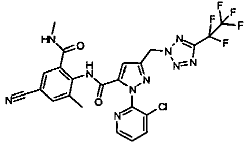 | 3.05 | 595 | DMSO: 2.68 (d, 3H, NHC*H*₃) |
| 214 | 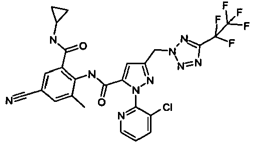 | 3.26 | 621 | DMSO: 0.46 (m, 2H, NHCH(C*H*₂)₂), 0.60 (m, 2H, NHCH(C*H*₂)₂), 2.71 (m, 1H, NHC*H*(CH₂)₂) |
| 215 | 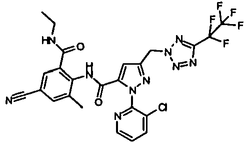 | 3.24 | 609 | DMSO: 1.02 (t, 3H, NHCH₂C*H*₃), 3.16 (m, 2H, NHC*H*₂CH₃) |
| 216 | 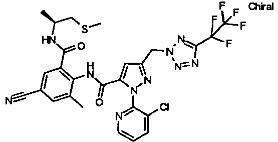 | 3.68 | 669 | DMSO: 1.10 (d, 3H, NHCH(C*H*₃)CH₂SCH₃), 2.21 (s, 3H, NHCH(CH₃)CH₂SC*H*₃), 2.48 (m, 1H, NHCH(CH₃)C*H*₂SCH₃), 2.51 (m, 1H, NHCH(CH₃)C*H*₂SCH₃), 3.98 (m, 1H, NHC*H*(CH₃)CH₂SCH₃) |
| 217 | 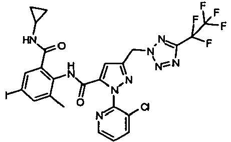 | | | DMSO: 0.43 (m, 2H, NHCH(C*H*₂)₂), 0.59 (m, 2H, NHCH(C*H*₂)₂), 2.66 (m, 1H, NHC*H*(CH₂)₂) |
| 218 | 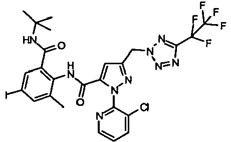 | 4.50 | 738 | DMSO: 1.21 (s, 9H, NHC(C*H*₃)₃) |
| 219 | 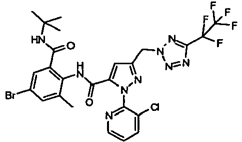 | 4.36 | 690 | DMSO: 1.28 (s, 9H, NHC(C*H*₃)₃) |
| 220 | 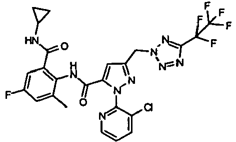 | 3.30 | 614 | DMSO: 0.42 (m, 2H, NHCH(C*H*₂)₂), 0.59 (m, 2H, NHCH(C*H*₂)₂), 2.66 (m, 1H, NHC*H*(CH₂)₂) |
| 221 | 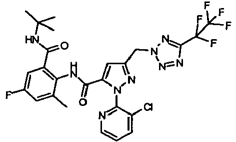 | 3.88 | 630 | DMSO: 1.21 (s, 9H, NHC(C*H*₃)₃) |
| 222 | 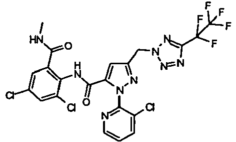 | 3.34 | 624 | DMSO: 2.65 (d, 3H, NHC*H*₃) |
| 223 | 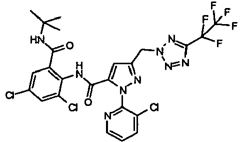 | 4.16 | 666 | DMSO: 1.20 (s, 9H, NHC(C*H*₃)₃) |
| 224 | 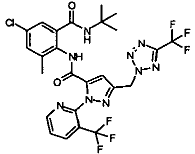 | 4.00 | 630 | DMSO: 1.20 (s, 9H, NHC(C*H*₃)₃) |
| 225 | 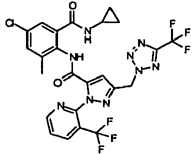 | 3.39 | 614 | DMSO: 0.40-0.44 (m, 2H, NHCH(C*H*₂)₂), 0.55-0.61 (m, 2H, NHCH(C*H*₂)₂), 2.62-2.67 (m, 1H, NHC*H*(CH₂)₂) |
| 226 | 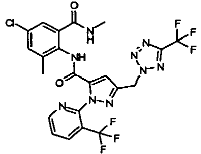 | 3.16 | 588 | DMSO: 2.64 (d, 3H, NHC*H*₃) |
| 227 | 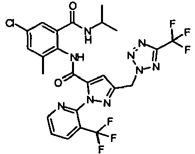 | 3.64 | 616 | DMSO: 0.99 (d, 6H, NHCH(C*H*₃)₂), 3.92 (m, 1H, NHC*H*(CH₃)₂) |
| 228 | 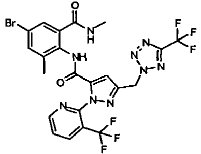 | 3.28 | 632 | DMSO: 2.64 (d, 3H, NHC*H*₃) |
| 229 | 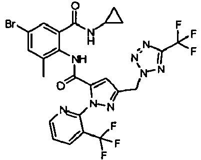 | 3.52 | 658 | DMSO: 0.37-0.42 (m, 2H, NHCH(C*H*₂)₂), 0.55-0.60 (m, 2H, NHCH(C*H*₂)₂), 2.62-2.67 (m, 1H, NHC*H*(CH₂)₂) |
| 230 | 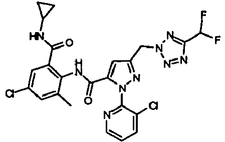 | 2.72 | 562 | DMSO: 0.41 (m, 2H, NHCH(C*H*₂)₂), 0.59 (m, 2H, NHCH(C*H*₂)₂), 2.66 (m, 1H, NHC*H*(CH₂)₂) |
| 231 | 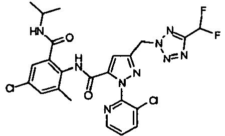 | 2.95 | 564 | DMSO: 1.00 (d, 6H, NHCH(C*H*₃)₂), 3.87 (m, 1H, NHC*H*(CH₃)₂) |
| 232 | 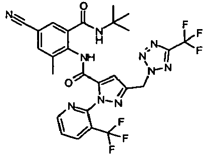 | 3.59 | 621 | DMSO: 1.21 (s, 9H, NHC(C*H*₃)₃) |
| 233 | 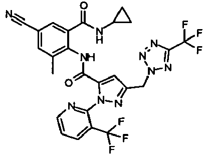 | 3.07 | 605 | DMSO: 0.39-0.43 (m, 2H, NHCH(C*H*₂)₂), 0.57-0.62 (m, 2H, NHCH(C*H*₂)₂), 2.65-2.69 (m, 1H, NHC*H*(CH₂)₂) |
| 234 | 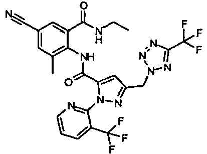 | 3.07 | 593 | DMSO: 0.98 (t, 3H, NHCH₂C*H*₃), 3.13 (q, 2H, NHC*H*₂CH₃) |
| 235 | 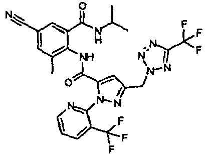 | 3.28 | 607 | DMSO: 1.00 (d, 6H, NHCH(C*H*₃)₂), 3.84-3.91 (m, 1H, NHC*H*(CH₃)₂) |
| 236 | 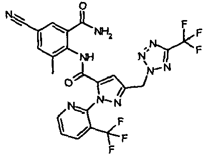 | 2.67 | 565 | DMSO: 7.56 (bs, 1H, N*H*₂), 7.85 (bs, 1H, N*H*₂) |
| 237 | 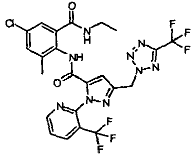 | 3.43 | 602 | DMSO: 0.96 (t, 3H, NHCH₂C*H*₃), 3.12 (q, 2H, NHC*H*₂CH₃) |
| 238 | 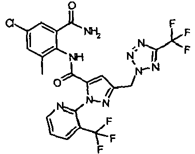 | 2.97 | 574 | DMSO: 7.46 (bs, 1H, N*H*₂), 7.70 (bs, 1H, N*H*₂) |
| 239 | 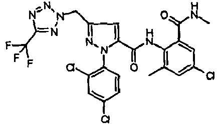 | 3.97 | 588 | DMSO: 2.65 (d, 3H, NHC*H*₃) |
| 240 | 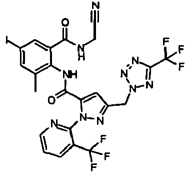 | | 705 | DMSO: 4.13 (d, 2H, NHC*H*₂CN) |
| 241 | 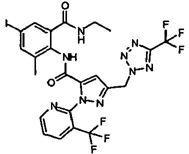 | | 694 | DMSO: 0.99 (t, 3H, NHCH₂C*H*₃), 3.14 (q, 2H, NHC*H*₂CH₃) |
| 242 | 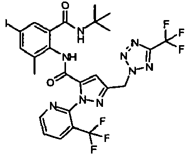 | 4.25 | 722 | DMSO: 1.21 (s, 9H, NHC(C*H*₃)₃) |
| 243 | 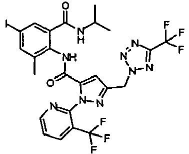 | 3.90 | 707 | DMSO: 0.97 (d, 6H, NHCH(C*H*₃)₂), 3.83-3.88 (m, 1H, NHC*H*(CH₃)₂) |
| 244 | 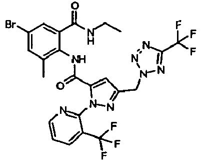 | 3.52 | 647 | DMSO: 0.99 (t, 3H, NHCH₂C*H*₃), 3.14 (q, 2H, NHC*H*₂CH₃) |
| 245 | 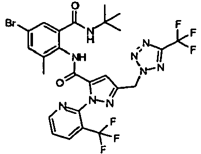 | 4.13 | 675 | DMSO: 1.22 (s, 9H, NHC(C*H*₃)₃) |
| 246 | 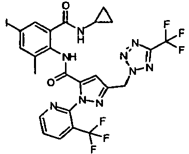 | | 706 | DMSO: 0.40-0.46 (m, 2H, NHCH(C*H*₂)₂), 0.57-0.61 (m, 2H, NHCH(C*H*₂)₂), 2.65-2.70 (m, 1H, NHC*H*(CH₂)₂) |
| 247 | 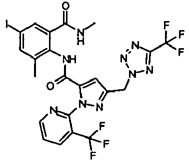 | 3.40 | 680 | DMSO: 2.65 (d, 3H, NHC*H*₃) |
| 248 | 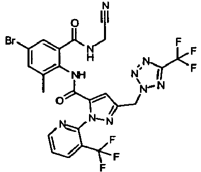 | 3.21 | 657 | DMSO: 4.14 (d, 2H, NHC*H*₂CN) |
| 249 | 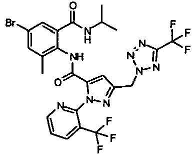 | 3.76 | 660 | 0.99 (d, 6H, NHCH(C*H*₃)₂), 3.83-3.90 (m, 1H, NHC*H*(CH₃)₂) |
| 250 | 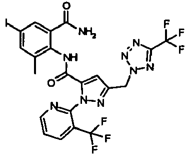 | 3.16 | 666 | DMSO: 7.33 (bs, 1H, N*H*₂), 7.42 (bs, 1H, N*H*₂) |
| 251 | 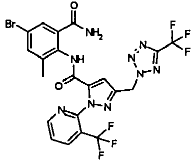 | 3.04 | 619 | DMSO: 7.31 (bs, 1H, N*H*₂), 7.54 (bs, 1H, N*H*₂) |
| 252 | 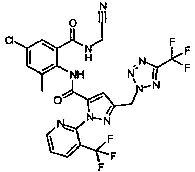 | 3.28 | 613 | DMSO: 4.14 (d, 2H, NHC*H*₂CN) |
| 253 | 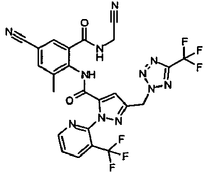 | 2.81 | 604 | DMSO: 4.15 (d, 2H, NHC*H*₂CN) |
| 254 | 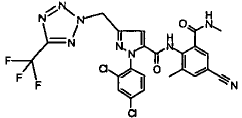 | 3.60 | 579 | DMSO: 2.67 (d, 3H, NHC*H*₃) |
| 255 | 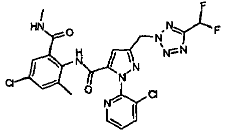 | 2.46 | 536 | DMSO: 2.67 (d, 3H, NHC*H*₃) |
| 256 | 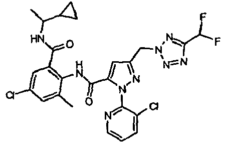 | 3.24 | 590 | DMSO: 0.10-0.37 (m, 4H, NHCH(CH₃)CH(C*H*₂)₂), 0.83 (m, 1H, NHCH(CH₃)C*H*(CH₂)₂), 1.07 (d, 3H, NHCH(C*H*₃)CH(CH₂)₂), 3.33 (m, 1H, NHC*H*(CH₃)CH(CH₂)₂) |
| 257 | 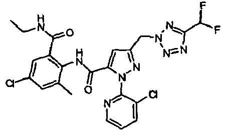 | 2.69 | 550 | DMSO: 1.00 (t, 3H, NHCH₂C*H*₃), 3.15 (m, 2H, NHC*H*₂CH₃) |
| 258 | 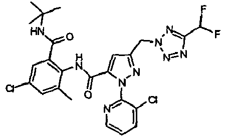 | 3.32 | 578 | DMSO: 1.21 (s, 9H, NHC(C*H*₃)₃) |
| 259 | 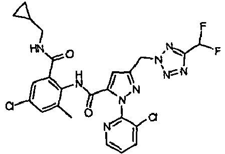 | 3.04 | 576 | DMSO: 0.12 (m, 2H, NHCH₂CH(C*H*₂)₂), 0.32 (m, 2H, NHCH₂CH(C*H*₂)₂), 0.90 (m, 1H, NHCH₂C*H*(CH₂)₂), 3.01 (t, 2H, NHC*H*₂CH(CH₂)₂) |
| 260 | 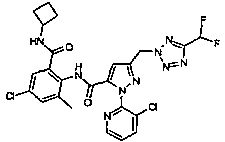 | 3.09 | 576 | DMSO: 1.62 (m, 2H, NHCH(C*H*₂)₃), 1.93 (m, 2H, NHCH(C*H*₂)₃), 2.11 (m, 2H, NHCH(C*H*₂)₃), 4.23 (m, 1H, NHC*H*(CH₂)₃) |
| 261 | 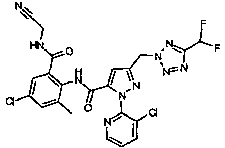 | 2.50 | 561 | DMSO: 4.15 (d, 2H, NHC*H*₂CN) |
| 262 | 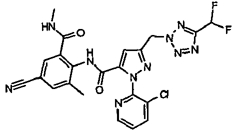 | 2.17 | 527 | DMSO: 2.68 (d, 3H, NHC*H*₃) |
| 263 | 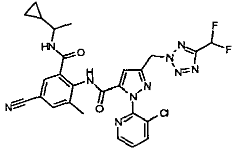 | 2.86 | 581 | DMSO: 0.12-0.38 (m, 4H, NHCH(CH₃)CH(C*H*₂)₂), 0.84 (m, 1H, NHCH(CH₃)C*H*(CH₂)₂), 1.07 (d, 3H, NHCH(C*H*₃)CH(CH₂)₂), 3.39 (m, 1H, NHC*H*(CH₃)CH(CH₂)₂) |
| 264 | 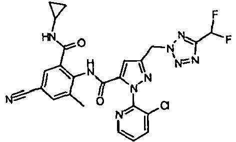 | 2.39 | 553 | DMSO: 0.42 (m, 2H, NHCH(C*H*₂)₂), 0.58 (m, 2H, NHCH(C*H*₂)₂), 2.67 (m, 1H, NHC*H*(CH₂)₂) |
| 265 | 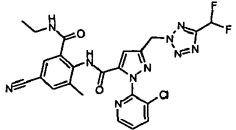 | 2.36 | 541 | DMSO: 1.01 (t, 3H, NHCH₂C*H*₃), 3.16 (m, 2H, NHC*H*₂CH₃) |
| 266 | 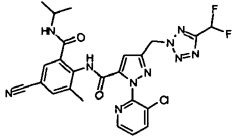 | 2.58 | 555 | DMSO: 1.04 (d, 6H, NHCH(C*H*₃)₂), 3.92 (m, 1H, NHC*H*(CH₃)₂) |
| 267 | 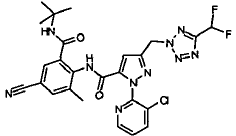 | 2.88 | 569 | DMSO: 1.23 (s, 9H, NHC(C*H*₃)₃) |
| 268 | 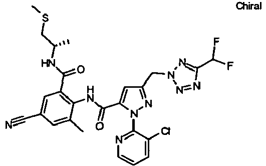 | 2.77 | 601 | DMSO: 1.10 (d, 3H, NHCH(C*H*₃)CH₂SCH₃), 2.21 (s, 3H, NHCH(CH₃)CH₂SC*H*₃), 2.46 (m, 1H, NHCH(CH₃)C*H*₂SCH₃), 2.58 (m, 1H, NHCH(CH₃)C*H*₂SCH₃), 3.98 (m, 1H, NHC*H*(CH₃)CH₂SCH₃) |
| 269 | 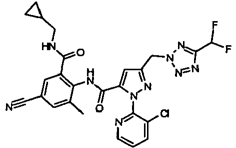 | 2.66 | 567 | DMSO: 0.14 (m, 2H, NHCH₂CH(C*H*₂)₂), 0.33 (m, 2H, NHCH₂CH(C*H*₂)₂), 0.90 (m, 1H, NHCH₂C*H*(CH₂)₂), 3.03 (t, 2H, NHC*H*₂CH(CH₂)₂) |
| 270 | 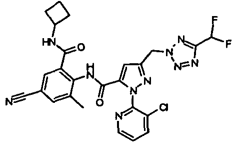 | 2.70 | 567 | DMSO: 1.63 (m, 2H, NHCH(C*H*₂)₃), 1.94 (m, 2H, NHCH(C*H*₂)₃), 2.13 (m, 2H, NHCH(C*H*₂)₃), 4.22 (m, 1H, NHC*H*(CH₂)₃) |
| 271 | 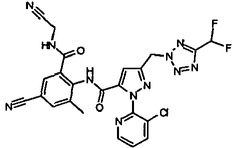 | 2.19 | 552 | DMSO: 4.16 (d, 2H, NHC*H*₂CN) |
| 272 | 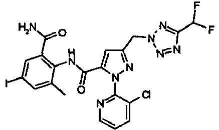 | 2.53 | 614 | DMSO: 7.25-7.60 (bs, 2H, N*H*₂) |
| 273 | 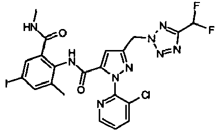 | 2.73 | 628 | DMSO: 2.69 (d, 3H, NHC*H*₃) |
| 274 | 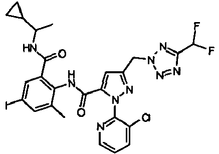 | 3.52 | 682 | DMSO: 0.09-0.41 (m, 4H, NHCH(CH₃)CH(C*H*₂)₂), 0.81 (m, 1H, NHCH(CH₃)C*H*(CH₂)₂), 1.05 (d, 3H, NHCH(C*H*₃)CH(CH₂)₂), 3.32 (m, 1H, NHC*H*(CH₃)CH(CH₂)₂) |
| 275 | 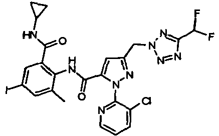 | 2.96 | 654 | DMSO: 0.44 (m, 2H, NHCH(C*H*₂)₂), 0.60 (m, 2H, NHCH(C*H*₂)₂), 2.69 (m, 1H, NHC*H*(CH₂)₂) |
| 276 | 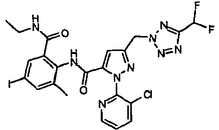 | 2.98 | 642 | DMSO: 1.01 (t, 3H, NHCH₂C*H*₃), 3.12 (m, 2H, NHC*H*₂CH₃) |
| 277 | 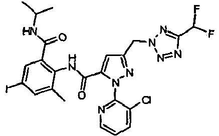 | 3.20 | 656 | DMSO: 1.07 (d, 6H, NHCH(C*H*₃)₂), 3.91 (m, 1H, NHC*H*(CH₃)₂) |
| 278 | 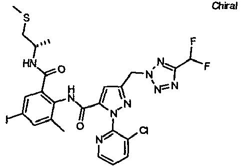 | 3.41 | 702 | DMSO: 1.11 (d, 3H, NHCH(C*H*₃)CH₂SCH₃), 2.12 (s, 3H, NHCH(CH₃)CH₂SC*H*₃), 2.47 (m, 1H, NHCH(CH₃)C*H*₂SCH₃), 2.56 (m, 1H, NHCH(CH₃)C*H*₂SCH₃), 3.97 (m, 1H, NHC*H*(CH₃)CH₂SCH₃) |
| 279 | 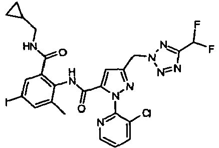 | 3.27 | 668 | DMSO: 0.12 (m, 2H, NHCH₂CH(C*H*₂)₂), 0.32 (m, 2H, NHCH₂CH(C*H*₂)₂), 0.88 (m, 1H, NHCH₂C*H*(CH₂)₂), 3.10 (t, 2H, NHC*H*₂CH(CH₂)₂) |
| 280 | 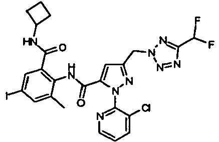 | 3.34 | 668 | DMSO: 1.59 (m, 2H, NHCH(C*H*₂)₃), 1.90 (m, 2H, NHCH(C*H*₂)₃), 2.13 (m, 2H, NHCH(C*H*₂)₃), 4.22 (m, 1H, NHC*H*(CH₂)₃) |
| 281 | 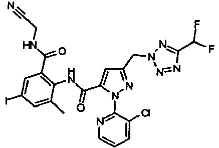 | 2.68 | 653 | DMSO: 4.14 (d, 2H, NHC*H*₂CN) |
| 282 | 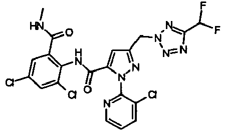 | 2.48 | 556 | DMSO: 2.65 (d, 3H, NHC*H*₃) |
| 283 | 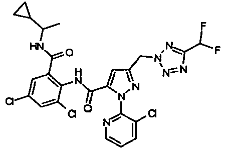 | 3.23 | 610 | DMSO: 0.10-0.37 (m, 4H, NHCH(CH₃)CH(C*H*₂)₂), 0.80 (m, 1H, NHCH(CH₃)C*H*(CH₂)₂), 1.11 (d, 3H, NHCH(C*H*₃)CH(CH₂)₂), 3.30 (m, 1H, NHC*H*(CH₃)CH(CH₂)₂) |
| 284 | 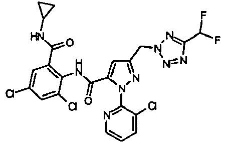 | 2.70 | 582 | DMSO: 0.42 (m, 2H, NHCH(C*H*₂)₂), 0.60 (m, 2H, NHCH(C*H*₂)₂), 2.67 (m, 1H, NHC*H*(CH₂)₂) |
| 285 | 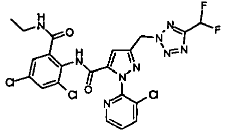 | 2.70 | 570 | DMSO: 0.98 (t, 3H, NHCH₂C*H*₃), 3.13 (m, 2H, NHC*H*₂CH₃) |
| 286 | 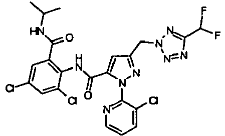 | 2.91 | 584 | DMSO: 1.01 (d, 6H, NHCH(C*H*₃)₂), 3.89 (m, 1H, NHC*H*(CH₃)₂) |
| 287 | 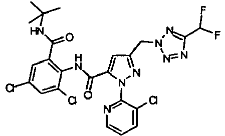 | 3.28 | 598 | DMSO: 1.21 (s, 9H, NHC(C*H*₃)₃) |
| 288 | 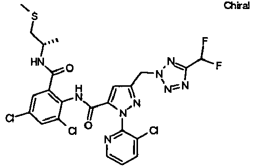 | 3.13 | 630 | DMSO: 1.07 (d, 3H, NHCH(C*H*₃)CH₂SCH₃), 2.00 (s, 3H, NHCH(CH₃)CH₂SC*H*₃), 2.45 (m, 1H, NHCH(CH₃)C*H*₂SCH₃), 2.55 (m, 1H, NHCH(CH₃)C*H*₂SCH₃), 3.94 (m, 1H, NHC*H*(CH₃)CH₂SCH₃) |
| 289 | 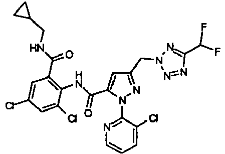 | 3.01 | 596 | DMSO: 0.10 (m, 2H, NHCH₂CH(C*H*₂)₂), 0.30 (m, 2H, NHCH₂CH(C*H*₂)₂), 0.85 (m, 1H, NHCH₂C*H*(CH₂)₂), 2.99 (t, 2H, NHC*H*₂CH(CH₂)₂) |
| 290 | 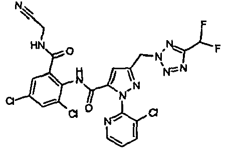 | 2.48 | 581 | DMSO: 4.15 (d, 2H, NHC*H*₂CN) |
| 291 | 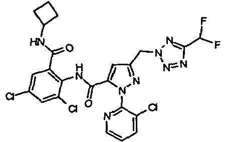 | 3.06 | 596 | DMSO: 1.62 (m, 2H, NHCH(C*H*₂)₃), 1.89 (m, 2H, NHCH(C*H*₂)₃), 2.13 (m, 2H, NHCH(C*H*₂)₃), 4.20 (m, 1H, NHC*H*(CH₂)₃) |
| 292 | 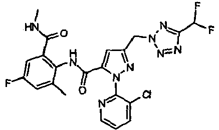 | 2.21 | 520 | DMSO: 2.66 (d, 3H, NHC*H*₃) |
| 293 | 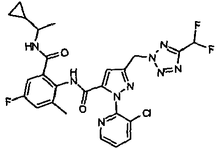 | 2.92 | 574 | DMSO: 0.11-0.40 (m, 4H, NHCH(CH₃)CH(C*H*₂)₂), 0.81 (m, 1H, NHCH(CH₃)C*H*(CH₂)₂), 1.05 (d, 3H, NHCH(C*H*₃)CH(CH₂)₂), 3.33 (m, 1H, NHC*H*(CH₃)CH(CH₂)₂) |
| 294 | 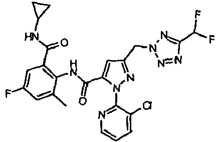 | 2.41 | 546 | DMSO: 0.43 (m, 2H, NHCH(C*H*₂)₂), 0.59 (m, 2H, NHCH(C*H*₂)₂), 2.68 (m, 1H, NHC*H*(CH₂)₂) |
| 295 | 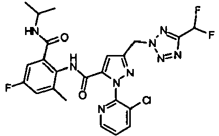 | 2.64 | 548 | DMSO: 1.02 (d, 6H, NHCH(C*H*₃)₂), 3.92 (m, 1H, NHC*H*(CH₃)₂) |
| 296 | 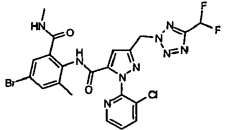 | 2.61 | 580 | DMSO: 2.66 (d, 3H, NHC*H*₃) |
| 297 | 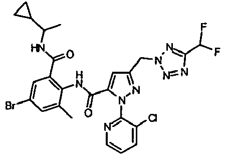 | 3.36 | 634 | DMSO: 0.11-0.37 (m, 4H, NHCH(CH₃)CH(C*H*₂)₂), 0.83 (m, 1H, NHCH(CH₃)C*H*(CH₂)₂), 1.05 (d, 3H, NHCH(C*H*₃)CH(CH₂)₂), 3.33 (m, 1H, NHC*H*(CH₃)CH(CH₂)₂) |
| 298 | 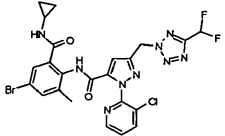 | 2.85 | 606 | DMSO: 0.43 (m, 2H, NHCH(C*H*₂)₂), 0.59 (m, 2H, NHCH(C*H*₂)₂), 2.68 (m, 1H, NHC*H*(CH₂)₂) |
| 299 | 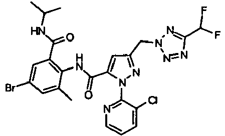 | 3.08 | 608 | DMSO: 1.02 (d, 6H, NHCH(C*H*₃)₂), 3.91 (m, 1H, NHC*H*(CH₃)₂) |
| 300 | 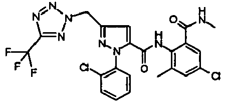 | 3.47 | 554 | DMSO: 2.66 (d, 3H, NHC*H*₃) |
| 301 | 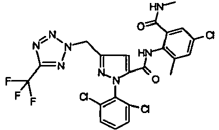 | 3.32 | 588 | DMSO: 2.64 (d, 3H, NHC*H*₃) |
| 302 | 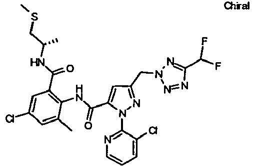 | 3.18 | 610 | DMSO: 1.11 (d, 3H, NHCH(C*H*₃)CH₂SCH₃), 2.15 (s, 3H, NHCH(CH₃)CH₂SC*H*₃), 2.45 (m, 1H, NHCH(CH₃)C*H*₂SCH₃), 2.55 (m, 1H, NHCH(CH₃)C*H*₂SCH₃), 3.98 (m, 1H, NHC*H*(CH₃)CH₂SCH₃) |
| 303 | 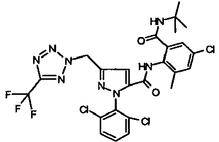 | 4.50 | 630 | DMSO: 1.20 (s, 9H, NHC(C*H*₃)₃) |
| 304 | 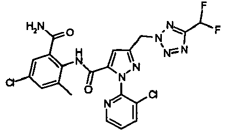 | 2.32 | 522 | DMSO: 7.23-7.60 (bs, 2H, N*H*₂) |
| 305 | 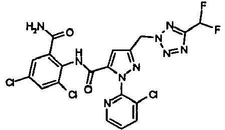 | 2.31 | 542 | DMSO: 7.34-7.60 (bs, 2H, N*H*₂) |
| 306 | 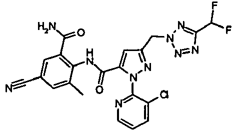 | 1.97 | 513 | DMSO: 7.35-7.52 (bs, 1H, N*H*₂), 7.60-7.80 (bs, 1H, N*H*₂) |
| 307 | 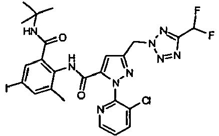 | 3.50 | 670 | DMSO: 1.21 (s, 9H, NHC(C*H*₃)₃) |
| 308 | 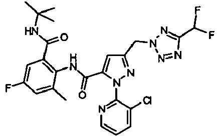 | 2.90 | 562 | DMSO: 1.21 (s, 9H, NHC(C*H*₃)₃) |
| 309 | 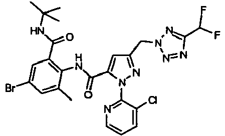 | 3.36 | 622 | DMSO: 1.22 (s, 9H, NHC(C*H*₃)₃) |
| 310 | 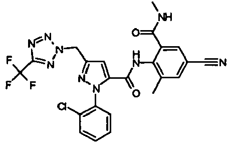 | 3.09 | 544 | DMSO: 2.67 (d, 3H, NHC*H*₃) |
| 311 | 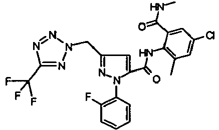 | 3.32 | 537 | DMSO: 2.67 (d, 3H, NHC*H*₃) |
| 312 | 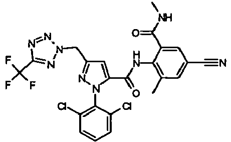 | 3.28 | 579 | DMSO: 2.66 (d, 3H, NHC*H*₃) |
| 313 | 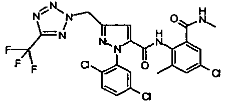 | 3.93 | 588 | DMSO: 2.66 (d, 3H, NHC*H*₃) |
| 314 | 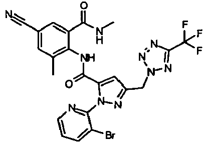 | 2.58 | 589 | DMSO: 2.79 (d, 3H, NHC*H*₃) |
| 315 | 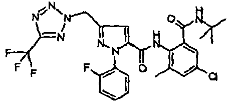 | 4.17 | 588 | DMSO: 1.21 (s, 9H, NHC(C*H*₃)₃) |
| 316 | 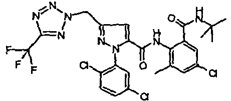 | 4.77 | 630 | DMSO: 1.21 (s, 9H, NHC(C*H*₃)₃) |
| 317 | 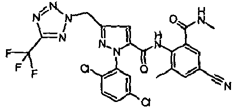 | 3.48 | 579 | DMSO: 2.67 (d, 3H, NHC*H*₃) |
| 318 | 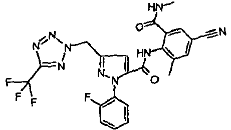 | 2.92 | 528 | DMSO: 2.68 (d, 3H, NHC*H*₃) |
| 319 | 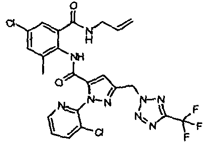 | 3.34 | 581 | DMSO: 4.99 (d, 2H, NHC*H*₂CH=CH₂), 5.13 (d, 2H, NHCH₂CH=C*H*₂), 5.71-5.79 (m, 1H, NHCH₂C*H*=CH₂) |
| 320 | 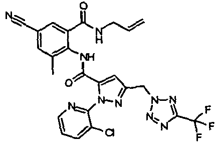 | 2.97 | 572 | DMSO: 5.01 (d, 2H, NHC*H*₂CH=CH₂), 5.13 (d, 2H, NHCH₂CH=C*H*₂), 5.71-5.80 (m, 1H, NHCH₂C*H*=CH₂) |
| 321 | 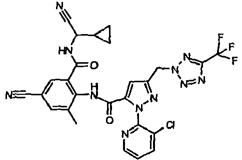 | 3.05 | 610 | DMSO: 0.26-0.58 (m, 4H, NHCH(CN)CH(C*H*₂)₂), 1.26 (m, 1H, NHCH(CN)C*H*(CH₂)₂), 4.39 (m, 1H, NHC*H*(CN)CH(CH₂)₂), |
| 322 | 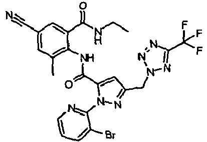 | 2.88 | 603 | DMSO: 1.01 (t, 3H, NHCH₂C*H*₃), 3.15 (m, 2H, NHC*H*₂CH₃) |
| 323 | 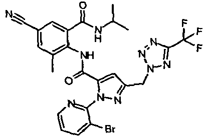 | 3.10 | 617 | DMSO: 1.04 (d, 6H, NHCH(C*H*₃)₂), 3.89-3.95 (m, 1H, N*H*CH(CH₃)₂) |
| 324 | 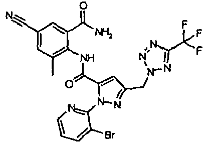 | 2.46 | 575 | DMSO: 7.45 (bs, 1H, N*H*₂), 7.70 (bs, 1H, N*H*₂) |
| 325 | 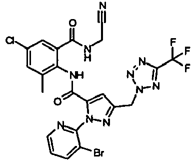 | 2.72 | 623 | DMSO: 4.15 (d, 2H, NHC*H*₂CN) |
| 326 | 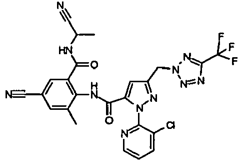 | 2.76 | 584 | DMSO: 1.37 (d, 3H, NHCH(CN)C*H*₃), 4.78 (m, 1H, NHC*H*(CN)CH₃) |
| 327 | 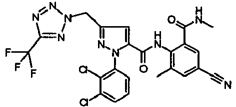 | 3.40 | 579 | DMSO: 2.67 (d, 3H, NHC*H*₃) |
| 328 | 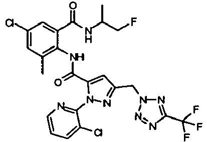 | 3.30 | 601 | DMSO: 1.06 (d, 3H, NHCH(C*H*₃)CH₂F), 4.06-4.36 (m, 3H, NHC*H*(CH₃)C*H*₂F) |
| 329 | 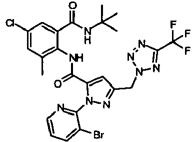 | 3.88 | 640 | DMSO: 1.22 (s, 9H, NHC(C*H*₃)₃) |
| 330 | 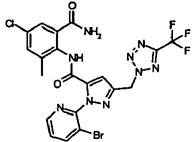 | 2.76 | 584 | DMSO: 7.30 (bs, 1H, N*H*₂), 7.50 (bs, 1H, N*H*₂) |
| 331 | 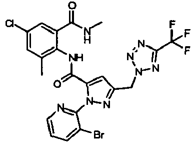 | 3.01 | 598 | DMSO: 2.67 (d, 3H, NHC*H*₃) |
| 332 | 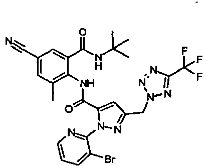 | 3.43 | 631 | DMSO: 1.21 (s, 9H, NHC(C*H*₃)₃) |
| 333 | 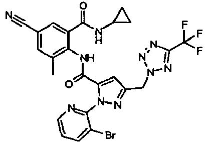 | 2.85 | 616 | DMSO: 0.40-0.44 (m, 2H, NHCH(C*H*₂)₂), 0.57-0.62 (m, 2H, NHCH(C*H*₂)₂), 2.64-2.70 (m, 1H, NHC*H*(CH₂)₂) |
| 334 | 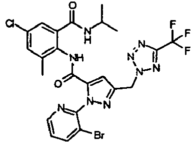 | 3.49 | 626 | DMSO: 1.02 (d, 6H, NHCH(C*H*₃)₂), 3.88-3.94 (m, 1H, NHC*H*(CH₃)₂) |
| 335 | 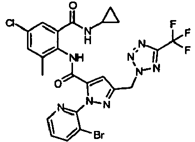 | 3.25 | 624 | DMSO: 0.43-0.46 (m, 2H, NHCH(C*H*₂)₂), 0.56-0.61 (m, 2H, NHCH(C*H*₂)₂), 2.65-2.72 (m, 1H, NHC*H*(CH₂)₂) |
| 336 | 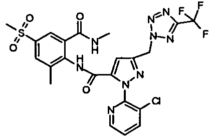 | 2.36 | 598 | DMSO: 2.69 (d, 3H, NHC*H*₃) |
| 337 | 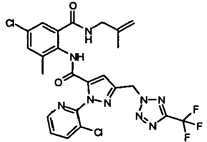 | 3.62 | 595 | DMSO: 1.63 (s, 3H, NHCH₂C(C*H*₃)=CH₂), 3.69 (d, 2H, NHC*H*₂C(CH₃)=CH₂), 4.74 (d, 2H, NHCH₂C(CH₃)=C*H*₂) |
| 338 | 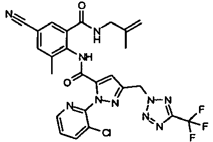 | 3.21 | 585 | DMSO: 1.64 (s, 3H, NHCH₂C(C*H*₃)=CH₂), 3.70 (d, 2H, NHC*H*₂C(CH₃)=CH₂), 4.76 (d, 2H, NHCH₂C(CH₃)=C*H*₂) |
| 339 | 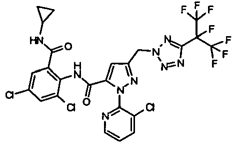 | 3.89 | 700 | DMSO: 0.42 (m, 2H, NHCH(C*H*₂)₂), 0.59 (m, 2H, NHCH(C*H*₂)₂), 2.66 (m, 1H, NHC*H*(CH₂)₂) |
| 340 | 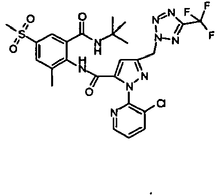 | 3.00 | 640 | DMSO: 1.24 (s, 9H, NHC(C*H*₃)₃) |
| 341 | 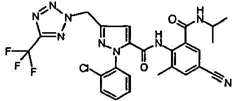 | 3.60 | 572 | DMSO: 1.03-1.04 (m, 6H, NHCH(C*H*₃)₂), 3.90-3.94 (m, 1H, NHC*H*(CH₃)₂) |
| 342 | 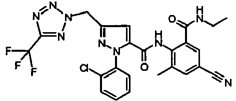 | 3.36 | 558 | DMSO: 1.01-1.02 (m, 3H, NHCH₂C*H*₃), 3.17-3.19 (m, 2H, NHC*H*₂CH₃) |
| 343 | 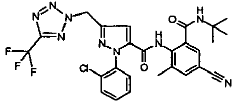 | 3.92 | 586 | DMSO: 1.23 (s, 9H, NHC(C*H*₃)₃) |
| 344 | 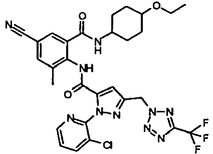 | 3.60 | 658 | DMSO: 1.11 (t, 3H, NHCH(CH₂)₄CHOCH₂C*H*₃), 1.40-1.55 (m, 8H, NHCH(C*H*₂)₄CHOCH₂CH₃), 1.72-1.76 (m, 1H, NHC*H*(CH₂)₄CHOCH₂CH₃), 3.42 (q, 2H, NHCH(CH₂)₄CHOC*H*₂CH₃), 3.65-3.70 (m, 1H, NHCH(CH₂)₄C*H*OCH₂CH₃) |
| 345 | 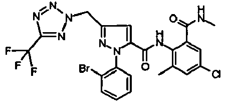 | 3.50 | 598 | DMSO: 2.66 (d, 3H, NHC*H*₃) |
| 346 | 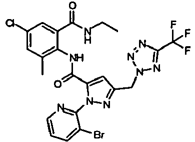 | 3.23 | 613 | DMSO: 1.00 (t, 3H, NHCH₂C*H*₃), 3.14 (q, 2H, NHC*H*₂CH₃) |
| 347 | 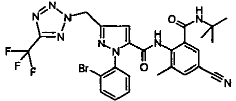 | 3.95 | 631 | DMSO: 1.19 (s, 9H, NHC(C*H*₃)₃) |
| 348 | 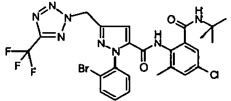 | 4.43 | 641 | DMSO: 1.18 (s, 9H, NHC(C*H*₃)₃) |
| 349 | 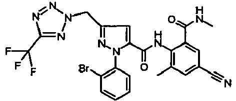 | 3.15 | 590 | DMSO: 2.65 (d, 3H, NHC*H*₃) |
| 350 | 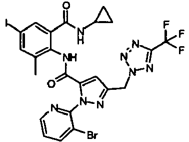 | 3.45 | 716 | DMSO: 0.39-0.43 (m, 2H, NHCH(C*H*₂)₂), 0.55-0.61 (m, 2H, NHCH(C*H*₂)₂), 2.63-2.70 (m, 1H, NHC*H*(CH₂)₂) |
| 351 | 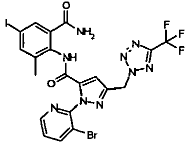 | 2.95 | 676 | DMSO: 7.42 (bs, 1H, N*H*₂), 7.70 (bs, 1H, N*H*₂) |
| 352 | 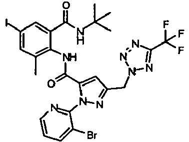 | 4.09 | 732 | DMSO: 1.19 (s, 9H, NHC(C*H*₃)₃) |
| 353 | 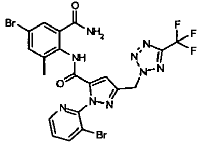 | 2.82 | 628 | DMSO: 7.45 (bs, 1H, N*H*₂), 7.72 (bs, 1H, N*H*₂) |
| 354 | 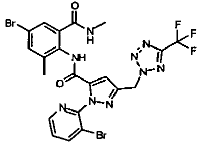 | 3.12 | 643 | DMSO: 2.65 (d, 3H, NHC*H*₃) |
| 355 | 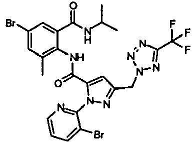 | 3.66 | 670 | DMSO: 0.99 (d, 6H, NHCH(C*H*₃)₂), 3.86-3.90 (m, 1H, NHC*H*(CH₃)₂) |
| 356 | 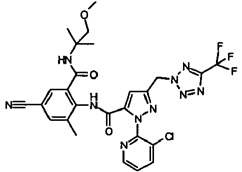 | 3.29 | 617 | CD₃CN: 1.29 (s, 6H, NHC(C*H*₃)₂ CH₂OCH₃), 3.21 (s, 3H, , NHC(CH₃)₂ CH₂OC*H*₃), 3.38 (s, 2H, NHC(CH₃)₂ C*H*₂OCH₃) |
| 357 | 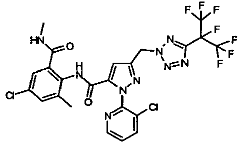 | 3.72 | 654 | DMSO: 2.66 (d, 3H, NHC*H*₃) |
| 358 | 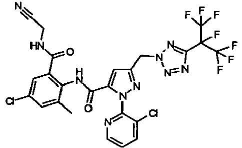 | 3.62 | 679 | DMSO: 4.16 (d, 2H, NHC*H*₂CN) |
| 359 | 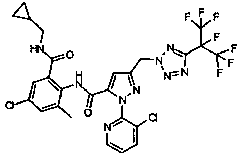 | 4.26 | 694 | DMSO: 0.10 (m, 2H, NHCH₂CH(C*H*₂)₂), 0.30 (m, 2H, NHCH₂CH(C*H*₂)₂), 0.87 (m, 1H, NHCH₂C*H*(CH₂)₂), 3.01 (t, 2H, NHC*H*₂CH(CH₂)₂) |
| 360 | 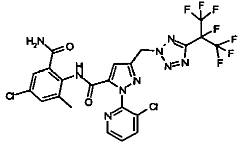 | 3.46 | 640 | CD₃CN: 6.20 (bs, 1H, N*H*₂), 6.97 (bs, 1H, N*H*₂) |
| 361 | 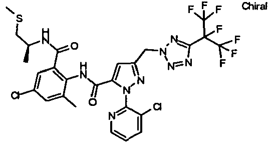 | 4.39 | 728 | DMSO: 1.11 (d, 3H, NHCH(C*H*₃)CH₂SCH₃), 2.15 (s, 3H, NHCH(CH₃)CH₂SC*H*₃), 2.48 (m, 1H, NHCH(CH₃)C*H*₂SCH₃), 2.55 (m, 1H, NHCH(CH₃)C*H*₂SCH₃), 3.98 (m, 1H, NHC*H*(CH)CH₂SCH₃) |
| 362 | 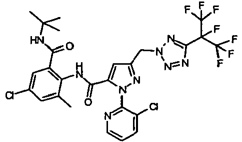 | 4.56 | 696 | DMSO: 1.19 (s, 9H, NHC(C*H*₃)₃) |
| 363 | 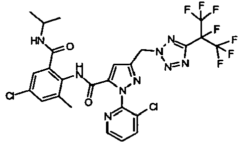 | 4.21 | 682 | DMSO: 1.18 (d, 6H, NHCH(C*H*₃)₂), 3.92 (m, 1H, NHC*H*(CH₃)₂) |
| 364 | 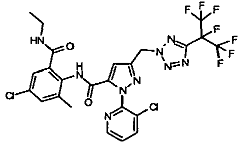 | 3.96 | 668 | DMSO: 0.99 (t, 3H, NHCH₂C*H*₃), 3.13 (m, 2H, NHC*H*₂CH₃) |
| 365 | 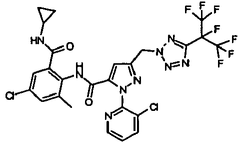 | 3.94 | 680 | DMSO: 0.41 (m, 2H, NHCH(C*H*₂)₂), 0.58 (m, 2H, NHCH(C*H*₂)₂), 2.69 (m, 1H, NHC*H*(CH₂)₂) |
| 366 | 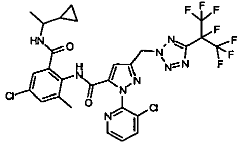 | 4.50 | 708 | DMSO: 0.09-0.36 (m, 4H, NHCH(CH₃)CH(C*H*₂)₂), 0.83 (m, 1H, NHCH(CH₃)C*H*(CH₂)₂), 1.05 (d, 3H, NHCH(C*H*₃)CH(CH₂)₂), 3.34 (m, 1H, NHC*H*(CH₃)CH(CH₂)₂) |
| 367 | 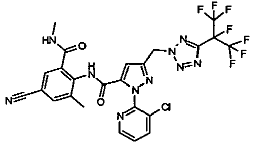 | 3.35 | 645 | DMSO: 2.65 (d, 3H, NHC*H*₃) |
| 368 | 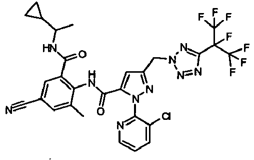 | 4.07 | 699 | DMSO: 0.11-0.38 (m, 4H, NHCH(CH₃)CH(C*H*₂)₂), 0.82 (m, 1H, NHCH(CH₃)C*H*(CH₂)₂), 1.12 (d, 3H, NHCH(C*H*₃)CH(CH₂)₂), 3.34 (m, 1H, NHC*H*(CH₃)CH(CH₂)₂) |
| 369 | 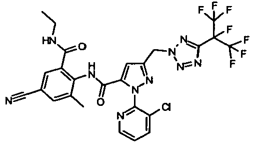 | 3.57 | 659 | DMSO: 0.97 (t, 3H, NHCH₂C*H*₃), 3.12 (m, 2H, NHC*H*₂CH₃) |
| 370 | 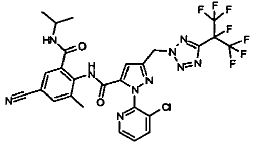 | 3.79 | 673 | DMSO: 1.08 (d, 6H, NHCH(C*H*₃)₂), 3.93 (m, 1H, NHC*H*(CH₃)₂) |
| 371 | 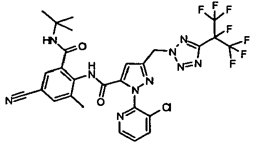 | 4.10 | 687 | DMSO: 1.23 (s, 9H, NHC(C*H*₃)₃) |
| 372 | 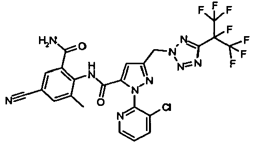 | 3.13 | 631 | DMSO: 7.50-8.00 (bs, 2H, N*H*₂) |
| 373 | 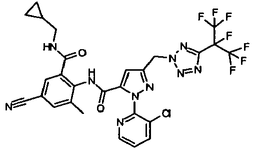 | 3.84 | 685 | DMSO: 0.13 (m, 2H, NHCH₂CH(C*H*₂)₂), 0.33 (m, 2H, NHCH₂CH(C*H*₂)₂), 0.90 (m, 1H, NHCH₂C*H*(CH₂)₂), 3.03 (t, 2H, NHC*H*₂CH(CH₂)₂) |
| 374 | 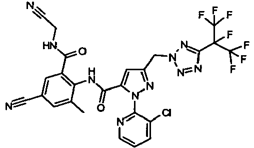 | 3.29 | 670 | DMSO: 4.17 (d, 2H, NHC*H*₂CN) |
| 375 | 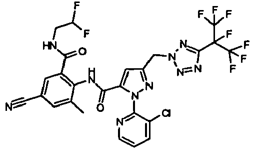 | 3.57 | 695 | DMSO: 3.50 (m, 2H, NHC*H*₂CHF₂), 5.83 (tt, 1H, NHCH₂C*H*F₂) |
| 376 | 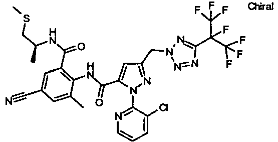 | 3.96 | 719 | DMSO: 1.10 (d, 3H, NHCH(C*H*₃)CH₂SCH₃), 2.20 (s, 3H, NHCH(CH₃)CH₂SC*H*₃), 2.46 (m, 1H, NHCH(CH₃)C*H*₂SCH₃), 2.51 (m, 1H, NHCH(CH₃)C*H*₂SCH₃), 3.98 (m, 1H, NHC*H*(CH₃)CH₂SCH₃) |
| 377 | 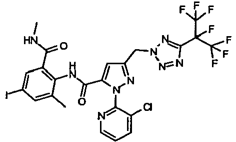 | 3.94 | 746 | DMSO: 2.65 (d, 3H, NHC*H*₃) |
| 378 | 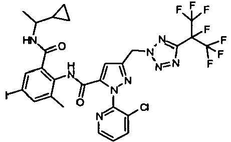 | 4.74 | 800 | DMSO: 0.09-0.37 (m, 4H, NHCH(CH₃)CH(C*H*₂)₂), 0.83 (m, 1H, NHCH(CH₃)C*H*(CH₂)₂), 1.05 (d, 3H, NHCH(C*H*₃)CH(CH₂)₂), 3.32 (m, 1H, NHC*H*(CH₃)CH(CH₂)₂) |
| 379 | 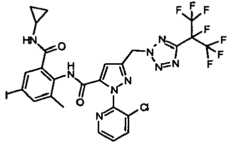 | 4.18 | 772 | DMSO: 0.40 (m, 2H, NHCH(C*H*₂)₂), 0.58 (m, 2H, NHCH(C*H*₂)₂), 2.64 (m, 1H, NHC*H*(CH₂)₂) |
| 380 | 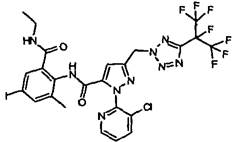 | 4.20 | 760 | DMSO: 0.99 (t, 3H, NHCH₂C*H*₃), 3.13 (m, 2H, NHC*H*₂CH₃) |
| 381 | 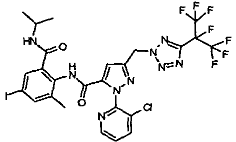 | 4.44 | 774 | DMSO: 1.80 (d, 6H, NHCH(C*H*₃)₂), 3.91 (m, 1H, NHC*H*(CH₃)₂) |
| 382 | 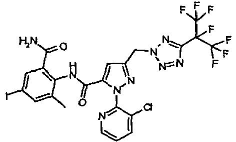 | 3.67 | 732 | DMSO: 7.20-7.60 (bs, 2H, N*H*₂) |
| 383 | 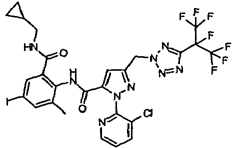 | 4.51 | 786 | DMSO: 0.02 (m, 2H, NHCH₂CH(C*H*₂)₂), 0.22 (m, 2H, NHCH₂CH(C*H₂*)₂), 0.77 (m, 1H, NHCH₂CH(C*H*₂)₂), 2.91 (t, 2H, NHC*H*₂CH(CH₂)₂) |
| 384 | 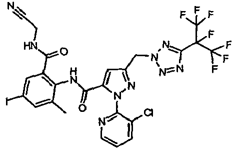 | 3.82 | 771 | DMSO: 4.14 (d, 2H, NHC*H*₂CN) |
| 385 | 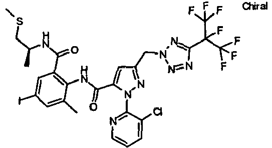 | 4.63 | 820 | DMSO: 1.09 (d, 3H, NHCH(C*H*₃)CH₂SCH₃), 2.15 (s, 3H, NHCH(CH₃)CH₂SC*H*₃), 2.47 (m, 1H, NHCH(CH₃)C*H*₂SCH₃), 2.55 (m, 1H, NHCH(CH₃)C*H*₂SCH₃), 3.97 (m, 1H, NHC*H*(CH₃)CH₂SCH₃) |
| 386 | 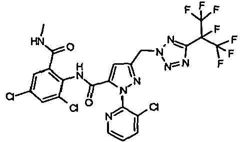 | 3.65 | 674 | DMSO: 2.65 (d, 3H, NHC*H*₃) |
| 387 | 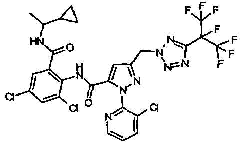 | 4.42 | 728 | DMSO: 0.09-0.37 (m, 4H, NHCH(CH₃)CH(C*H*₂)₂), 0.81 (m, 1H, NHCH(CH₃)C*H*(CH₂)₂), 1.04 (d, 3H, NHCH(C*H*₃)CH(CH₂)₂), 3.30 (m, 1H, NHC*H*(CH₃)CH(CH₂)₂) |
| 388 | 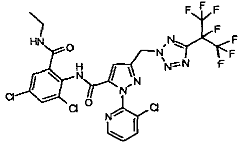 | 3.89 | 688 | DMSO: 0.98 (t, 3H, NHCH₂C*H*₃), 3.15 (m, 2H, NHC*H*₂CH₃) |
| 389 | 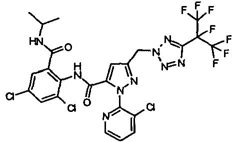 | 4.12 | 702 | DMSO: 1.00 (d, 6H, NHCH(C*H*₃)₂), 3.89 (m, 1H, NHC*H*(CH₃)₂) |
| 390 | 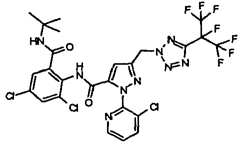 | 4.47 | 716 | DMSO: 1.20 (s, 9H, NHC(C*H*₃)₃) |
| 391 | 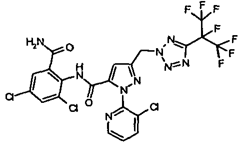 | 3.41 | 660 | DMSO: 7.40 (bs, 1H, N*H*₂), 7.77 (bs, 1H, N*H*₂) |
| 392 | 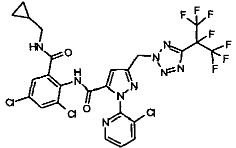 | 4.17 | 714 | DMSO: 0.09 (m, 2H, NHCH₂CH(C*H*₂)₂), 0.31 (m, 2H, NHCH₂CH(C*H*₂)₂), 0.86 (m, 1H, NHCH₂C*H*(CH₂)₂), 2.99 (t, 2H, NHC*H*₂CH(CH₂)₂) |
| 393 | 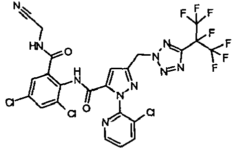 | 3.59 | 699 | DMSO: 4.15 (d, 2H, NHC*H*₂CN) |
| 394 | 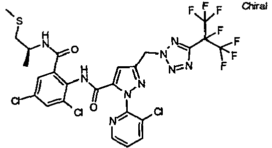 | 4.30 | 748 | DMSO: 1.04 (d, 3H, NHCH(C*H*₃)CH₂SCH₃), 2.07 (s, 3H, NHCH(CH₃)CH₂SC*H*₃), 2.40 (m, 1H, NHCH(CH₃)C*H*₂SCH₃), 2.55 (m, 1H, NHCH(CH₃)C*H*₂SCH₃), 3.92 (m, 1H, NHC*H*(CH₃)CH₂SCH₃) |
| 395 | 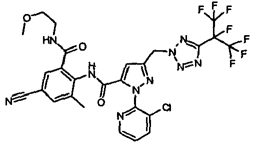 | 3.48 | 689 | DMSO: 3.20 (s, 3H, NHCH₂CH₂OC*H*₃), 3.28-3.40 (m, 4H, NHCH₂C*H*₂OCH₃) |
| 396 | 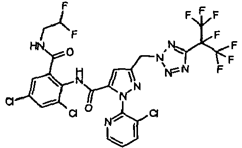 | 3.89 | 724 | DMSO: 3.50 (m, 2H, NHC*H*₂CHF₂), 5.86 (tt, 1H, NHCH₂C*H*F₂) |
| 397 | 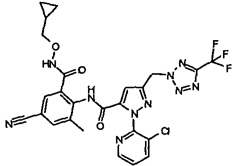 | 2.96 | 601 | DMSO: 0.23 (m, 2H, NHOCH₂CH(C*H*₂)₂), 0.52 (m, 2H, NHOCH₂CH(C*H*₂)₂), 1.07 (m, 1H, NHOCH₂C*H*(CH₂)₂), 3.66 (d, 2H, NHOC*H*₂CH(CH₂)₂) |
| 398 | 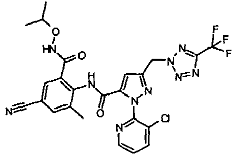 | 2.88 | 589 | DMSO: 1.16 (d, 6H, NHOCH(C*H*₃)₂), 4.08 (m, 1H, NHOC*H*(CH₃)₂) |
| 399 | 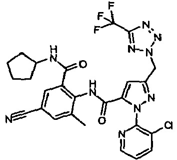 | 3.37 | 599 | DMSO: 1.34-1.77 (m, 8H, NHCH(C*H*₂)₄), 4.04 (m, 1H, NHC*H*(CH₂)₄) |
| 400 | 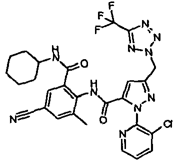 | 3.63 | 613 | DMSO: 1.04-1.80 (m, 10H, NHCH(C*H*₂)₅), 3.56 (m, 1H, NHC*H*(CH₂)₅) |
| 401 | 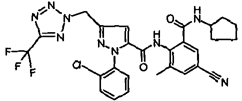 | 3.84 | 598 | DMSO: 1.40-1.77 (m, 8H, NHCH(C*H*₂)₄), 4.02-4.05 (m, 1H, NHC*H*(CH₂)₄) |
| 402 | 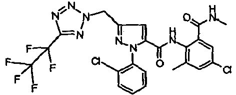 | 3.78 | 603 | DMSO: 2.65 (d, 3H, NHC*H*₃) |
| 403 | 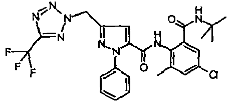 | 4.14 | 561 | DMSO: 1.24 (s, 9H, NHC(C*H*₃)₃) |
| 404 | 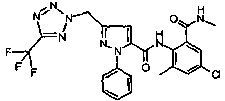 | 3.19 | 519 | DMSO: 2.72 (d, 3H, NHC*H*₃) |
| 405 | 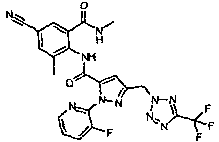 | 2.46 | 529 | DMSO: 2.67 (d, 3H, NHC*H*₃) |
| 406 | 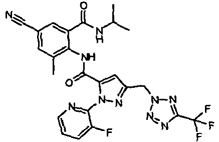 | 2.88 | 557 | DMSO: 1.01 (d, 6H, NHCH(C*H*₃)₂), 3.86-3.91 (m, 1H, NHC*H*(CH₃)₂) |
| 407 | 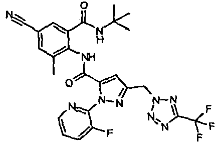 | 3.20 | 571 | DMSO: 1.21 (s, 9H, NHC(C*H*₃)₃) |
| 408 | 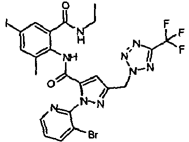 | 3.51 | 704 | DMSO: 0.97 (t, 3H, NHCH₂C*H*₃), 3.12 (q, 2H, NHC*H*₂CH₃) |
| 409 | 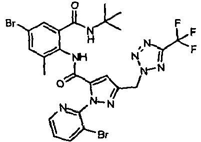 | 4.00 | 684 | DMSO: 1.20 (s, 9H, NHC(C*H*₃)₃) |
| 410 | 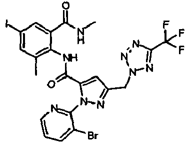 | 3.26 | 690 | DMSO: 2.64 (d, 3H, NHC*H*₃) |
| 411 | 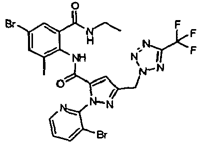 | 3.36 | 656 | DMSO: 0.97 (t, 3H, NHCH₂C*H*₃), 3.13 (q, 2H, NHC*H*₂CH₃) |
| 412 | 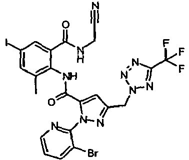 | 3.16 | 716 | DMSO: 4.15 (d, 2H, NHC*H*₂CN) |
| 413 | 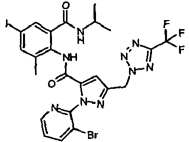 | 3.77 | 718 | DMSO: 1.00 (d, 6H, NHCH(C*H*₃)₂), 3.86-3.91 (m, 1H, NHC*H*(CH₃)₂) |
| 414 | 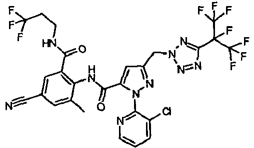 | 3.92 | 727 | DMSO: 2.49 (m, 2H, NHCH₂C*H*₂CF₃), 3.49 (m, 2H, NHC*H*₂CH₂CF₃) |
| 415 | 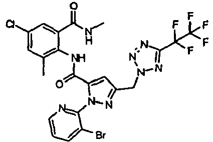 | 3.36 | 649 | DMSO: 2.65 (d, 3H, NHC*H*₃) |
| 416 | 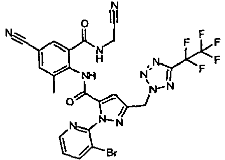 | 2.80 | 665 | DMSO: 4.18 (d, 2H, NHC*H*₂CN) |
| 417 | 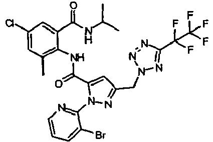 | 3.87 | 677 | DMSO: 1.00 (d, 6H, NHCH(C*H*₃)₂), 3.86-3.91 (m, 1H, NHC*H*(CH₃)₂) |
| 418 | 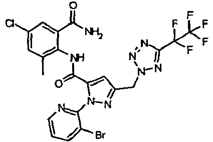 | 3.13 | 635 | DMSO: 7.45 (bs, 1H, N*H*₂), 7.71 (bs, 1H, N*H*₂) |
| 419 | 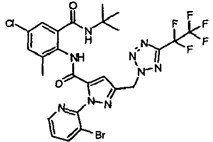 | 4.23 | 691 | DMSO: 1.19 (s, 9H, NHC(C*H*₃)₃) |
| 420 | 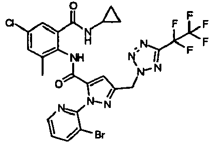 | 3.62 | 677 | DMSO: 0.39-0.43 (m, 2H, NHCH(C*H*₂)₂), 0.55-0.61 (m, 2H, NHCH(C*H*₂)₂), 2.64-2.70 (m, 1H, NHC*H*(CH₂)₂) |
| 421 | 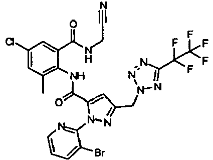 | 3.31 | 674 | DMSO: 4.16 (d, 2H, NHC*H*₂CN) |
| 422 | 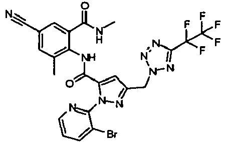 | 3.03 | 640 | DMSO: 2.65 (d, 3H, NHC*H*₃) |
| 423 | 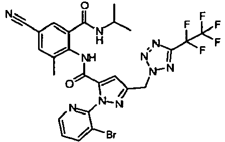 | 3.46 | 668 | DMSO: 1.01 (d, 6H, NHCH(C*H*₃)₂), 3.86-3.92 (m, 1H, NHC*H*(CH₃)₂) |
| 424 | 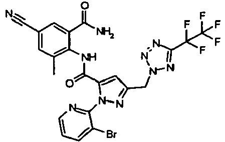 | 2.80 | 626 | DMSO: 7.55 (bs, 1H, N*H*₂), 7.86 (bs, 1H, N*H*₂) |
| 425 | 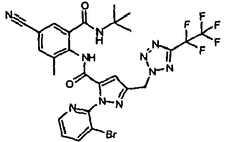 | 3.78 | 682 | DMSO: 1.21 (s, 9H, NHC(C*H*₃)₃) |
| 426 | 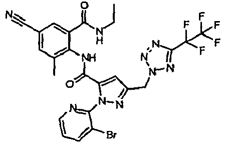 | 3.23 | 654 | DMSO: 0.98 (t, 3H, NHCH₂C*H*₃), 3.14 (q, 2H, NHC*H*₂CH₃) |
| 427 | 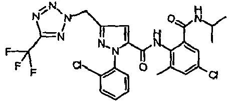 | 3.93 | 582 | DMSO: 0.99-1.03 (m, 6H, NHCH(C*H*₃)₂), 3.86-3.91 (m, 1H, NHC*H*(CH₃)₂) |
| 428 | 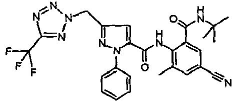 | 3.72 | 552 | DMSO: 1.25 (s, 9H, NHC(C*H*₃)₃) |
| 429 | 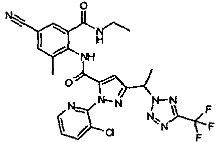 | 3.11 | 574 | DMSO: 0.98 (t, 3H, NHCH₂C*H*₃), 3.13 (q, 2H, NHC*H*₂CH₃) |
| 430 | 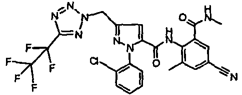 | 3.43 | 594 | DMSO: 2.65 (d, 3H, NHC*H*₃) |
| 431 | 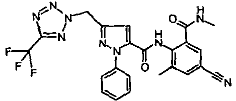 | 2.81 | 510 | DMSO: 2.72 (d, 3H, NHC*H*₃) |
| 432 | 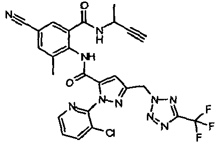 | 2.97 | 584 | DMSO: 1.21 (d, 3H, NHCH(C*H*₃)C≡CH), 3.12 (s, 1H, NHCH(CH₃)C≡C*H*), 4.61-4.65 (m, 1H, NHC*H*(CH₃)C≡CH) |
| 433 | 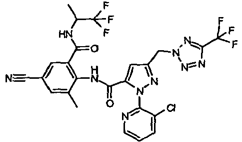 | 3.25 | 628 | DMSO: 1.13 (d, 3H, NHCH(C*H*₃)CF₃), 4.52-4.58 (m, 1H, NHC*H*(CH₃)CF₃) |
| 434 | 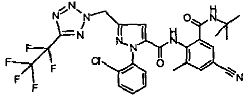 | 4.25 | 636 | DMSO: 1.20 (s, 9H, NHC(C*H*₃)₃) |
| 435 | 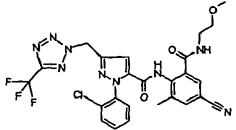 | 3.18 | 588 | DMSO: 2.20 (s, 3H, NH(CH₂)₂OC*H*₃), 3.26-3.30 (m, 4H, NH(C*H*₂)₂OCH₃) |
| 436 | 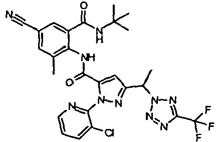 | 3.70 | 601 | DMSO: 1.20 (s, 9H, NHC(C*H*₃)₃) |
| 437 | 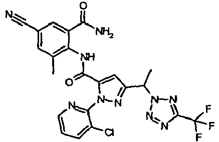 | 2.66 | 545 | DMSO: 7.55 (bs, 1H, N*H*₂), 7.85 (bs, 1H, N*H*₂) |
| 438 | 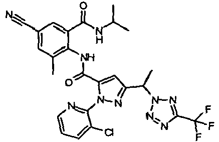 | 3.35 | 587 | DMSO: 1.01 (d, 6H, NHCH(C*H*₃)₂), 3.84-3.93 (m, 1H, NHC*H*(CH₃)₂) |
| 439 | 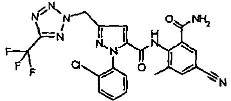 | 2.82 | 530 | DMSO: 7.81-7.89 (m, 2H, N*H*₂) |
| 440 | 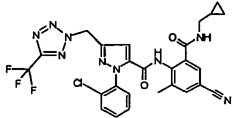 | 3.61 | 584 | DMSO: 0.04-0.11 (m, 2H, NHCH₂CH(C*H*₂)₂), 0.27-0.31 (m, 2H, NHCH₂CH(C*H*₂)₂), 0.80-0.84 (m, 1H, NHCH₂C*H*(CH₂)₂), 2.97-2.99 (m, 2H, NHC*H*₂CH(CH₂)₂) |
| 441 | 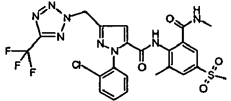 | 2.73 | 597 | DMSO: 2.68 (d, 3H, NHC*H*₃) |
| 442 | 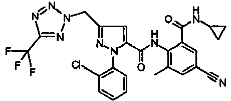 | 3.29 | 570 | DMSO: 0.40-0.43 (m, 2H, NHCH(C*H*₂)₂), 0.60-0.62 (m, 2H, NHCH(C*H*₂)₂), 2.66-2.67 (m, 1H, NHC*H*(CH₂)₂) |
| 443 | 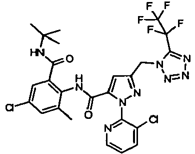 | 3.92 | 646 | DMSO: 1.30 (s, 9H, NHC(C*H*₃)₃) |
| 444 | 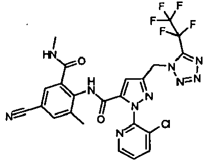 | 2.73 | 595 | CD₃CN: 2.79 (d, 3H, NHC*H*₃) |
| 445 | 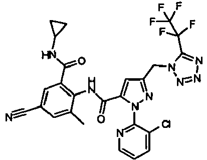 | 2.95 | 621 | DMSO: 0.48 (m, 2H, NHCH(C*H*₂)₂), 0.62 (m, 2H, NECH(C*H*₂)₂), 2.65 (m, 1H, NHC*H*(CH₂)₂) |
| 446 | 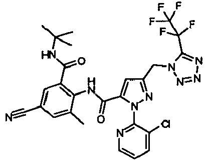 | 3.47 | 637 | DMSO: 1.32 (s, 9H, NHC(C*H*₃)₃) |
| 447 | 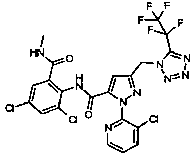 | 3.03 | 624 | DMSO: 2.74 (d, 3H, NHC*H*₃) |
| 448 | 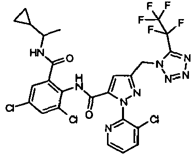 | 3.81 | 678 | DMSO: 0.10-0.37 (m, 4H, NHCH(CH₃)CH(C*H*₂)₂), 0.81 (m, 1H, NHCH(CH₃)C*H*(CH₂)₂), 1.04 (d, 3H, NHCH(C*H*₃)CH(CH₂)₂), 3.29 (m, 1H, NHC*H*(CH₃)CH(CH₂)₂) |
| 449 | 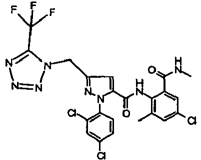 | 3.64 | 589 | DMSO: 2.66 (d, 3H, NHC*H*₃) |
| 450 | 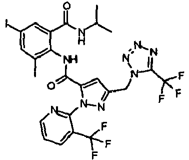 | 3.54 | 707 | DMSO: 0.96 (d, 6H, NHCH(C*H*₃)₂), 3.83-3.88 (m, 1H, NHC*H*(CH₃)₂) |
| 451 | 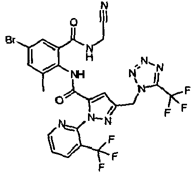 | 2.90 | 657 | DMSO: 4.14 (d, 2H, NHC*H*₂CN) |
| 452 | 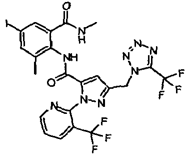 | 3.07 | 680 | DMSO: 2.65 (d, 3H, NHC*H*₃) |
| 453 | 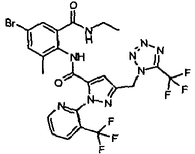 | 3.17 | 648 | DMSO: 0.94 (t, 3H, NHCH₂C*H*₃), 3.10 (q, 2H, NHC*H*₂CH₃) |
| 454 | 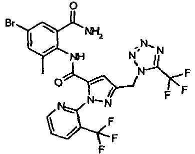 | 2.72 | 617 | DMSO: 7.30 (bs, 1H, N*H*₂), 7.50 (bs, 1H, N*H*₂) |
| 455 | 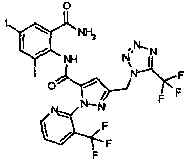 | 2.83 | 666 | DMSO: 7.20 (bs, 1H, N*H*₂), 7.28 (bs, 1H, N*H*₂) |
| 456 | 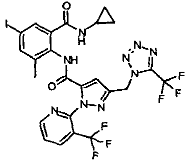 | 3.31 | 706 | DMSO: 0.41-0.46 (m, 2H, NHCH(C*H*₂), 0.57-0.61 (m, 2H, NHCH(C*H*₂)₂), 2.65-2.70 . (m, 1H, NHC*H*(CH₂)₂) |
| 457 | 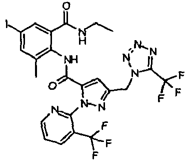 | 3.30 | 694 | DMSO: 0.99 (t, 3H, NHCH₂C*H*₃), 3.14 (q, 2H, NHC*H*₂CH₃) |
| 458 | 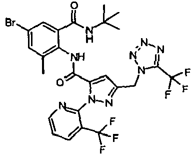 | 3.78 | 674 | DMSO: 1.20 (s, 9H, NHC(C*H*₃)₃) |
| 459 | 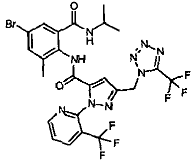 | 3.29 | 660 | DMSO: 1.02 (d, 6H, NHCH(C*H*₃)₂), 3.88-3.93 (m, 1H, NHC*H*(CH₃)₂) |
| 460 | 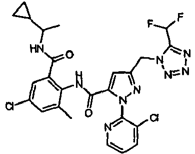 | 3.07 | 590 | DMSO: 0.11-0.37 (m, 4H, NHCH(CH₃)CH(C*H*₂)₂), 0.83 (m, 1H, NHCH(CH₃)C*H*(CH₂)₂), 1.06 (d, 3H, NHCH(C*H*₃)CH(CH₂)₂), 3.33 (m, 1H, NHC*H*(CH₃)CH(CH₂)₂) |
| 461 | 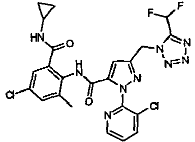 | 2.50 | 562 | DMSO: 0.43 (m, 2H, NHCH(C*H*₂)₂), 0.60 (m, 2H, NHCH(C*H*₂)₂), 2.69 (m, 1H, NHC*H*(CH₂)₂) |
| 462 | 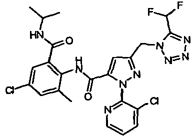 | 2.75 | 564 | DMSO: 1.03 (d, 6H, NHCH(C*H*₃)₂), 3.92 (m, 1H, NHC*H*(CH₃)₂) |
| 463 | 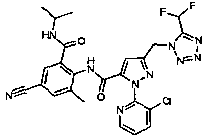 | 2.36 | 555 | DMSO: 1.08 (d, 6H, NHCH(C*H*₃)₂), 3.93 (m, 1H, NHC*H*(CH₃)₂) |
| 464 | 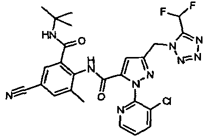 | 2.59 | 569 | DMSO: 1.36 (s, 9H, NHC(C*H*₃)₃) |
| 465 | 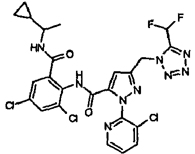 | 3.02 | 610 | DMSO: 0.11-0.37 (m, 4H, NHCH(CH₃)CH(C*H*₂)₂), 0.80 (m, 1H, NHCH(CH₃)C*H*(CH₂)₂), 1.04 (d, 3H, NHCH(C*H*₃)CH(CH₂)₂), 3.30 (m, 1H, NHC*H*(CH₃)CH(CH₂)₂) |
| 466 | 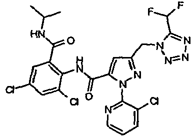 | 2.71 | 584 | DMSO: 1.01 (d, 6H, NHCH(C*H*₃)₂), 3.89 (m, 1H, NHC*H*(CH₃)₂) |
| 467 | 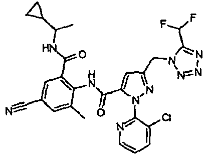 | 2.58 | 581 | DMSO: 0.12-0.39 (m, 4H, NHCH(CH₃)CH(C*H*₂)₂), 0.85 (m, 1H, NHCH(CH₃)C*H*(CH₂)₂), 1.07 (d, 3H, NHCH(C*H*₃)CH(CH₂)₂), 3.34 (m, 1H, NHC*H*(CH₃)CH(CH₂)₂) |
| 468 | 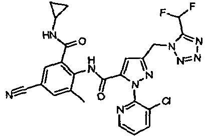 | 2.12 | 553 | DMSO: 0.50 (m, 2H, NHCH(C*H*₂)₂), 0.62 (m, 2H, NHCH(C*H*₂)₂), 2.70 (m, 1H, NHC*H*(CH₂)₂) |
| 469 | 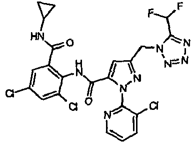 | 2.44 | 582 | DMSO: 0.42 (m, 2H, NHCH(C*H*₂)₂), 0.58 (m, 2H, NHCH(C*H*₂)₂), 2.66 (m, 1H, NHC*H*(CH₂)₂) |
| 470 | 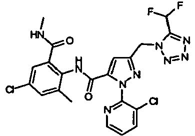 | 2.26 | 536 | CD₃CN: 2.75 (d, 3H, NHC*H*₃) |
| 471 | 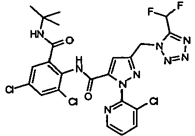 | 3.05 | 598 | DMSO: 1.27 (s, 9H, NHC(C*H*₃)₃) |
| 472 | 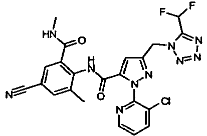 | 1.97 | 527 | CD₃CN: 2.78 (d, 3H, NHC*H*₃) |
| 473 | 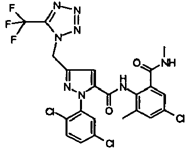 | 3.52 | 589 | DMSO: 2.67 (d, 3H, NHC*H*₃) |
| 474 | 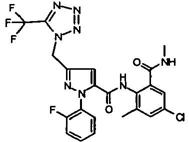 | 2.94 | 537 | DMSO: 2.67 (d, 3H, NHC*H*₃) |
| 475 | 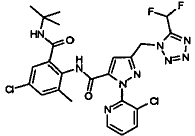 | 3.09 | 578 | CD₃CN: 1.30 (s, 9H, NHC(C*H*₃)₃) |
| 476 | 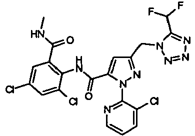 | 2.22 | 556 | DMSO: 2.65 (d, 3H, NHC*H*₃) |
| 477 | 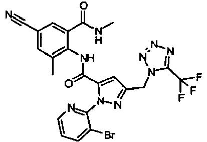 | 2.47 | 589 | |
| 478 | 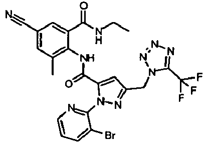 | 2.66 | 603 | |
| 479 | 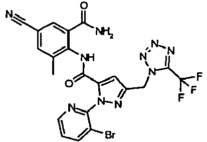 | 2.28 | 575 | |
| 480 | 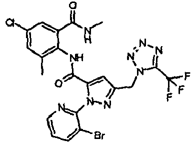 | 2.79 | 598 | |
| 481 | 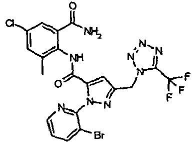 | 2.43 | 584 | |
| 482 | 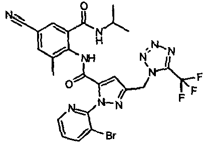 | 2.74 | 617 | |
| 483 | 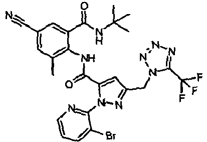 | 3.07 | 631 | |
| 484 | 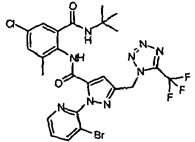 | 3.52 | 640 | |
| 485 | 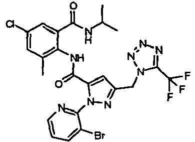 | 3.14 | 626 | |
| 486 | 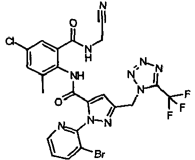 | 2.64 | 623 | |
| 487 | 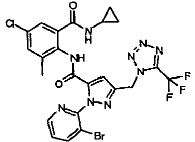 | 2.90 | 624 | |
| 488 | 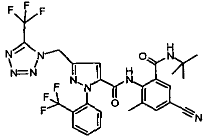 | 3.58 | 620 | DMSO: 1.23 (s, 9H, NHC(C*H*₃)₃) |
| 489 | 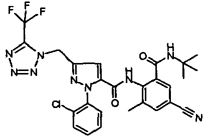 | 3.52 | 586 | DMSO: 1.23 (s, 9H, NHC(C*H*₃)₃) |
| 490 | 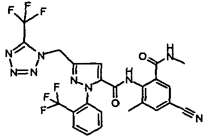 | 2.91 | 578 | DMSO: 2.66 (d, 3H, NHC*H*₃) |
| 491 | 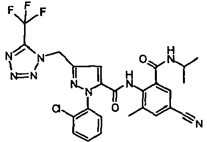 | 3.25 | 572 | DMSO: 1.02-1.04 (m, 6H, NHCH(C*H*₃)₂), 3.90-3.95 (m, 1H, NHC*H*(CH₃)₂) |
| 492 | 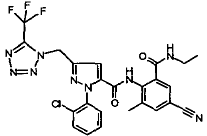 | 3.01 | 558 | DMSO: 1.02 (t, 3H, NHCH₂C*H*₃), 3.08 (q, 2H, NHC*H*₂CH₃) |
| 493 | 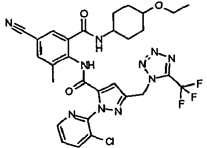 | 3.37 | 657 | |
| 494 | 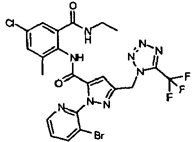 | 2.88 | 612 | |
| 495 | 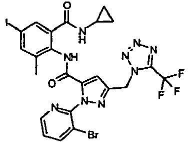 | 3.11 | 716 | DMSO: 0.39-0.43 (m, 2H, NHCH(C*H*₂)₂), 0.56-0.61 (m, 2H, NHCH(C*H*₂)₂), 2.63-2.67 (m, 1H, NHC*H*(CH₂)₂) |
| 496 | 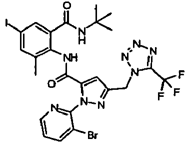 | 3.73 | 732 | DMSO: 1.20 (s, 9H, NHC(C*H*₃)₃) |
| 497 | 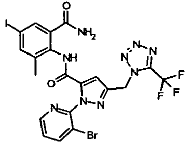 | 2.63 | 676 | DMSO: 7.43 (bs, 1H, N*H*₂), 7.70 (bs, 1H, N*H*₂) |
| 498 | 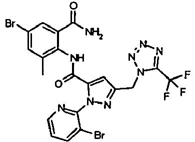 | 2.51 | 628 | DMSO: 7.49 (bs, 1H, N*H*₂), 7.70 (bs, 1H, N*H*₂) |
| 499 | 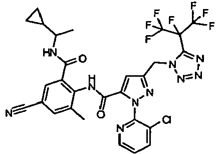 | 3.80 | 699 | DMSO: 0.12-0.48 (m, 4H, NHCH(CH₃)CH(C*H*₂)₂), 0.85 (m, 1H, NHCH(CH₃)C*H*(CH₂)₂), 1.06 (d, 3H, NHCH(C*H*₃)CH(CH₂)₂), 3.36 (m, 1H, NHC*H*(CH₃)CH(CH₂)₂) |
| 500 | 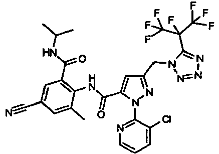 | 3.50 | 673 | DMSO: 1.04 (d, 6H, NHCH(C*H*₃)₂), 3.92 (m, 1H, NHC*H*(CH₃)₂) |
| 501 | 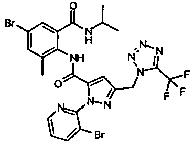 | 3.27 | 671 | DMSO: 1.01 (d, 6H, NHCH(C*H*₃)₂), 3.86-3.91 (m, 1H, NHC*H*(CH₃)₂) |
| 502 | 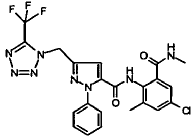 | 2.84 | 519 | DMSO: 2.72 (d, 3H, NHC*H*₃) |
| 503 | 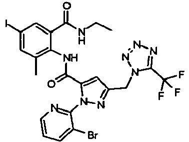 | 3.16 | 704 | DMSO: 0.98 (t, 3H, NHCH₂C*H*₃), 3.12 (q, 2H, NHC*H*₂CH₃) |
| 504 | 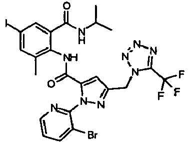 | 3.41 | 718 | DMSO: 1.00 (d, 6H, NHCH(C*H*₃)₂), 3.86-3.91 (m, 1H, NHC*H*(CH₃)₂) |
| 505 | 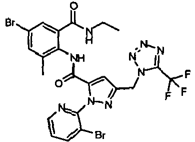 | 3.02 | 656 | DMSO: 0.98 (t, 3H, NHCH₂C*H*₃), 3.13 (q, 2H, NHC*H*₂CH₃) |
| 506 | 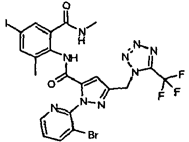 | 2.91 | 690 | DMSO: 2.64 (d, 3H, NHC*H*₃) |
| 507 | 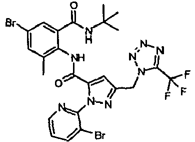 | 3.65 | 684 | DMSO: 1.20 (s, 9H, NHC(C*H*₃)₃) |
| 508 | 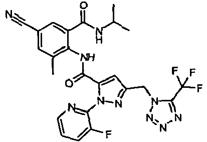 | 2.55 | 556 | |
| 509 | 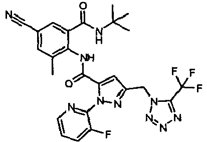 | 2.92 | 570 | |
| 510 | 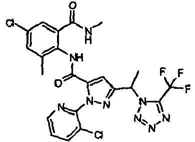 | 2.86 | 568 | |
| 511 | 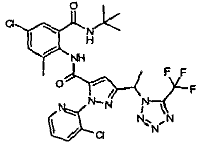 | 3.75 | 610 | |
| 512 | 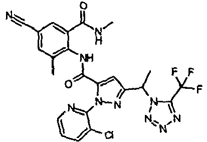 | 2.53 | 558 | |
| 513 | 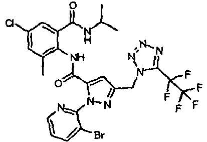 | 3.54 | 677 | DMSO: 1.01 (d, 6H, NHCH(C*H*₃)₂), 3.85-3.93 (m, 1H, NHC*H*(CH₃)₂) |
| 514 | 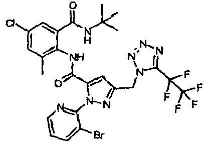 | 3.91 | 691 | DMSO: 1.20 (s, 9H, NHC(C*H*₃)₃) |
| 515 | 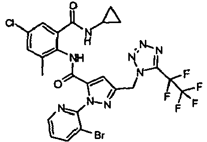 | 3.29 | 675 | DMSO: 0.39-0.43 (m, 2H, NHCH(C*H*₂)₂), 0.56-0.61 (m, 2H, NHCH(C*H*₂)₂), 2.63-2.69 (m, 1H, NHC*H*(CH₂)₂) |
| 516 | 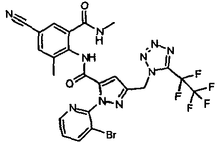 | 2.72 | 640 | DMSO: 2.66 (d, 3H, NHC*H*₃) |
| 517 | 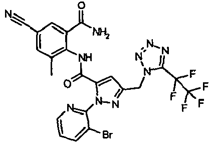 | 2.53 | 626 | DMSO: 7.56 (bs, 1H, N*H*₂), 7.86 (bs, 1H, N*H*₂) |
| 518 | 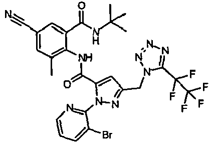 | 3.47 | 682 | DMSO: 1.21 (s, 9H, NHC(C*H*₃)₃) |
| 519 | 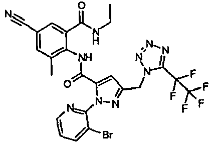 | 2.94 | 654 | DMSO: 0.99 (t, 3H, NHCH₂C*H*₃), 3.14 (q, 2H, NHC*H*₂CH₃) |
| 520 | 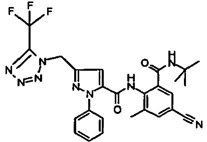 | 3.35 | 552 | DMSO: 1.25 (s, 9H, NHC(C*H*₃)₃) |
| 521 | 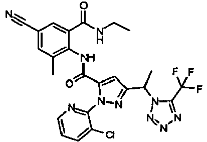 | 2.74 | 572 | |
| 522 | 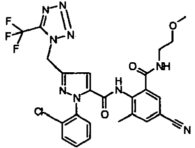 | 2.80 | 598 | DMSO: 2.33 (s, 3H, NH(CH₂)₂OC*H*₃), 3.26-3.29 (m, 4H, NH(C*H*₂)₂OCH₃) |
| 523 | 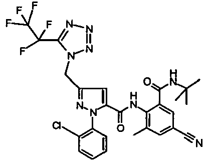 | 3.92 | 636 | DMSO: 1.20 (s, 9H, NHC(C*H*₃)₃) |
| 524 | 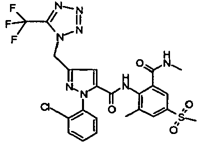 | 2.43 | 597 | DMSO: 2.68 (d, 3H, NHC*H*₃) |
| 525 | 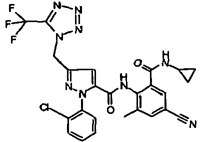 | 2.95 | 570 | DMSO: 0.39-0.46 (m, 2H, NHCH(C*H*₂)₂), 0.60-0.63 (m, 2H, NHCH(C*H*₂)₂), 2.63-2.68 (m, 1H, NHC*H*(CH₂)₂) |
| 526 | 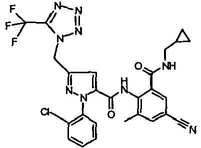 | 3.25 | 584 | DMSO: 0.28-0.31 (m, 2H, NHCH₂CH(C*H*₂)₂), 0.38-0.39 (m, 2H, NHCH₂CH(C*H*₂)₂), 0.84-0.91 (m, 1H, NHCH₂C*H*(CH₂)₂), 2.97-3.00 (m, 2H, NHC*H*₂CH(CH₂)₂) |
| 527 | 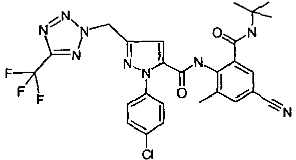 | 4.16 | 586 | DMSO: 1.23 (s, 9H, NHC(C*H*₃)₃) |
| 528 | 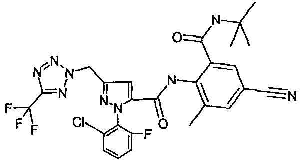 | 3.88 | 604 | DMSO: 1.18 (s, 9H, NHC(C*H*₃)₃) |
| 529 | 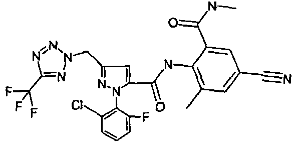 | 3.13 | 562 | DMSO: 2.66 (d, 3H, NHC*H*₃) |
| 530 | 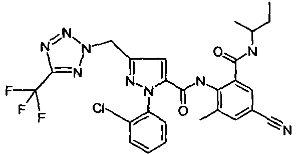 | 3.81 | 586 | DMSO: 0.76 (t, 3H, NHCH(CH₃)CH₂C*H*₃), 0.97 (d, 3H, NHCH(C*H*₃)CH₂CH₃), 1.33-1.42 (m, 2H, NHCH(CH₃)C*H*₂CH₃), 3.71-3.75 (m, 1H, NHC*H*(CH₃)CH₂CH₃) |
| 531 | 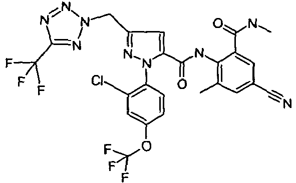 | 3.82 | 628 | DMSO: 2.65 (d, 3H, NHC*H*₃) |
| 532 | 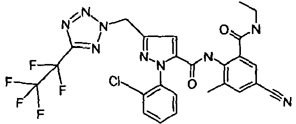 | 3.87 | 608 | DMSO: 1.00 (t, 3H, NHCH₂C*H*₃), 3.16 (q, 2H, NHC*H*₂CH₃) |
| 533 | 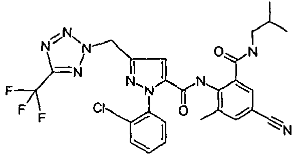 | 3.84 | 586 | DMSO: 0.81 (d, 6H, NHCH₂CH(C*H*₃)₂), 1.64-1.71 (m, 1H, NHCH₂C*H*(CH₃)₂), 2.91-2.94 (m, 2H, NHC*H*₂CH(CH₃)₂) |
| 534 | 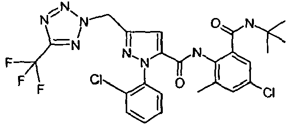 | 4.36 | 595 | DMSO: 1.19 (s, 9H, NHC(C*H*₃)₃) |
| 535 | 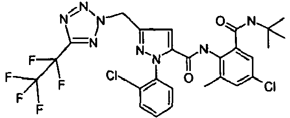 | 4.73 | 646 | DMSO: 1.19 (s, 9H, NHC(C*H*₃)₃) |
| 536 | 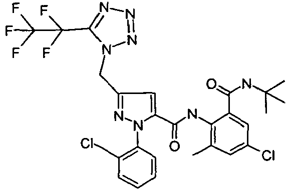 | 4.41 | 646 | DMSO: 1.18 (s, 9H, NHC(C*H*₃)₃) |
| 537 | 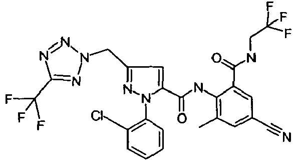 | 3.48 | 612 | DMSO: 3.84-3.93 (m, 2H, NHC*H*₂CF₃) |
| 538 | 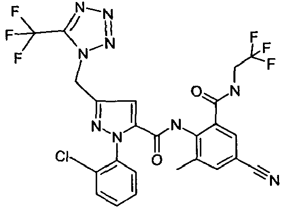 | 3.20 | 612 | DMSO: 3.91-4.00 (m, 2H, NHC*H*₂CF₃) |
| 539 | 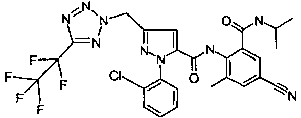 | 3.91 | 622 | DMSO: 1.05 (d, 6H, NHCH(C*H*₃)₂), 3.86-3.91 (m, 1H, NHC*H*(CH₃)₂) |
| 540 | 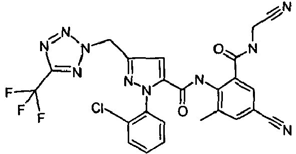 | 2.97 | 569 | DMSO: 4.15 (d, 2H, NHC*H*₂CN) |
| 541 | 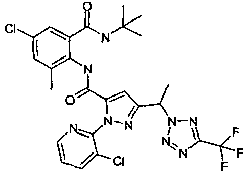 | 4.10 | 611 | DMSO: 1.19 (s, 9H, NHC(C*H*₃)₃) |
| 542 | 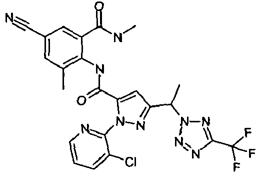 | 2.86 | 559 | DMSO: 2.65 (d, 3H, NHC*H*₃) |
| 543 | 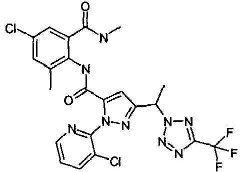 | 3.26 | 569 | DMSO: 2.64 (d, 3H, NHC*H*₃) |
| 544 | 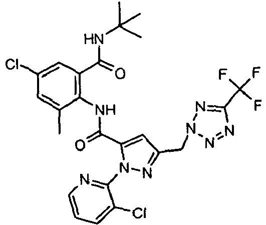 | 3.85 | 595 | DMSO: 1.22 (s, 9H, NHC(C*H*₃)₃) |

### Analytische Methoden:

Die Bestimmung der in der voranstehenden Tabelle und dem Herstellungsbeispiel angegebenen logP-Werte erfolgte gemäß EEC-Directive 79/831 Annex V.A8 durch HPLC.

Die Kalibrierung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die Messungen erfolgten an einem Agilent 1100 LC-System mit einer 50*4,6 Zorbax Eclipse C18 1,8µm-Säule (Ofentemperatur 55°C, Fluss 2,0 ml/min).

Es wurde folgende Eluenten verwendet:

| | |
|---|---|
| Eluent A: | Acetonitril + 1000µl Ameisensäure /1 |
| Eluent B: | Millipore Wasser + 900µl Ameisensäure /1 |

### Gradient:

| Time/ min | Eluent A/% | Eluent B/% |
|---|---|---|
| 0 | 10 | 90 |
| 4,25 | 95 | 5 |
| 5,50 | 95 | 5 |
| 5,55 | 10 | 90 |
| 5,70 | 10 | 90 |

Die MH⁺-Signale wurden mit einem Agilent MSD-System mit ESI und positiver oder negativer Ionisation bestimmt.

Die NMR-Spektren wurden mit einem Bruker Avance 400, ausgestattet mit einem Durchflussprobenkopf (60µl Volumen), bestimmt. Als Lösungsmittel wurden CD₃CN oder d6-DMSO-verwendet, wobei als Referenz Tetramethylsilan (0.00 ppm) eingesetzt wurde.

Die Aufspaltung der Signale wurde wie folgt beschrieben: s (Singulett), bs (breites Singulett), d (Duplett), t (Triplett), q (Quartett), m (Multiplett).

### Anwendungsbeispiele

### Beispiel A

### Phaedon-Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von ≥ 80 % bei einer Aufwandmenge von 100 g / ha:

### Bsp Nr:

1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 36, 37, 38, 39, 40, 41, 42, 43, 44, 45, 46, 47, 48, 49, 50, 52, 53, 54, , 55, 56, 57, 58, 59, 60, 61, 62, 63, 64, 65, 66, 67, 68, 69, 70, 71, 72, 73, 74, 75, 76, 77, 78, 84, 85, 86, 87, 88, 89, 91, 92, 93, 95, 96, 97, 98, 99, 100, 101, 102, 103, 104, 105, 106, 107, 108, 109, 110, 111, 112, 113, 114, 115, 116, 117, 119, 120, 121, 122, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 180, 181, 182, 183, 184, 85, 186, 187, 188, 189, 191, 192, 194, 195, 196, 197, 198, 199, 200, 201, 202, 203, 204, 205, 206, 207, 208, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 269, 270, 271, 272,273, 277, 279, 280, 281, 282, 284, 286, 287, 289, 290, 291, 295, 296, 297, 298, 300, 301, 302, 304, 305, 308, 309, 310, 31 l, 312, 313, 314, 315, 317, 318, 319, 320, 321, 322, 323, 325, 327, 328, 329, 330, 331, 332, 333, 334, 335, 336, 336, 337, 338, 339, 340, 341, 342, 343, 344, 345, 346, 347, 348, 349, 350, 351, 352, 353, 354, 355, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 410, 411, 412, 413, 423, 424, 425, 426, 427, 428, 430, 431, 432, 433, 434, 435, 436, 437, 438, 440, 441, 442, 443, 444, 445, 446, 447, 448, 449, 450, 452, 453, 455, 456, 457, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 490, 491, 492, 494, 495, 496, 497, 498, 499, 500, 501, 503, 504, 505, 506, 507, 508, 509, 510, 511, 512, 513, 514, 515, 516, 517, 518, 519, 520, 521, 525, 526, 527, 528, 529, 530, 531, 532, 533, 535, 537, 538, 539, 540, 541, 542, 543, 544

### Beispiel B

### Spodoptera frugiperda-Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von ≥ 80 % bei einer Aufwandmenge von 100 g / ha:

### Bsp Nr:

1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67,, 68, 69, 70, 71, 73, 74, 76, 77, 78, 79, 80, 81, 82, 83, 84, 85, 86, 87, 88, 89, 91, 92, 95, 96, 97, 98, 99, 100, 101, 102, 103, 104, 105, 106, 107, 108, 109, 110, 111, 112, 113, 114, 115, 116, 117, 118, 119, 120, 121, 122, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 189, 190, 191, 192, 195, 196, 197, 198, 199, 200, 201, 202, 203, 204, 205, 206, 207, 208, 210, 211, 212, 213,214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 277, 278, 279, 280, 281, 282, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 345, 346, 347, 348, 349, 350, 351, 352, 353, 354, 355, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408,410, 411, 412, 413, 423, 424, 425, 426, 427, 428, 430, 431, 432, 433, 434, 435, 436, 437, 438, 440, 441, 442, 443, 444, 445, 446, 447, 448, 449, 450, 452, 453, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 494, 495, 496, 497, 498, 499, 500, 501, 502, 503, 504, 505, 506, 507, 508, 509, 510, 511, 513, 514, 515, 516, 518, 519, 520, 521, 522, 523, 525, 526, 527, 528, 529, 530, 531, 532, 533, 534, 535, 536, 537,538,539,540,541,542,543,544

### Beispiel C

### Myzus-Test (MYZUPE Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis),* die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von ≥ 80 % bei einer Aufwandmenge von 100 g / ha :
Bsp Nr : 2, 5, 7, 8, 11, 13, 15, 16, 17, 18, 19, 20, 21, 22, 23, 24, 26, 27, 29, 30, 31, 32, 41, 42, 43, 44, 45, 52, 53, 54, 55, 59, 60, 64, 65, 67, 70, 74, 76, 77, 78, 80, 81, 82, 86, 89, 91, 92, 95, 96, 98, 102, 104, 105, 113, 114, 129, 134, 136, 153, 154, 158, 159, 160, 161, 162, 163, 164, 165, 166, 167, 168, 169, 170, 171, 172, 173, 174, 175, 176, 177, 178, 179, 180, 181, 182, 183, 186, 188, 189, 191, 196, 200, 201, 203, 211, 214, 217, 219, 220, 221, 222, 223, 224, 236,238, 240, 241, 242, 246, 255, 259, 263, 264, 265, 266, 269, 270, 271, 272, 273, 274, 280, 281, 284, 286, 289, 290, 295, 296, 298, 304, 305, 308, 309, 310, 312, 313, 314, 315, 316, 317, 318, 320, 322, 328, 330, 332, 333, 334, 335, 336, 337, 338, 339, 340, 340, 341, 342, 345, 346, 349, 351, 353, 354, 360, 361, 367, 372, 376, 377, 385, 391, 397, 398, 405, 406, 407, 410, 411, 412, 423, 424, 425, 426, 427, 428, 430, 432, 433, 434, 435, 437, 438, 440, 441, 442, 443, 444, 445, 446, 447, 448, 449, 450, 452, 455, 459, 462, 463, 464, 465, 466, 467, 470, 471, 472, 475, 476, 477, 478, 479, 480, 481, 483, 484, 485, 486, 487, 488, 491, 492, 494, 495, 497, 498, 500, 501, 503, 504, 505, 506, 507, 508, 509, 510, 511, 512, 513, 515, 516, 517, 518, 519, 521, 522, 525, 526, 528, 529, 530, 532, 533, 540, 542, 543, 544

Bei diesem Test zeigen z. B. die folgenden Verbindungen der Herstellungsbeispiele Wirkung von ≥ 80 % bei einer Aufwandmenge von 20 g / ha :
Bsp Nr: 97, 301, 329, 460, 461

### Beispiel D

### Lucilia cuprina-Test (LUCICU)

### Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele Wirkung von ≥ 80 % bei einer Aufwandmenge von 100 ppm :
Bsp Nr: 2, 3, 5, 6, 7, 10, 11, 13, 218, 220, 222, 223, 229, 295

### Beispiel E

### Boophilus microplus -Test (BOOPMI Injektion)

### Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele Wirkung von ≥ 80 % bei einer Aufwandmenge von 20 µg / Tier:
Bsp Nr: 2, 3, 5, 6, 7, 10, 11, 13, 218, 220, 222, 223, 229, 295

### Beispiel F

### Musca domestica-Test (MUSCDO)

### Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die einen Schwamm enthalten, der mit der Wirkstoffzubereitung der gewünschten Konzentration behandelt wurde, werden mit *Musca domestica Adulten* besetzt.

Nach der gewünschten Zeit wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z. B. die folgenden Verbindungen der Herstellungsbeispiele Wirkung von ≥ 80 % bei einer Aufwandmenge von 100 ppm :
Bsp Nr: 2, 3, 5, 7, 10, 11, 13, 133, 136, 137, 144, 151, 162, 175, 178, 218, 223

### Beispiel G

### Phaedon-Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele überlegene Wirksamkeit gegenüber dem Stand der Technik: siehe Tabelle

| **Wirkstoffe** | **Konzentration g/ha** | **Abtötungsgrad in % / 7 Tage** |
|---|---|---|
| 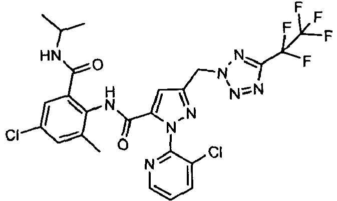 | 0,8 | 100 |
| Referenzverbindung | | |
| 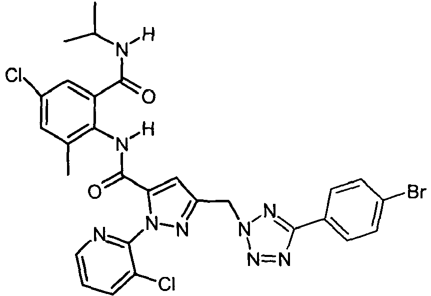 | 0,8 | 0 |
| Bekannt | | |
| 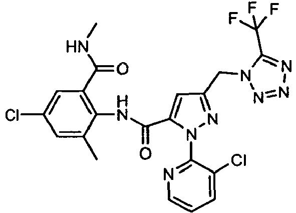 | 4 | 67 |
| Referenzverbindung | | |
| 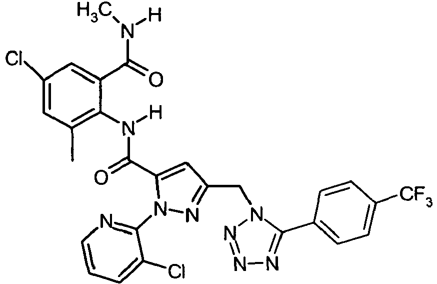 | 4 | 0 |
| Bekannt | | |

### Beispiel H

### Spodoptera frugiperda-Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele überlegene Wirksamkeit gegenüber dem Stand der Technik: siehe Tabelle

| **Wirkstoffe** | **Konzentration g/ha** | **Abtötungsgrad in % / 7 Tage** |
|---|---|---|
| 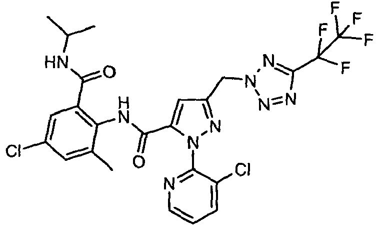 | 0,8 | 100 |
| Referenzverbindung | | |
| 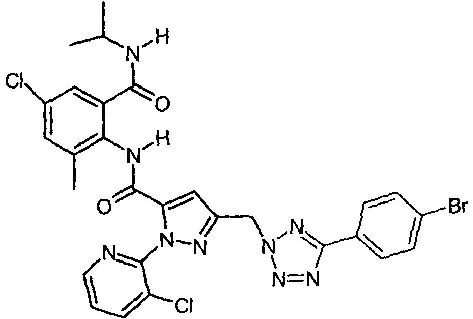 | 0,8 | 0 |
| Bekannt | | |
| 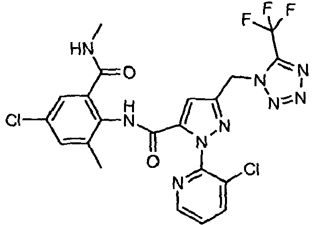 | 4 | 100 |
| Referenzverbindung | | |
| 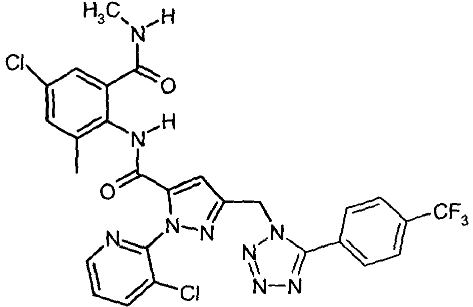 | 4 | 33 |
| Bekannt | | |

### Beispiel I

### Myzus-Test (MYZUPE Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis),* die von allen Stadien der Grünen Pfirsichblattlaus *(Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele überlegene Wirksamkeit gegenüber dem Stand der Technik: siehe Tabelle

| **Wirkstoffe** | **Konzentration g/ha** | **Abtötungsgrad in % / 7 Tage** |
|---|---|---|
| 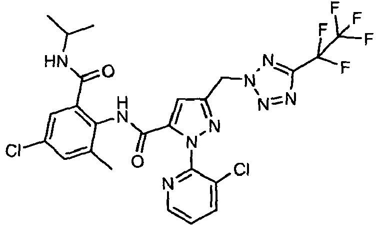 | 100 | 70 |
| Referenzverbindung | | |
| 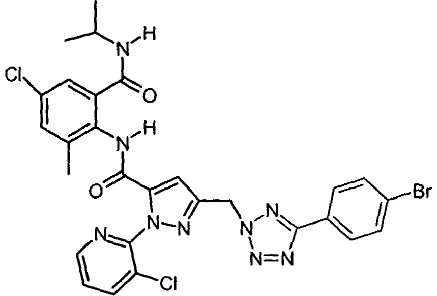 | 100 | 0 |
| Bekannt | | |
| 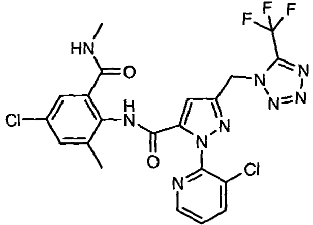 | 100 | 100 |
| Referenzverbindung | | |
| 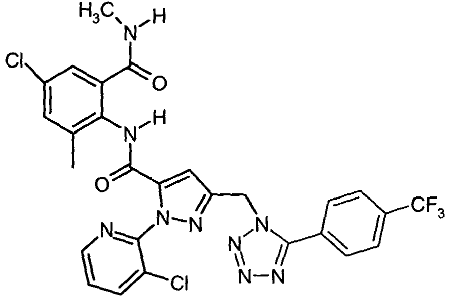 | 100 | 80 |
| Bekannt | | |

### Beispiel J

### Tetranychus-Test (OP-resistent/Spritzbehandlung)

| | | |
|---|---|---|
| Lösungsmittel: | 78 | Gewichtsteile Aceton |
| | 1,5 | Gewichststeile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (Phaseolus vulgaris), die von allen Stadien der Gemeinen Spinnmilbe (Tetranychus urticae) befallen sind, werden mit einer Wirkstoffzubereitung in der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigte die folgende Wirkstoffkombination gemäß vorliegender Anmeldung eine synergistisch verstärkte Wirksamkeit im Vergleich zu den einzeln angewendeten Wirkstoffen:
Wirkung von ≥ 80 % bei einer Aufwandmenge von 20 g/ha:
Bsp Nr: 363, 449

## Patentansprüche

1. Tetrazolsubstituierte Anthranilsäureamide der Formel (I) 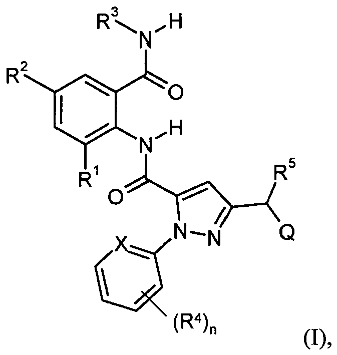 in welcher
R¹ für Methyl oder Chlor steht
R² für Halogen, Cyano, Methyl oder C₁-C₄-Alkylsulfonyl steht,
R³ für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl, C₃-C₁₂-Cycloalkyl, C₃-C₁₂-Cycloalkyl-C₁-C₆-Alkyl steht, wobei die Substituenten unabhängig voneinander ausgewählt sein können aus Halogen, Amino, Cyano, Nitro, Hydroxy, C₁-C₆-Alkyl, C₃-C₆-Cycloallryl, C₁-C₄-Alkoxy, C₁-C₄-Haloalkoxy, C₁-C₄-Alkylthio, C₂-C₆-Alkoxycarbonyl, C₂-C₆-Alkylcarbonyl C₃-C₆-Cycloalkylamino oder einem 5- oder 6-gliedrigen heteroaromatischen Ring,
n für 1, 2, 3 oder 4 steht,
X für N, CH, CF, CCl, CBr steht
R⁴ unabhängig voneinander für Wasserstoff, Cyano, Halogen(C₁-C₆)alkyl, Halogen oder Halogen(C₁-C₄)alkoxy steht
R⁵ für Wasserstoff oder C₁-C₆-Alkyl steht
Q für den einfach substituierten Tetrazol-Rest Q-2 steht, 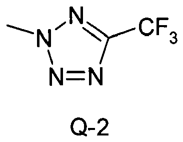
sowie Salze von Verbindungen der Formel (I).

2. Verbindungen der Formel (I-1) gemäß Anspruch 1 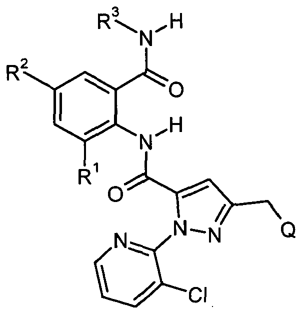 (I-1),
in welcher
R¹ für Methyl oder Chlor steht,
R² für Halogen, Cyano oder Methyl steht,
R³ für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl, C₃-C₁₂-Cycloalkyl, C₃-C₁₂-Cycloalkyl-C₁-C₆-Alkyl steht, wobei die Substituenten unabhängig voneinander ausgewählt sein können aus Halogen, Amino, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₂-C₆-Alkoxycarbonyl, C₂-C₆-Alkylcarbonyl C₃-C₆-Cycloalkylamino oder einem 5- oder 6-gliedrigen heteroaromatischen Ring,
Q für den einfach substituierten Tetrazol-Rest Q-2 steht, 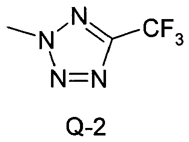
sowie Salze von Verbindungen der Formel (I-1).

3. Verbindungen der Formel (I-1) gemäß Anspruch 2, wobei
R¹ für Methyl steht,
R² für Halogen, Cyano oder Methyl steht,
R³ für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl, C₃-C₆-Cycloalkyl, C₃-C₆-Cycloalkyl-C₁-C₆-Alkyl steht, wobei die Substituenten unabhängig voneinander ausgewählt sein können aus Halogen, Cyano, Amino, Hydroxy, C₁-C₆-Alkyl, C₁-C₄-Alkoxy, C₁-C₄-Haloalkoxy, C₁-C₄-Alkylthio, C₃-C₆-Cycloalkyl, einem 5 oder 6-gliedrigen heteroaromatischen Ring enthaltend 1-2 Heteroatome aus der Reihe N,O, S, wobei nicht zwei Sauerstoffatome im Ring benachbart sind,
Q für den Rest Q-2 steht,
sowie Salze von Verbindungen der Formel (I).

4. Zusammensetzung enthaltend mindestens eine Verbindung der Formel (I) oder der Formel (I-1) gemäß einem der Ansprüche 1, 2 oder 3 und mindestens ein Salz der Formel (XI) 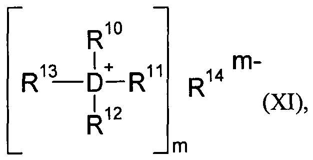 in welcher
D für Stickstoff oder Phosphor steht,
R¹⁰, R¹¹, R¹², and R¹³ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
m für 1, 2, 3 oder 4 steht,
R¹⁴ für ein anorganisches oder organisches Anion steht,

5. Zusammensetzung enthaltend mindestens eine Verbindung der Formel (I) oder der Formel (I-1) gemäß einem der Ansprüche 1, 2 oder 3 und mindestens einen Penetrationsförderer der Formel (XII)
R-O-(-AO)ᵥ-R' (XII),
in welcher
R für geradkettiges oder verzweigtes Alkyl mit 4 bis 20 Kohlenstoffatomen steht,
R' für Wasserstoff, Methyl, Ethyl, n-Propyl, i-Propyl, n-Butyl, i-Butyl, t-Butyl, n-Pentyl oder n-Hexyl steht,
AO für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
V für Zahlen von 2 bis 30 steht.

6. Verfahren zur Bekämpfung tierischer Schädlinge -ausgenommen zur therapeutischen Behandlung des tierischen oder menschlichen Körpers-, **dadurch gekennzeichnet, dass** man Verbindungen der Formel (I) oder der Formel (1-1) gemäß einem der Ansprüche 1,2 oder 3 oder eine Zusammensetzung gemäß den Ansprüchen 4 bis 5 auf tierische Schädlinge und/oder deren Lebensraum und/oder Saatgut und/oder Pflanzenvermehrungsmaterial und/oder später aus Pflanzenvermehrungsmaterial entstehende Pflanzenteile einwirken lässt.

7. Verfahren zur Herstellung agrochemischer Zusammensetzungen, **dadurch gekennzeichnet, dass** man Verbindungen der Formel (I) oder der Formel (I-1) gemäß einem der Ansprüche 1,2 oder 3 oder eine Zusammensetzung gemäß den Ansprüchen 4 bis 5 mit Streckmitteln und/oder oberflächenaktiven Stoffen vermischt.

8. Verwendung von Verbindungen der Formel (I) oder der Formel (I-1) gemäß einem der Ansprüche 1,2 oder 3 oder einer Zusammensetzung gemäß den Ansprüchen 4 bis 5 zur Bekämpfung von Schädlingen im Pflanzenschutz, im Materialschutz, im Hygienesektor und/oder im veterinärmedizinischen Sektor- ausgenommen zur therapeutischen Behandlung des tierischen oder menschlichen Körpers.

## Claims

1. Tetrazole-substituted anthranilamides of the formula (I) 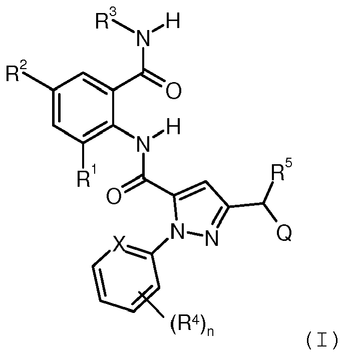 in which
R¹ represents methyl or chlorine,
R² represents halogen, cyano, methyl or C₁-C₄-alkylsulphonyl,
R³ represents hydrogen or represents C₁-C₆-alkyl, C₁-C₆-alkoxy, C₂-C₆-alkenyl, C₂-C₆-alkynyl, C₃-C₁₂-cycloalkyl, C₃-C₁₂-cycloalkyl-C₁-C₆-alkyl, each of which is optionally mono- or polysubstituted by identical or different substituents, where the substituents independently of one another may be selected from the group consisting of halogen, amino, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₂-C₆-alkoxycarbonyl, C₂-C₆-alkylcarbonyl, C₃-C₆-cycloalkylamino and a 5- or 6-membered heteroaromatic ring,
n represents 1, 2, 3 or 4,
X represents N, CH, CF, CCl, CBr,
R⁴ independently of one another represents hydrogen, cyano, halo-C₁-C₆-alkyl, halogen or halo-C₁-C₄-alkoxy,
R⁵ represents hydrogen or C₁-C₆-alkyl,
Q represents the monosubstituted tetrazole radical Q-2, 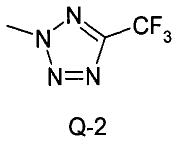
and also salts of compounds of the formula (I).

2. Compounds of the formula (I-1) according to Claim 1 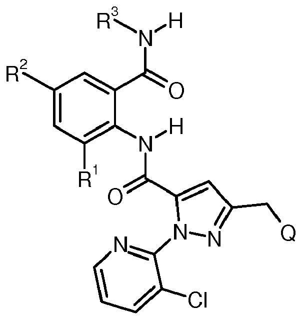 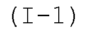 in which
R¹ represents methyl or chlorine,
R² represents halogen, cyano or methyl,
R³ represents hydrogen or represents C₁-C₆-alkyl, C₁-C₆-alkoxy, C₂-C₆-alkenyl, C₂-C₆-alkynyl, C₃-C₁₂-cycloalkyl, C₃-C₁₂-cycloalkyl-C₁-C₆-alkyl, each of which is optionally mono- or polysubstituted by identical or different substituents, where the substituents independently of one another may be selected from the group consisting of halogen, amino, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₂-C₆-alkoxycarbonyl, C₂-C₆-alkylcarbonyl, C₃-C₆-cycloalkylamino and a 5- or 6-membered heteroaromatic ring,
Q represents the monosubstituted tetrazole radical Q-2, 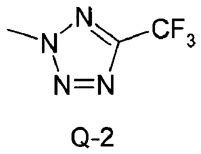 and also salts of compounds of the formula (I-1).

3. Compounds of the formula (I-1) according to Claim 2, where
R¹ represents methyl,
R² represents halogen, cyano or methyl,
R³ represents hydrogen or represents C₁-C₆-alkyl, C₁-C₆-alkoxy, C₂-C₆-alkenyl, C₂-C₆-alkynyl, C₃-C₆-cycloalkyl, C₃-C₆-cycloalkyl-C₁-C₆-alkyl, each of which is optionally mono- or polysubstituted by identical or different substituents, where the substituents independently of one another may be selected from the group consisting of halogen, cyano, amino, hydroxyl, C₁-C₆-alkyl, C₁-C₄-alkoxy, C₁-C₄-haloalkoxy, C₁-C₄-alkylthio, C₃-C₆-cycloalkyl and a 5- or 6-membered heteroaromatic ring which contains 1 or 2 heteroatoms from the group consisting of N, O and S, where two oxygen atoms are not adjacent to one another in the ring,
Q represents the radical Q-2,
and also salts of compounds of the formula (I).

4. Composition, comprising at least one compound of the formula (I) or the formula (I-1) according to any of Claims 1 to 3 and at least one salt of the formula (XI) 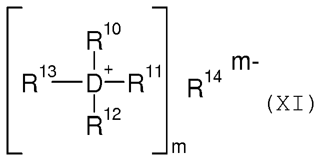 in which
D represents nitrogen or phosphorus,
R¹⁰, R¹¹, R¹², and R¹³ independently of one another represent hydrogen or in each case optionally substituted C₁-C₈-alkyl or mono- or polyunsaturated, optionally substituted C₁-C₈-alkylene, where the substituents may be selected from the group consisting of halogen, nitro and cyano,
m represents 1, 2, 3 or 4,
R¹⁴ represents an inorganic or organic anion,

5. Composition, comprising at least one compound of the formula (I) or the formula (I-1) according to any of Claims 1 to 3 and at least one penetrant of the formula (XII)
R-O-(-AO)ᵥ-R' (XII),
in which
R represents straight-chain or branched alkyl having 4 to 20 carbon atoms,
R' represents hydrogen, methyl, ethyl, n-propyl, isopropyl, n-butyl, isobutyl, tert-butyl, n-pentyl or n-hexyl,
AO represents an ethylene oxide radical, a propylene oxide radical, a butylene oxide radical or represents mixtures of ethylene oxide and propylene oxide radicals or butylene oxide radicals and
V represents a number from 2 to 30.

6. Method for controlling animal pests, with the exception of for the therapeutic treatment of the animal or human body, **characterized in that** compounds of the formula (I) or the formula (I-1) according to any of Claims 1 to 3 or a composition according to Claim 4 or 5 are/is allowed to act on animal pests and/or their habitat and/or seed and/or plant propagation material and/or plant parts formed later from plant propagation material.

7. Process for preparing agrochemical compositions, **characterized in that** compounds of the formula (I) or the formula (I-1) according to any of Claims 1 to 3 or a composition according to Claim 4 or 5 are/is mixed with extenders and/or surfactants.

8. Use of compounds of the formula (I) or the formula (I-1) according to any of Claims 1 to 3 or a composition according to Claim 4 or 5 for controlling pests in crop protection, in the protection of materials, in the hygiene sector and/or in the veterinary sector, with the exception of for the therapeutic treatment of the animal or human body.

## Revendications

1. Amides de l'acide anthranilique à substitution tétrazole de formule (I) 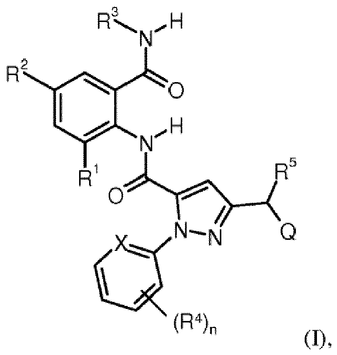 dans lesquels
R¹ représente méthyle ou chlore,
R² représente halogène, cyano, méthyle ou alkylsulfonyle en C₁-C₄,
R³ représente hydrogène ou alkyle en C₁-C₆, alcoxy en C₁-C₆, alcényle en C₂-C₆, alcynyle en C₂-C₆, cycloalkyle en C₃-C₁₂, cycloalkyle en C₃-C₁₂-alkyle en C₁-C₆, chacun éventuellement substitué une ou plusieurs fois, de manière identique ou différente, les substituants pouvant être choisis indépendamment les uns des autres parmi halogène, amino, cyano, nitro, hydroxy, alkyle en C₁-C₆, cycloalkyle en C₃-C₆, alcoxy en C₁-C₄, haloalcoxy en C₁-C₄, alkylthio en C₁-C₄, alcoxycarbonyle en C₂-C₆, alkylcarbonyle en C₂-C₆, cycloalkylamino en C₃-C₆ ou un cycle hétéroaromatique à 5 ou 6 éléments,
n représente 1, 2, 3 ou 4,
X représente N, CH, CF, CCl, CBr,
les R⁴ représentent indépendamment les uns des autres hydrogène, cyano, halogéno-alkyle en C₁-C₆, halogène ou halogéno-alcoxy en C₁-C₄,
R⁵ représente hydrogène ou alkyle en C₁-C₆,
Q représente le radical tétrazole Q-2 monosubstitué, 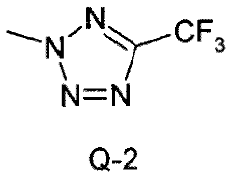 ainsi que les sels des composés de formule (I).

2. Composés de formule (1-1) selon la revendication 1 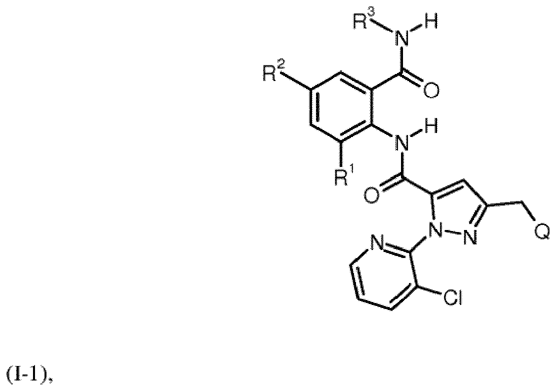 dans lesquels
R¹ représente méthyle ou chlore,
R² représente halogène, cyano ou méthyle,
R³ représente hydrogène ou alkyle en C₁-C₆, alcoxy en C₁-C₆, alcényle en C₂-C₆, alcynyle en C₂-C₆, cycloalkyle en C₃-C₁₂, cycloalkyle en C₃-C₁₂-alkyle en C₁-C₆, chacun éventuellement substitué une ou plusieurs fois, de manière identique ou différente, les substituants pouvant être choisis indépendamment les uns des autres parmi halogène, amino, cyano, nitro, hydroxy, alkyle en C₁-C₆, cycloalkyle en C₃-C₆, alcoxy en C₁-C₄, haloalcoxy en C₁-C₄, alkylthio en C₁-C₄, alcoxycarbonyle en C₂-C₆, alkylcarbonyle en C₂-C₆, cycloalkylamino en C₃-C₆ ou un cycle hétéroaromatique à 5 ou 6 éléments,
Q représente le radical tétrazole Q-2 monosubstitué, 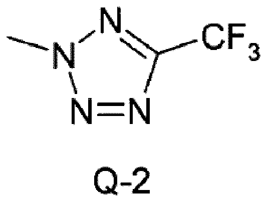 ainsi que les sels des composés de formule (1-1).

3. Composés de formule (1-1) selon la revendication 2, dans lesquels
R¹ représente méthyle,
R² représente halogène, cyano ou méthyle,
R³ représente hydrogène ou alkyle en C₁-C₆, alcoxy en C₁-C₆, alcényle en C₂-C₆, alcynyle en C₂-C₆, cycloalkyle en C₃-C₆, cycloalkyle en C₃-C₆-alkyle en C₁-C₆, chacun éventuellement substitué une ou plusieurs fois, de manière identique ou différente, les substituants pouvant être choisis indépendamment les uns des autres parmi halogène, cyano, amino, hydroxy, alkyle en C₁-C₆, alcoxy en C₁-C₄, haloalcoxy en C₁-C₄, alkylthio en C₁-C₄, cycloalkyle en C₃-C₆, un cycle hétéroaromatique à 5 ou 6 éléments contenant 1 à 2 hétéroatomes de la série N, O, S, deux atomes d'oxygène n'étant pas voisins dans le cycle,
Q représente le radical Q-2,
ainsi que les sels des composés de formule (I).

4. Composition contenant au moins un composé de formule (I) ou de formule (I-1) selon l'une quelconque des revendications 1, 2 ou 3 et au moins un sel de formule (XI) 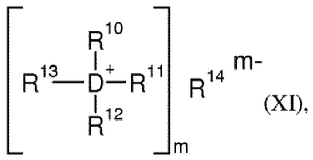 dans laquelle
D représente azote ou phosphore,
R¹⁰, R¹¹, R¹² et R¹³ représentent indépendamment les uns des autres hydrogène ou alkyle en C₁-C₈ à chaque fois éventuellement substitué ou alkylène en C₁-C₈ mono- ou polyinsaturé, éventuellement substitué, les substituants pouvant être choisis parmi halogène, nitro et cyano,
m représente 1, 2, 3 ou 4,
R¹⁴ représente un anion inorganique ou organique.

5. Composition contenant au moins un composé de formule (I) ou de formule (I-1) selon l'une quelconque des revendications 1, 2 ou 3 et au moins un promoteur de pénétration de formule (XII)
R-O-(-AO)ᵥ-R' (XII)
dans laquelle
R représente un alkyle linéaire ou ramifié contenant 4 à 20 atomes de carbone,
R' représente hydrogène, méthyle, éthyle, n-propyle, i-propyle, n-butyle, i-butyle, t-butyle, n-pentyle ou n-hexyle,
AO représente un radical oxyde d'éthylène, un radical oxyde de propylène, un radical oxyde de butylène ou des mélanges de radicaux oxyde d'éthylène et oxyde de propylène ou de radicaux oxyde de butylène, et
V représente des nombres de 2 à 30.

6. Procédé de lutte contre les animaux nuisibles, à l'exception du traitement thérapeutique du corps animal ou humain, **caractérisé en ce que** des composés de formule (I) ou de formule (I-1) selon l'une quelconque des revendications 1, 2 ou 3 ou une composition selon les revendications 4 à 5 sont laissés agir sur des animaux nuisibles et/ou leur habitat et/ou des graines et/ou des matériels de multiplication de plantes et/ou des parties de plantes formées ultérieurement à partir de matériels de multiplication de plantes.

7. Procédé de fabrication de compositions agrochimiques, **caractérisé en ce que** des composés de formule (I) ou de formule (I-1) selon l'une quelconque des revendications 1, 2 ou 3 ou une composition selon les revendications 4 à 5 sont mélangés avec des diluants et/ou des substances tensioactives.

8. Utilisation de composés de formule (I) ou de formule (1-1) selon l'une quelconque des revendications 1, 2 ou 3 ou d'une composition selon les revendications 4 à 5 pour lutter contre les nuisibles dans le cadre de la protection des plantes, de la protection des matériaux, dans le secteur de l'hygiène et/ou dans le secteur médical vétérinaire, à l'exception du traitement thérapeutique du corps animal ou humain.
